# EUROPÄISCHE PATENTSCHRIFT

(11) **EP 2 215 093 B1**
(45) Veröffentlichungstag und Bekanntmachung des Hinweises auf die Patenterteilung: **14.12.2011**
(21) Anmeldenummer: 08840365.4
(22) Anmeldetag: 16.10.2008
(51) Int. Cl.: C07D 495/04, C07D 519/00, A61K 31/519, A61P 11/00

(54) **NEUE PIPERAZINO-DIHYDROTHIENOPYRIMIDIN-DERIVATE**
NOVEL PIPERAZINO-DIHYDROTHIENOPYRIMIDINE DERIVATIVES
NOUVEAUX DÉRIVÉS DE PIPÉRAZINO-DIHYDROTHIÉNOPYRIMIDINE

(30) Priorität: 19.10.2007 EP 07118906
(43) Veröffentlichungstag der Anmeldung: 11.08.2010
(73) Patentinhaber: Boehringer Ingelheim International GmbH, 55216 Ingelheim am Rhein (DE)
(72) Erfinder: POUZET, Pascale, 55216 Ingelheim Am Rhein (DE); HOENKE, Christoph, 55216 Ingelheim Am Rhein (DE); NICKOLAUS, Peter, 55216 Ingelheim Am Rhein (DE); GOEGGEL, Rolf, 55216 Ingelheim Am Rhein (DE); FOX, Thomas, 55216 Ingelheim Am Rhein (DE); FIEGEN, Dennis, 55216 Ingelheim Am Rhein (DE); KLINDER, Klaus, 55216 Ingelheim Am Rhein (DE)
(74) Vertreter: Hammann, Heinz
(86) Internationale Anmeldenummer: PCT/EP2008/063970
(87) Internationale Veröffentlichungsnummer: WO 2009/050236

(56) Entgegenhaltungen:
- EP-A- 1 847 543
- WO-A-2006/111549
- CHAKRABORTI ET AL.: "3D-QSAR Studies on thieno[3,2-d]pyrimidines as Phosphodiesterase IV Inhibitors" BIOORG. MED. CHEM. LETT., Bd. 13, Nr. 8, 1. Januar 2003 (2003-01-01), Seiten 1403-1408, XP002392463 ISSN: 0960-894X
- ODINGO: "Inhibitors of PDE4: A review of recent patent literature" EXPERT OPIN. THER. PATENTS, Bd. 15, Nr. 7, 1. Januar 2005 (2005-01-01), Seiten 773-787, XP002413778 ISSN: 1354-3776

## Beschreibung

Die Erfindung betrifft neue Dihydrothienopyrimidinsulfoxide der Formel 1, sowie pharmakologisch verträglichen Salze, Diastereomere, Enantiomere, Racemate, Hydrate oder Solvate davon, 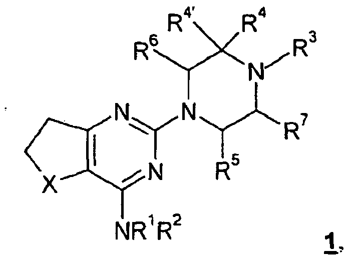 worin **X** SO oder SO₂, vorzugsweise jedoch SO, ist und worin R³, R⁴, R^{4'}, R⁵, R⁶ und R⁷ die in Anspruch 1 genannten Bedeutungen haben kann, sowie pharmazeutische Zusammensetzungen, die diese Verbindungen beinhalten.

Diese neuen Dihydrothienopyrimidinsulfoxide sind geeignet zur Behandlung von Atemwegs- oder gastrointestinalen Beschwerden oder Erkrankungen, entzündlichen Erkrankungen der Gelenke, der Haut oder der Augen, Erkrankungen des periphären oder zentralen Nervensystems oder Krebserkrankungen.

### STAND DER TECHNIK

US 3,318,881 und BE 663693 offenbaren die Herstellung von Dihydrothieno[3,2-d]pyrimidine die kardiovaskulären und sedative Eigenschaften besitzen. WO 2006/111549 und EP06112779.1 (EP1847543) offenbaren jeweils Dihydrothienopyrimidin-sulfoxide nach der obigen Formel **1**, wobei R⁴, R^{4'}, R⁵, R⁶, R⁷ jedoch ausschließlich Wasserstoff sein können.

### BESCHREIBUNG DER ERFINDUNG

Überraschenderweise konnte nun gefunden werden, dass Dihydrothienopyrimidinsulfoxide der Formel **1** in denen die Piperazin-Einheit der Formel **1** über den R³-Rest hinaus modifiziert ist, d.h. entweder weitere Substituenten umfasst, über Alkylengruppen verbrückt ist oder mit weiteren Ringen annelliert ist, besonders geeignet sind zur Behandlung entzündlicher Erkrankungen und gegenüber den entsprechenden Dihydrothienopyrimidinsulfoxiden aus dem Stand der Technik überlegen sind.

Gegenstand der vorliegenden Erfindung sind deshalb Verbindungen der Formel **1** 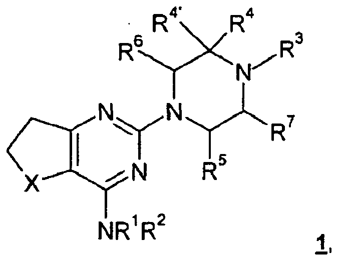 wie sie in den Ansprüchen definiert sind
sowie pharmakologisch verträgliche Salze davon.

Ein Gegenstand der vorliegenden Erfindung sind die Verbindungen der Formel **A** 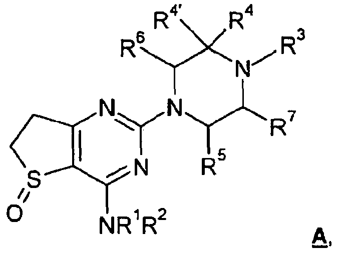 insbesondere jedoch sowohl die R-Enantiomere nach Formel **A'** als auch die S-Enantiomere nach Formel **A"** bezüglich des Stereozentrums am Sulfoxid-Schwefel-Atom der Verbindungen der Formel **1**,

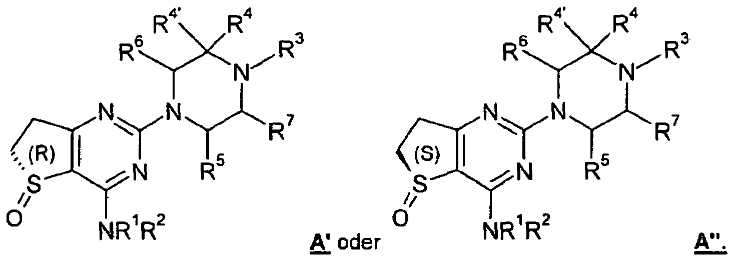

Ein besonders bevorzugter Gegenstand der Erfindung sind weiterhin die oben genannten Verbindungen der Formel **1**, wobei die Verbindungen ausgewählt sind aus der Gruppe bestehend aus 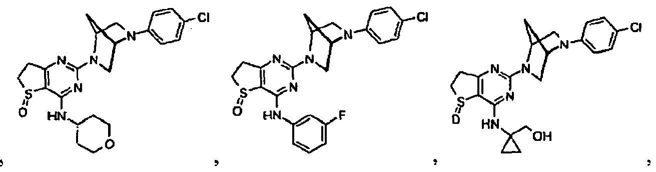 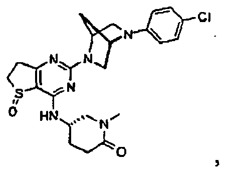 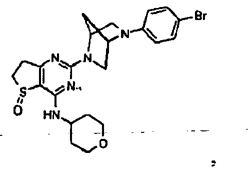 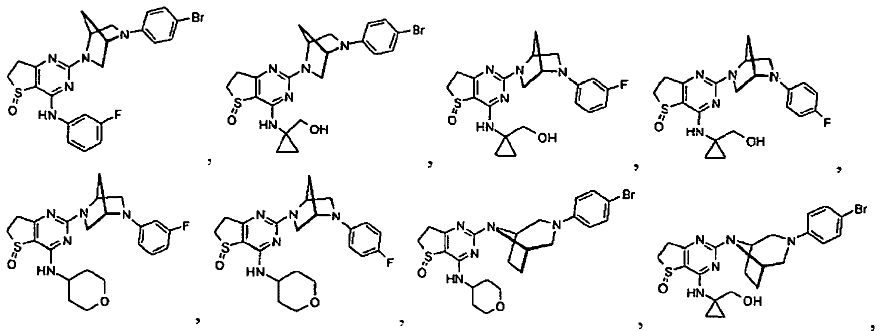 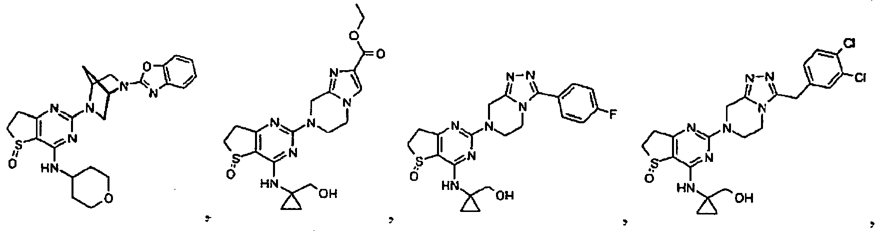 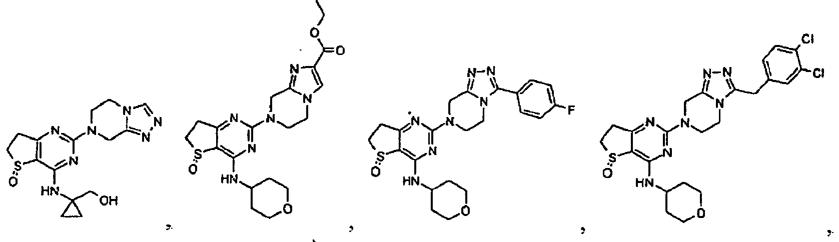 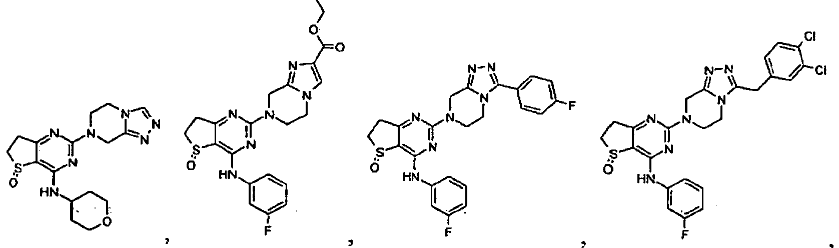 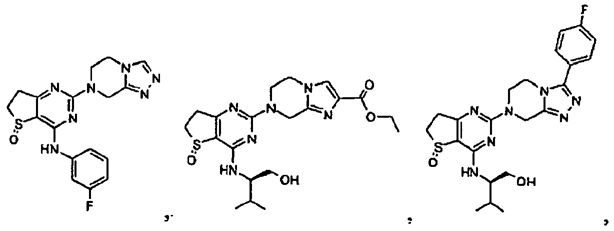 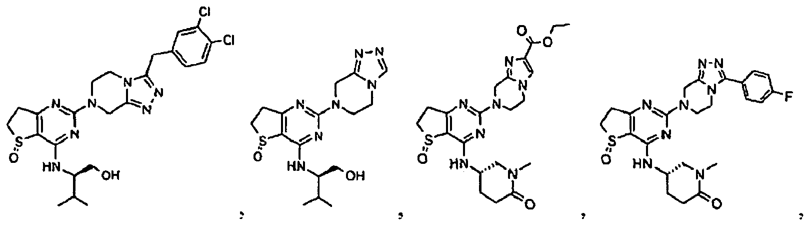 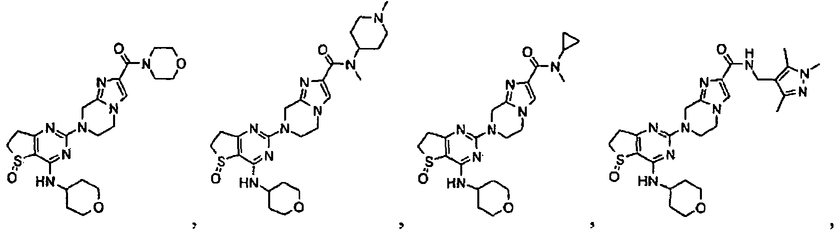 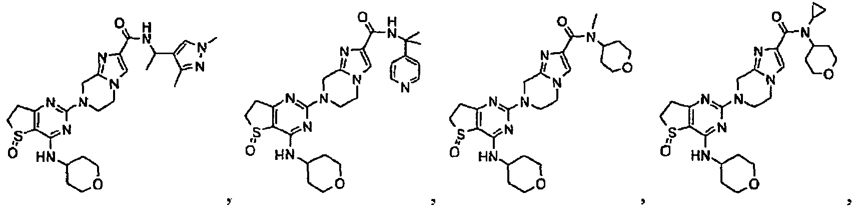 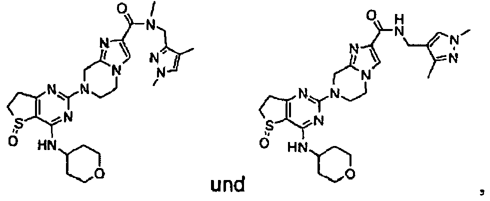 sowie pharmakologisch verträgliche Salze davon, insbesondere jedoch sowohl die R-Enantiomere als auch die S-Enantiomere bezüglich des Stereozentrums am Sulfoxid-Schwefel-Atom der oben genannten Verbindungen.

Ein weiterer Gegenstand der Erfindung sind die obigen Verbindungen der Formel **1** als Arzneimittel.

Ein weiterer Gegenstand der Erfindung ist die Verwendung der obigen Verbindungen nach Formel **1** zur Herstellung eines Medikaments zur Behandlung von Atemwegs- oder gastrointestinalen Beschwerden oder Erkrankungen, wie auch entzündliche Erkrankungen der Gelenke, der Haut oder der Augen, Krebserkrankungen, sowie von Erkrankungen des periphären oder zentralen Nervensystems.

Ein weiterer Gegenstand der Erfindung ist die Verwendung der obigen Verbindungen nach Formel **1** zur Herstellung eines Medikaments zur Vorbeugung und Behandlung von Atemwegs- oder Lungenerkrankungen, die mit einer erhöhten Schleimproduktion, Entzündungen und / oder obstruktiven Erkrankungen der Atemwege einhergehen.

Ein weiterer Gegenstand der Erfindung ist die Verwendung der obigen Verbindungen nach Formel **1** zur Herstellung eines Medikament zur Behandlung von entzündlichen und obstruktiven Erkrankungen wie COPD, chronischer Sinusitis, Asthma, Morbus Crohn, Colitis ulcerosa.

Ein weiterer Gegenstand der Erfindung ist die Verwendung der obigen Verbindungen nach Formel **1** zur Herstellung eines Medikaments zur Behandlung von entzündlichen Erkrankungen des Gastrointestinaltraktes.

Ein weiterer Gegenstand der Erfindung ist die Verwendung der obigen Verbindungen nach Formel **1** zur Herstellung eines Medikaments zur Vorbeugung und Behandlung von Erkrankungen des periphären oder zentralen Nervensystems wie Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Hirn-Trauma.

Ein weiterer Gegenstand der Erfindung sind pharmazeutische Formulierungen, die eine oder mehrere der obigen Verbindungen nach Formel **1** enthalten.

Ein weiterer Gegenstand der Erfindung sind pharmazeutische Formulierungen enthaltend eine oder mehrere Verbindungen der Formel **1** in Kombination mit einem oder mehreren Wirkstoffen ausgewählt aus der Gruppe bestehend aus Betamimetika, Corticosteroiden, weiteren PDE4-inhibitoren, EGFR-Hemmern und LTD4-Antagonisten, CCR3-Inhibitoren, iNOS-Inhibitoren und SYK-Inhibitoren.

### VERWENDETE BEGRIFFE UND DEFINITIONEN

Soweit nicht anders angegeben, sind alle Substituenten voneinander unabhängig. Sollten an einer Gruppe beispielsweise mehrere C₁₋₆-Alkylgruppen als Substituenten möglich sein, so könnte, beispielsweise im Fall von drei Substituenten C₁₋₆-Alkyl unabhängig voneinander beispielsweise einmal Methyl, einmal *n*-Propyl und einmal *tert*-Butyl bedeuten.

Im Rahmen dieser Anmeldung können bei der Definition von möglichen Substituenten, diese auch in Form einer Strukturformel dargestellt werden. Dabei wird ein Stern (*) in der Strukturformel des Substituenten als der Verknüpfungspunkt zum Rest des Moleküls verstanden. Weiterhin wird das auf den Verknüpfungspunkt folgende Atom des Substituenten als das Atom mit der Positionsnummer 1 verstanden. So werden zum Beispiel die Reste N-Piperidinyl **(I),** 4-Piperidinyl **(II),** 2-Tolyl **(III),** 3-Tolyl **(IV)** und 4-Tolyl **(V)** wie folgt dargestellt:

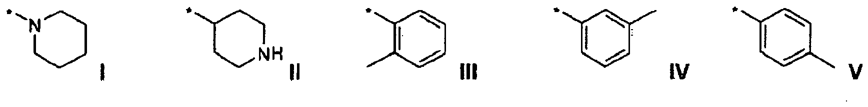

Befindet sich in der Strukturformel des Substituenten kein Stern (*), so kann an dem Substituenten jedes Wasserstoffatom entfernt werden und die dadurch frei werdende Valenz als Bindungsstelle zum Rest eines Molekül dienen, sofern die Anknüpfungsstelle zum Restmolekül nicht anderweitig bezeichnet oder definiert ist. So kann zum Beispiel **VI** die Bedeutung von 2-Tolyl, 3-Tolyl, 4-Tolyl und Benzyl haben.

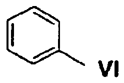

Unter dem Begriff "C₁₋₁₀-Alkyl" (auch soweit sie Bestandteil anderer Reste sind) werden verzweigte und unverzweigte Alkylgruppen mit 1 bis 10 Kohlenstoffatomen, unter dem Begriff "C₁₋₆-Alkyl" dementsprechend verzweigte und unverzweigte Alkylgruppen mit 1 bis 6 Kohlenstoffatomen verstanden. "C₁₋₄-Alkyl" steht entsprechend für verzweigte und unverzweigte Alkylgruppen mit 1 bis 4 Kohlenstoffatomen. Bevorzugt sind Alkylgruppen mit 1 bis 4 Kohlenstoffatomen. Beispielsweise werden hierfür genannt: Methyl, Ethyl, *n*-Propyl, *iso-*Propyl, *n*-Butyl, *iso*-Butyl, *sec*-Butyl, *tert*-Butyl, *n*-Pentyl, *iso*-Pentyl, *neo*-Pentyl oder Hexyl. Gegebenenfalls werden für vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl, Butyl, Pentyl und Hexyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert*-Butyl etc.

Unter dem Begriff "C₁₋₆-Alkylen" (auch soweit sie Bestandteil anderer Reste sind) werden verzweigte und unverzweigte Alkylengruppen mit 1 bis 6 Kohlenstoffatomen verstanden und unter dem Begriff "C₁₋₄-Alkylen" verzweigte und unverzweigte Alkylengruppen mit 1 bis 4 Kohlenstoffatomen verstanden. Bevorzugt sind Alkylengruppen mit 1 bis 4 Kohlenstoffatomen. Beispielsweise werden hierfür genannt: Methylen, Ethylen, Propylen, 1-Methylethylen, Butylen, 1-Methylpropylen, 1, 1-Dimethylethylen, 1, 2-Dimethylethylen, Pentylen, 1, 1-Dimethylpropylen, 2, 2, -Dimethylpropylen, 1, 2-Dimethylpropylen, 1, 3-Dimethylpropylen oder Hexylen. Sofern nicht anders beschrieben, umfassen die Definitionen Propylen, Butylen, Pentylen und Hexylen alle denkbaren isomeren Formen der jeweiligen Reste gleicher Kohlenstoffanzahl. So umfasst beispielsweise Propyl auch 1-Methylethylen und Butylen umfasst 1-Methylpropylen, 1, 1-Dimethylethylen, 1, 2-Dimethylethylen.

Sollte die Kohlenstoffkette mit einem Rest substituiert sein, der gemeinsam mit einem oder zwei Kohlenstoffatomen der Alkylenkette einen carbocyclischen Ring mit 3, 5 oder 6 Kohlenstoffatomen bildet, so sind damit unter anderem folgende Beispiele der Ringe umfasst:

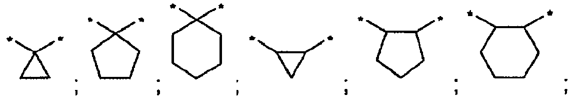

Unter dem Begriff "C₂₋₆-Alkenyl" (auch soweit sie Bestandteil anderer Reste sind) werden verzweigte und unverzweigte Alkenylgruppen mit 2 bis 6 Kohlenstoffatomen und unter dem Begriff "C₂₋₄-Alkenyl" verzweigte und unverzweigte Alkenylgruppen mit 2 bis 4 Kohlenstoffatomen verstanden, soweit sie mindestens eine Doppelbindung aufweisen. Bevorzugt sind Alkenylgruppen mit 2 bis 4 Kohlenstoffatome. Beispielsweise werden hierfür genannt: Ethenyl oder Vinyl, Propenyl, Butenyl, Pentenyl, oder Hexenyl. Sofern nicht anders beschrieben, umfassen die Definitionen Propenyl, Butenyl, Pentenyl und Hexenyl alle denkbaren isomeren Formen der jeweiligen Reste. So umfasst beispielsweise Propenyl 1-Propenyl und 2-Propenyl, Butenyl umfasst 1-, 2- und 3-Butenyl, 1-Methyl-1-propenyl, 1-Methyl-2-propenyl etc.

Unter dem Begriff "C₂₋₆-Alkenylen" (auch soweit sie Bestandteil anderer Reste sind) werden verzweigte und unverzweigte Alkenylengruppen mit 2 bis 6 Kohlenstoffatomen verstanden und unter dem Begriff "C₂₋₄-Alkenylen" verzweigte und unverzweigte Alkylengruppen mit 2 bis 4 Kohlenstoffatomen verstanden. Bevorzugt sind Alkenylengruppen mit 2 bis 4 Kohlenstoffatome. Beispielsweise werden hierfür genannt: Ethenylen, Propenylen, 1-Methylethenylen, Butenylen, 1-Methylpropenylen, 1, 1-Dimethylethenylen, 1, 2-Dimethylethenylen, Pentenylen, 1, 1-Dimethylpropenylen, 2, 2, -Dimethylpropenylen, 1, 2-Dimethylpropenylen, 1, 3-Dimethylpropenylen oder Hexenylen. Sofern nicht anders beschrieben, umfassen die Definitionen Propenylen, Butenylen, Pentenylen und Hexenylen alle denkbaren isomeren Formen der jeweiligen Reste gleicher Kohlenstoffanzahl. So umfasst beispielsweise Propenyl auch 1-Methylethenylen und Butenylen umfasst 1-Methylpropenylen, 1, 1-Dimethylethenylen, 1, 2-Dimethylethenylen.

Unter dem Begriff "C₂₋₆-Alkinyl" (auch soweit sie Bestandteil anderer Reste sind) werden verzweigte und unverzweigte Alkinylgruppen mit 2 bis 6 Kohlenstoffatomen und unter dem Begriff "C₂₋₄-Alkinyl" verzweigte und unverzweigte Alkinylgruppen mit 2 bis 4 Kohlenstoffatomen verstanden, soweit sie mindestens eine Dreifachbindung aufweisen. Bevorzugt sind Alkinylgruppen mit 2 bis 4 Kohlenstoffatome. Beispielsweise werden hierfür genannt: Ethinyl, Propinyl, Butinyl, Pentinyl, oder Hexinyl. Sofern nicht anders beschrieben, umfassen die Definitionen Propinyl, Butinyl, Pentinyl und Hexinyl alle denkbaren isomeren. Formen der jeweiligen Reste. So umfasst beispielsweise Propinyl 1-Propinyl und 2-Propinyl, Butinyl umfasst 1-, 2- und 3-Butinyl, 1-Methyl-1-propinyl, 1-Methyl-2-propinyl etc.

Unter dem Begriff "C₂₋₆-Alkinylen" (auch soweit sie Bestandteil anderer Reste sind) werden verzweigte und unverzweigte Alkinylengruppen mit 2 bis 6 Kohlenstoffatomen verstanden und unter dem Begriff "C₂₋₄-Alkinylen" verzweigte und unverzweigte Alkylengruppen mit 2 bis 4 Kohlenstoffatomen verstanden. Bevorzugt sind Alkinylengruppen mit 2 bis 4 Kohlenstoffatome. Beispielsweise werden hierfür genannt: Ethinylen, Propinylen, 1-Methylethinylen, Butinylen, 1-Methylpropinylen, 1, 1-Dimethylethinylen, 1, 2-Dimethylethinylen, Pentinylen, 1, 1-Dimethylpropinylen, 2, 2, -Dimethylpropinylen, 1, 2-Dimethylpropinylen, 1, 3-Dimethylpropinylen oder Hexinylen. Sofern nicht anders beschrieben, umfassen die Definitionen Propinylen, Butinylen, Pentinylen und Hexinylen alle denkbaren isomeren Formen der jeweiligen Reste gleicher Kohlenstoffanzahl. So umfasst beispielsweise Propinyl auch 1-Methylethinylen und Butinylen umfasst 1-Methylpropinylen, 1, 1-Dimethylethinylen, 1, 2-Dimethylethinylen.

Unter dem Begriff "Aryl" (auch soweit sie Bestandteil anderer Reste sind) werden aromatische Ringsysteme mit 6 bis 10 Kohlenstoffatomen verstanden. Beispielsweise werden hierfür genannt: Phenyl oder Naphthyl, bevorzugter Arylrest ist Phenyl. Soweit nicht anders beschrieben, können die Aromaten substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "Aryl-C₁₋₆-alkylen" (auch soweit sie Bestandteil anderer Reste sind) werden verzweigte und unverzweigte Alkylengruppen mit 1 bis 6 Kohlenstoffatomen verstanden, die mit einem aromatischen Ringsystem mit 6 oder 10 Kohlenstoffatomen substituiert sind. Beispielsweise werden hierfür genannt: Benzyl, 1- oder 2-Phenylethyl oder 1- oder 2-Naphthylethyl. Soweit nicht anders beschreiben, können die Aromaten substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "Heteroaryl-C₁₋₆-alkylen" (auch soweit sie Bestandteil anderer Reste sind) werden - obwohl auch bereits unter "Aryl-C₁₋₆-alkylen umfasst - verzweigte und unverzweigte Alkylengruppen mit 1 bis 6 Kohlenstoffatomen verstanden, die mit einem Heteroaryl substituiert sind.

Ein solches Heteroaryl umfasst fünf- oder sechsgliedrige heterocyclische Aromaten oder 5-10 gliedrige, bicyclische Heteroarylringe die ein, zwei oder drei Heteroatome, ausgewählt aus der Gruppe Sauerstoff, Schwefel und Stickstoff enthalten können und so viele konjugierte Doppelbindungen enthalten, dass ein aromatisches Systeme gebildet wird. Als Beispiele für fünf- oder sechsgliedrige heterocyclische Aromaten, werden genannt:

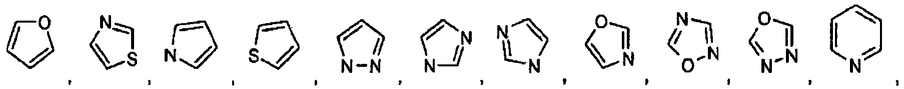

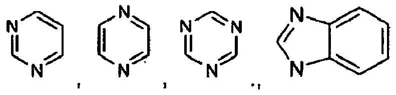

Soweit nicht anders beschreiben, können diese Heteroaryle substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Für die Heteroaryl-C₁₋₆-alkylene werden die folgenden Beispiele genannt:

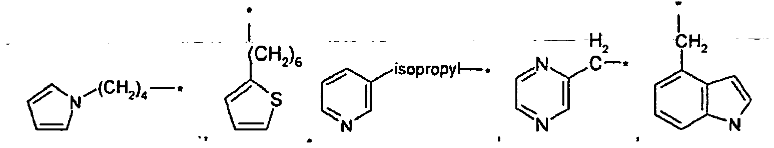

Unter dem Begriff "C₁-₆-Haloalkyl" (auch soweit sie Bestandteil anderer Reste sind) werden verzweigte und unverzweigte Alkylgruppen mit 1 bis 6 Kohlenstoffatomen verstanden, die mit einem oder mehreren Halogenatomen substituiert sind. Unter dem Begriff "C₁₋₄-Alkyl" verzweigte und unverzweigte Alkylgruppen mit 1 bis 4 Kohlenstoffatomen verstanden, die mit einem oder mehreren Halogenatomen substituiert sind. Bevorzugt sind Alkylgruppen mit 1 bis 4 Kohlenstoffatome. Beispielsweise werden hierfür genannt: CF₃, CHF₂, CH₂F, CH₂CF₃.

Unter dem Begriff "C₃₋₇-Cycloalkyl" (auch soweit sie Bestandteil anderer Reste sind) werden cyclische Alkylgruppen mit 3 bis 7 Kohlenstoffatomen verstanden. Beispielsweise werden hierfür genannt: Cyclopropyl, Cyclobutyl, Cyclopentyl, Cyclohexyl oder Cycloheptyl. Soweit nicht anders beschrieben, können die cyclischen Alkylgruppen substituiert sein mit einem oder mehreren Resten ausgewählt aus der Gruppe bestehend aus Methyl, Ethyl, iso-Propyl, *tert*-Butyl, Hydroxy, Fluor, Chlor, Brom und Jod.

Unter dem Begriffe "C₃₋₁₀-Cycloalkyl" werden zudem monocyclische Alkylgruppen mit 3 bis 7 Kohlenstoffatomen und auch bicyclische Alkylgruppen mit 7 bis 10 Kohlenstoffatomen verstanden oder auch monocyclische Alkylgruppen, die durch mindestens eine C₁₋₃-Kohlenstoffbrücke überbrückt sind.

Unter dem Begriff "heterocyclische Ringe" oder auch "Heterocyclus" werden fünf-, sechs- oder siebengliedrige, gesättigte oder ungesättigte heterocyclische Ringe verstanden die ein, zwei oder drei Heteroatome, ausgewählt aus der Gruppe Sauerstoff, Schwefel und Stickstoff enthalten können, dabei kann der Ring über ein Kohlenstoffatom oder falls vorhanden über ein Stickstoffatom mit dem Molekül verknüpft sein. Obwohl unter dem Begriff "heterocyclische Ringe" oder "Heterocyclus" umfasst, definiert der Begriff "heterocyclische, nichtaromatische Ringe" fünf-, sechs- oder siebengfiedrige ungesättigte Ringe. Als Beispiele werden genannt:

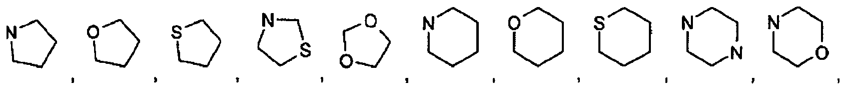

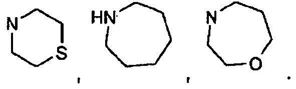

Obwohl unter dem Begriff "heterocyclische Ringe" oder "Heterocyclus" umfasst, definiert der Begriff "heterocyclische, aromatische Ringe" oder "Heteroaryl" fünf- oder sechsgliedrige heterocyclische Aromaten oder 5-10 gliedrige, bicyclische Heteroarylringe die ein, zwei, drei oder vier Heteroatome, ausgewählt aus der Gruppe Sauerstoff" Schwefel und Stickstoff, enthalten können und so viele konjugierte Doppelbindungen enthalten, dass ein aromatisches gebildet Systeme wird. Als Beispiele für fünf- oder sechsgliedrige heterocyclische Aromaten, werden genannt:

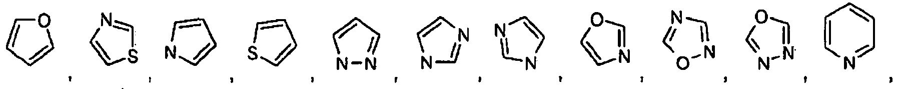

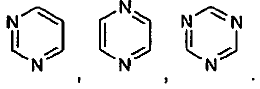

Soweit nicht anders erwähnt, kann ein heterocyclischer Ring (oder "Heterocyclus) mit einer Ketogruppe versehen sein. Als Beispiel hierfür werden genannt.

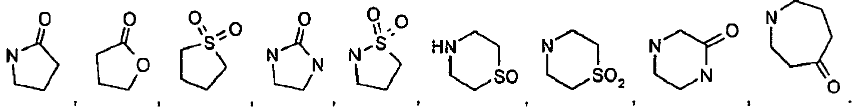

Obwohl unter "Cycloalkyl" bereits umfasst, werden unter dem Begriff "bicyclisches Cycloalkyle" in der Regel acht-, neun- oder zehngliedrige, bicyclische Kohlenstoff-Ringe verstanden. Beispielhaft werden genannt:

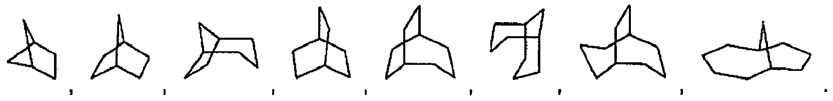

Obwohl unter "Heterocyclus" bereits umfasst, werden unter dem Begriff "bicyclische Heterocyclen" in der Regel acht-, neun- oder zehngliedrige, bicyclische Ringe verstanden, die ein oder mehrere Heteroatome, vorzugsweise 1-4, stärker bevorzugt, 1-3, noch stärker bevorzugt 1-2, insbesondere ein Heteroatom, ausgewählt aus der Gruppe Sauerstoff, Schwefel und Stickstoff enthalten können. Der Ring kann dabei über ein Kohlenstoffatom des Rings oder falls vorhanden über ein Stickstoffatom des Rings mit dem Molekül verknüpft sein. Beispielhaft werden genannt,

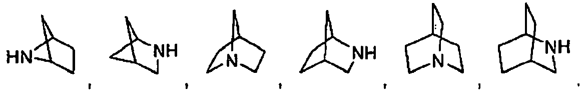

Obwohl unter "Aryl" bereits umfasst, wird unter einem "bicyclischen Aryl" ein 5-10 gliedriger, bicyclischer Arylring verstanden, der so viele konjugierte Doppelbindungen enthält, dass ein aromatisches System gebildet wird. Ein Beispiel für ein bicyclisches Aryl ist Naphthyl.

Obwohl unter "Heteroaryl" bereits umfasst, wird unter einem "bicydischen Heteroaryl" ein 5-10 gliedriger, bicyclischer Heteroarylring verstanden, der ein, zwei, drei oder vier Heteroatome, ausgewählt aus der Gruppe Sauerstoff, Schwefel und Stickstoff, enthalten kann und so viele konjugierte Doppelbindungen enthält, dass ein aromatisches System gebildet wird.

Obwohl unter dem Begriff "bicyclische Cycloalkyle" oder "bicyclisches Aryl" umfasst, definiert der Begriff "kondensiertes Cycloalkyl" oder "kondensiertes Aryl" bicyclische Ringe, in denen die die Ringe trennende Brücke eine direkte Einfachbindung bedeutet. Als Beispiel für einen kondensiertes, bicyclisches Cycloalkyl werden genannt:

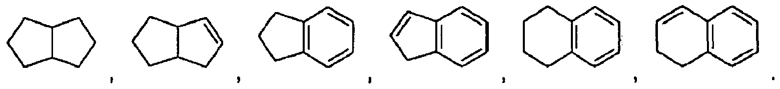

Obwohl unter dem Begriff "bicyclische Heterocyclen" oder "bicyclische Heteroaryle" umfasst, definiert der Begriff "kondensierte, bicyclische Heterocyclen " oder "kondensierte, bicyclische Heteroaryle"bicyclische 5-10 gliedrige Heteroringe die ein, zwei oder drei Heteroatome, ausgewählt aus der Gruppe Sauerstoff, Schwefel und Stickstoff enthalten und in denen die die Ringe trennende Brücke eine direkte Einfachbindung bedeutet. Die "kondensierten, bicyclischen Heteroaryle" enthalten zudem so viele konjugierte Doppelbindungen, dass ein aromatisches System gebildet wird. Beispielhaft werden genannt Pyrrolizin, Indol, Indolizin, Isoindol, Indazol, Purin, Chinolin, Isochinolin, Benzimidazol, Benzofuran, Benzopyran, Benzothiazol, Benzothiazol, Benzoisothiazol, Pyridopyrimidin, Pteridin, Pyrimidopyrimidin,

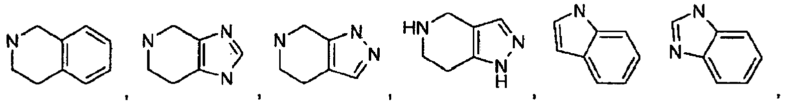

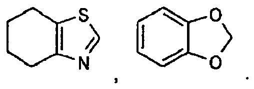

Unter dem Begriff "heterocyclische Spiroringe" (Spiro) werden 5-10 gliedrige, spirocyclische Ringe verstanden die gegebenenfalls ein, zwei oder drei Heteroatome, ausgewählt aus der Gruppe Sauerstoff, Schwefel und Stickstoff enthalten können, dabei kann der Ring über ein Kohlenstoffatom oder falls vorhanden über ein Stickstoffatom mit dem Molekül verknüpft sein. Soweit nicht anders erwähnt, kann ein spirocyclischer Ring mit einer Oxo-, Methyl- oder Ethylgruppe versehen sein. Als Beispiele hierfür werden genannt:

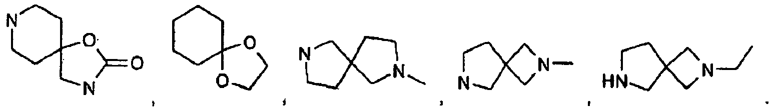

"Halogen" steht im Rahmen der vorliegenden Erfindung für Fluor, Chlor, Brom oder Jod. Sofern nicht gegenteilig angegeben, gelten Fluor, Chlor und Brom als bevorzugte Halogene.

Verbindungen der allgemeinen Formel **1** können Säuregruppen besitzen, hauptsächlich Carboxylgruppen, und/oder basische Gruppen wie z.B. Aminofunktionen. Verbindungen der allgemeinen Formel **1** können deshalb als innere Salze, als Salze mit pharmazeutisch verwendbaren anorganischen Säuren wie Salzsäure, Schwefelsäure, Phosphorsäure, Sulfonsäure oder organischen Säuren (wie beispielsweise Maleinsäure, Fumarsäure, Zitronensäure, Weinsäure oder Essigsäure) oder als Salze mit pharmazeutisch verwendbaren Basen wie Alkali- oder Erdalkalimetallhydroxiden oder Carbonaten, Zink- oder Ammoniumhydroxiden oder organischen Aminen wie z.B. Diethylamin, Triethylamin, Triethanolamin u.a. vorliegen.

Wie vorstehend genannt, können die Verbindungen der Formel **1** in ihre Salze, insbesondere für die pharmazeutische Anwendung, in ihre physiologisch und pharmakologisch verträglichen Salze überführt werden. Diese Salze können einerseits als physiologisch und pharmakologisch verträgliche Säureadditionssalze der Verbindungen der Formel **1** mit anorganischen oder organischen Säuren vorliegen. Andererseits kann die Verbindung der Formel **1** im Falle von R gleich Wasserstoff durch Umsetzung mit anorganischen Basen auch in physiologisch und pharmakologisch verträgliche Salze mit Alkali- oder Erdalkalimetallkationen als Gegenion überführt werden. Zur Darstellung der Säureadditionssalze kommen beispielsweise Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure in Betracht. Ferner können Mischungen der vorgenannten Säuren eingesetzt werden. Zur Darstellung der Alkali- und Erdalkalimetallsalze der Verbindung der Formel **1** in der R Wasserstoff bedeutet, kommen vorzugsweise die Alkali- und Erdalkalihydroxide und -hydride in Betracht, wobei die Hydroxide und Hydride der Alkalimetalle, besonders des Natriums und Kaliums bevorzugt, Natrium- und Kaliumhydroxid besonders bevorzugt sind.

Gegebenenfalls können die Verbindungen der allgemeinen Formel (1) in ihre Salze, insbesondere für die pharmazeutische Anwendung, in ihre pharmakologisch unbedenklichen Säureadditionssalze mit einer anorganischen oder organischen Säure, überführt werden. Als Säuren kommen hierfür beispielsweise Bernsteinsäure, Bromwasserstoffsäure, Essigsäure, Fumarsäure, Maleinsäure, Methansulfonsäure, Milchsäure, Phosphorsäure, Salzsäure, Schwefelsäure, Weinsäure oder Zitronensäure in Betracht. Ferner können Mischungen der vorgenannten Säuren eingesetzt werden.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie beispielsweise Oxal-, Fumar-, Diglycol- oder Methansulfonsäure.

Die erfindungsgemäßen Verbindungen können gegebenenfalls als Racemate vorliegen, sie können aber auch als reine Enantiomere, d.h. in (R)- oder (S)-Form gewonnen werden.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie beispielsweise Oxal-, Fumar-, Diglycol- oder Methansulfonsäure.

Gegenstand der Erfindung sind die jeweiligen Verbindungen der Formel **1** in Form ihrer pharmakologisch verträglichen Salze wie vorstehend beschrieben. Diese pharmakologisch verträglichen Salze der Verbindungen der Formel **1** können ebenfalls in Form ihrer jeweiligen Hydrate (z.B. Monohydrate, Dihydrate etc.) sowie in Form Ihrer jeweiligen Solvate vorliegen. Unter einem Hydrat der Verbindung nach Formel **1** versteht man im Rahmen der Erfindung ein kristallines, kristallwasserhaltiges Salz der Verbindung nach Formel **1.**

Unter einem Solvat der Verbindung nach Formel **1** versteht man im Rahmen der Erfindung ein kristallines Salz der Verbindung nach Formel **1,** welches Lösungsmittelmoleküle-Moleküle (z.B. Ethanol, Methanol etc) im Kristallgitter enthalten.

Dem Fachmann sind Standardverfahren zur Gewinnung von Hydraten und Solvaten (z.B. das Umkristallisieren aus dem entsprechenden Lösungsmittel bei Solvaten bzw. aus Wasser bei Hydraten) bekannt.

### SYNTHESEVORSCHRIFTEN

Die Verbindungen der allgemeinen Formel (I) können nach folgendem allgemeinen Syntheseschema hergestellt werden, wobei die Substituenten der allgemeinen Formel (I) die zuvor genannten Bedeutungen haben. Diese Verfahren sind als Erläuterung der Erfindung zu verstehen ohne selbige auf deren Gegenstand zu beschränken. Beispiele, die nicht von den Ansprüchen umfasst werden, sind als Referenz beispiele zu verstehen.

### ALLGEMEINE SYNTHESE SCHEMA

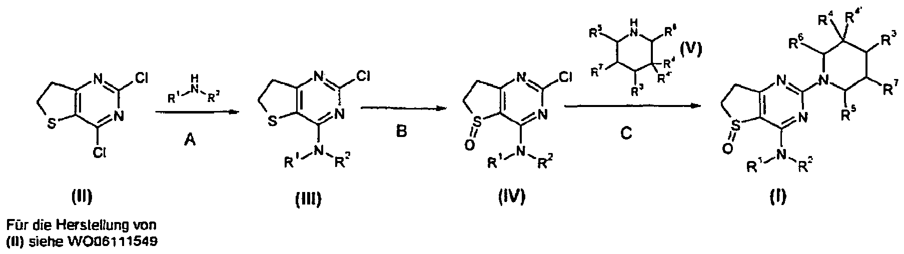

### 1. SYNTHESE VON (R)-2-{2-[(R)-4-(4-CHLORPHENYL)-2-METHYLPIPERAZIN-1-YL]-5-OXO-6,7-DIHYDRO-5H-5λ⁴-THIENO[3,2-d]PYRIMIDIN-4-YLAMINO}-3-METHYLBUTAN-1-OL (BEISPIEL 1)

### 1.1 (R)-2-(2-Chlor-6,7-dihydrothieno[3,2-d]pyrimidin-4-ylamino)-3-methylbutan-1-ol (III-1):

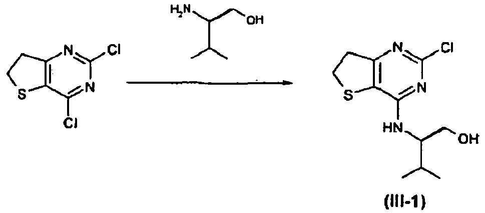

7,2 g 2,4-Dichlor-6,7-dihydrothieno[3,2-*d*]pyrimidin **(II)** werden in 36 ml Dioxan vorgelegt, anschliessend werden 18 ml Diisopropylethylamin und dann 6,1 g (R)-(-)-2-Amino-3-methyl-1-butanol zugegeben. Das Reaktionsgemisch wird bei 100°C, bis keine weitere Umsetzung erfolgt, erhitzt und nach Abkühlen eingedampft. Der Rückstand wird mit Petrolether/Essigester 9:1 im Ultraschallbad behandelt, der Feststoff abgesaugt und getrocknet. 8,3 g **(III-1)** werden als Feststoff erhalten. Analytische HPLC (Methode A): RT = 2,75 min

### 1.2 (R)-2-(2-Chlor-5-oxo-6,7-dihydro-5H-5λ⁴-thieno[-3,2-d]pyrimidin-4-ylamino)-3-methylbutan-1-ol (IV-1):

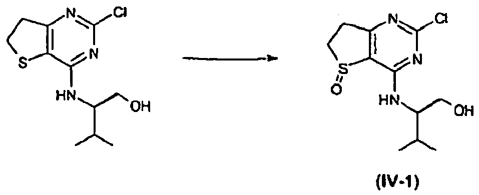

4,1 g S-(-)-1,1'-Bi-2-naphtol werden in 15 ml Chloroform unter Argon vorgelegt, dann 0,44 ml Titan(IV)-isopropylat und 0,54 ml Wasser zugegeben. Das Reaktionsgemisch wird 1 Stunde bei Raumtemperatur gerührt. Anschließend wird eine Suspension von 4,1 g **(III-1)** in 107 ml Dichlormethan zugegeben. Das Reaktionsgemisch wird auf -2°C abgekühlt und nach 30 Minuten werden 2,7 ml tert-Butylhydroperoxid 5-6 M in Decan zugetropft. Das Reaktionsgemisch wird bei -2 °C, bis keine weitere Umsetzung erfolgt, weitergerührt und mit NH₄OH basisch gestellt. Das Produkt wird mit Dichlormethan extrahiert und chromatographisch (Kieselgel, Essigester/Methanol 100/0 bis 86/14) gereinigt. 2,45 g **(IV-1)** werden als Feststoff erhalten. Analytische HPLC (Methode A): RT = 2,37 min

### 1.3 (R)-1-(4-Chlorphenyl)-3-methylpiperazin (V-1)

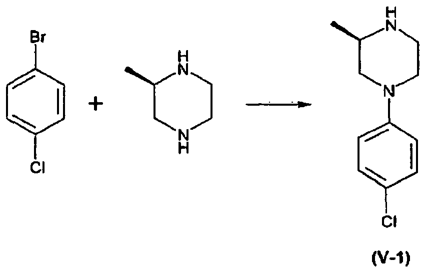

0,068 g 4-Bromchlorbenzol,1 g (R)-2-Methylpiperazin und 1,1 g Kalium-*tert*-Butoxid werden in 11 ml wasserfreies und entgastes Toluol suspendiert und unter Argon auf 80°C erhitzt. 0,055 g Tris(dibenzylidenaceton)dipatiadium(0) und 0,077 g rac-BtNAP werden zugefügt. Das Reaktionsgemisch wird 7 Stunden bei 90°C gerührt und mit Wasser versetzt. Das Produkt wird mit Essigester extrahiert und chromatographisch (präparative HPLC, Methode A) gereinigt. 0,249 g **(V-1)** werden als Trifluoracetat erhalten. Analytische HPLC-MS (Methode A): RT=1,08 min.

### 1.4 (R)-2-{2-[(R)-4-(4-Chlorphenyl)-2-methylpiperazin-1-yl]-5-oxo-6,7-dihydro-5H-5λ⁴-thieno[3,2-d]pyrimidin-4-ylamino}-3.methylbutan-1-ol (Beispiel 1)

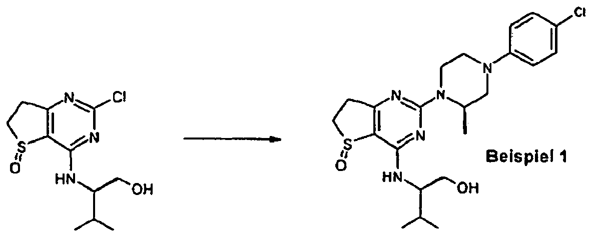

0,16 g **(IV-1)** wird in 2,5 ml Dioxan und 206 µl Diisopropylethylamin vorgelegt, mit 0,16 g **(V-1)** (siehe 1.3) versetzt und bei 140°C. bis keine weitere Umsetzung erfolgt, in der Mikrowelle erhitzt. Das Reaktionsgemisch wird mit Wasser versetzt, das Produkt abgesaugt und chromatographisch (präparative HPLC, Methode B) gereinigt. 0,03 g Beispiel 1 werden als Feststoff erhalten. Analytische HPLC-MS (Methode A): RT = 1,26 min.

### 2. SYNTHESE VON (S)-5-{2-[5-(4-CHLORPHENYL)-2,5-DIAZABICYCLO[2.2.1]HEPT-2-YL]-5-OXO-6,7-DIHYCRO-5H-5λ⁴-THIENO[3,2-d]PYRIMIDIN-4-YLAMINO}-1-METHYLPIPERIDIN-2-ON (BEISPIEL 9)

### 2.1 (S)-5-Dibenzylaminopiperidin-2-on:

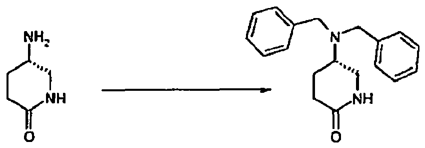

0,600 g 4-(S)-Amino-delta-valerolactam Hydrochlorid, 0,970 ml Benzylbromid und 1,5 g Natriumhydrogencarbonat werden in 30 ml Ethanol suspendiert. Das Reaktionsgemisch wird dann 8 Stunden bei 80°C gerührt und anschießend zur Trockne eingedampft. Der Rückstand wird in Wasser suspensiert und das Produkt mit Dichlormethan extrahiert und chromatographisch (Kieselgel, Dichlormethan/Methanol 100/0 bis 95/5) gereinigt. 0,500 g des Produktes werden als Öl erhalten. Analytische HPLC-MS (Methode A): RT = 1,01 min.

### 2.2 (S)-5-Dibenzylamino-1-methylpiperidin-2-on:

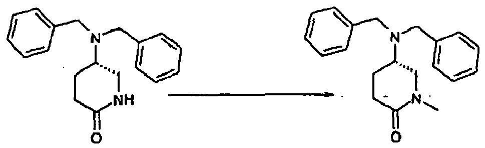

0,500 g (S)-5-Dibenzylaminopiperidin-2-on werden in 15 ml Tetrahydrofuran suspendiert. Unter Eisbadkühlung werden 0,175 g Kalium-*tert*-butylat zugegeben. Das Reaktionsgemisch wird dann 30 Minuten bei Raumtemperatur gerührt. Unter Eisbadkühlung werden 0,095 ml Methyliodid zugegeben. Das Reaktionsgemisch wird dann 48 Stunden bei Raumtemperatur gerührt und anschließend mit einer gesättigten NaCl Lösung versetzt. Das Produkt wird mit Essigester extrahiert. 0,450 g des Produktes werden als Öl erhalten. Analytische HPLC-MS (Methode A): RT = 1,07 min.

### 2.3 (S)-5-Amino-1-methylpiperidin-2-on:

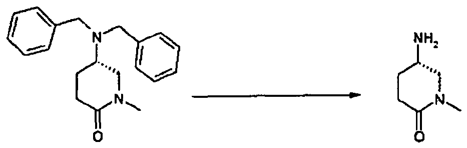

0,450 g (S)-5-Dibenzylamino-1-methylpiperidin-2-on werden in 25 ml Methanol suspendiert und mit 0,150 g Pd/C 10% bei einem Druck von 3 bar und einer Temperatur von 60 °C hydriert. Nach 16 Stunden wird der Katalysator abgesaugt und das Filtrat zur Trockne eingedampft. 0,190 g des Produktes werden als Öl erhalten. ¹H NMR (400 MHz, DMSO): 2,76 (3H, s).

### 2.4 (S)-5-(2-Chlor-6,7-dihydro-thieno[3,2-d]pyrimidin-4-ylamino)-1-methylpiperidin-2-on (III-2):

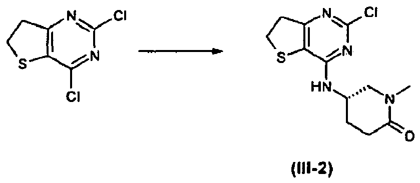

0,27 g **(II)** werden in 3 ml Dioxan vorgelegt, anschliessend werden 0,45 ml Diisopropylethylamin und dann 0,25 g (S)-5-Amino-1-methylpipendin-2-on zugegeben. Das Reaktionsgemisch wird bei 130°C, bis keine weitere Umsetzung erfolgt, erhitzt und nach Abkühlen eingedampft. Das Produkt wird mit Dichlormethan extrahiert und chromatographisch (präparative HPLC, Methode A) gereinigt. 0,26 g **(III-2)** werden als Feststoff erhalten. Analytische HPLC-MS (Methode A): RT = 1,06 min.

### 2.5 (S)-5-(2-Chlor-5-oxo-6,7-dihydro-5H-5λ⁴-thieno[3,2-d]pyrimidin-4-ylamino)-1-methylpiperidin-2-on (IV-2):

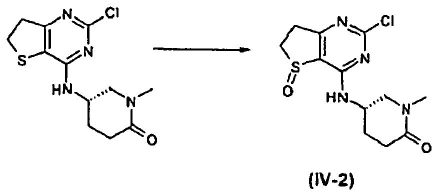

0,04 g S-(-)-1,1'-Bi-2-naphtol werden in 5 ml Chloroform unter Argon vorgelegt, dann 0,02 ml Titan(IV)-isopropylat und 0,025 ml Wasser zugegeben. Das Reaktionsgemisch wird 1 Stunde bei Raumtemperatur gerührt. Anschließend wird eine Suspension von 0,2 g **(III-2)** in 4 ml Dichlormethan zugegeben. Das Reaktionsgemisch wird auf -5°C abgekühlt und nach 20 Minuten werden 0,12 ml tert-Butylhydroperoxid 5-6 M in Decan zugetropft. Das Reaktionsgemisch wird bei -5°C, bis keine weitere Umsetzung erfolgt, weitergerührt und mit NH₄OH basisch gestellt. Das Produkt wird chromatographisch (Kieselgel, Essigester/Methanol 100/0 bis 60/40) gereinigt. 0,09 g **(IV-2)** werden als Feststoff erhalten. Analytische HPLC-MS (Methode A): RT = 0,83 min.

### 2.6 (S)-5-{2-[5-(4-Chlorphenyl)-2,5-diazabicyclo[2.2.1]hept-2-yl]-5-oxo-6,7-dihydro-5H-5λ⁴-thieno[3,2-d]pyrimidin-4-ylamino}-1-methylpiperidin-2-on (Beispiel 9)

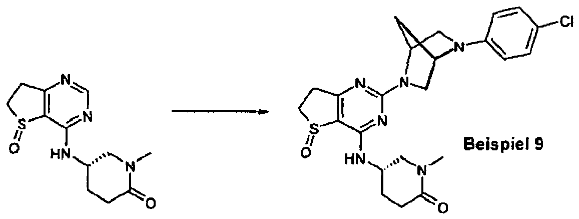

0,059 g **(IV-2)** wird in 0,5 ml Dioxan und 99 µl Diisopropylethylamin vorgelegt, mit 0,061 g 2-(4-Chlorphenyl)-2,5-diazabicyclo[2.2.1]heptan piperazin versetzt und bei 120°C, bis keine weitere Umsetzung erfolgt, in der Mikrowelle erhitzt. Das Reaktionsgemisch wird mit Wasser versetzt und das Produkt mit Dichlormethan extrahiert und chromatographisch (präparative HPLC, Methode C) gereinigt. Die Produktfraktionen werden mit Ammoniak basisch gestellt und mit Dichlormethan extrahiert. 0,04 g Beispiel 9 werden als Feststoff erhalten. Analytische HPLC-MS (Methode A): RT =1,13 min.

### 3. SYNTHESE VON {2-[5-(4-BROMPHENYL)-2,5-DIAZABICYCLO[2.2.1]HEPT-2-YL]-5-OXO-6,7-DIHYDRO-5H-5λ⁴-THIENO[3,2-d]PYRIMIDIN-4-YL}-(3-FLUORPHENYL)-AMIN (BEISPIEL 21)

### 3.1 (2-Chlor-6,7-dihydrothieno[3,2-d]pyrimidin-4-yl)-(3-fluorphenyl)-amin (III-3):

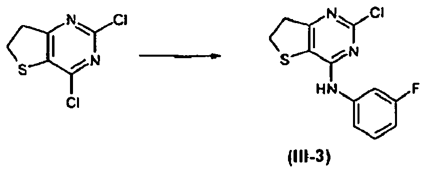

4 g **(II)** werden in 15 ml Dimethylformamid vorgelegt, anschliessend werden 4,5 ml Diisopropylethylamin und dann 2,5 ml 3-Fluor-phenylamin zugegeben. Das Reaktionsgemisch wird bei 120°C, bis keine weitere Umsetzung erfolgt, erhitzt und nach Abkühlen eingedampft. Der Rückstand wird mit Wasser versetzt. Das Produkt wird mit Dichlormethan extrahiert und chromatographisch (Kieselgel, Petrolether/Essigester 80/20 bis 60/40) gereinigt. 2,6 g **(III-3)** werden als Feststoff erhalten. Analytische HPLC (Methode A): RT = 3,27 min.

### 3.2 2-Chlor-5-oxo-6,7-dihydro-5H-5λ⁴-thieno[3,2-d]pyrimidin-4-yl)-(3-fluorphenyl)-amin (IV-3):

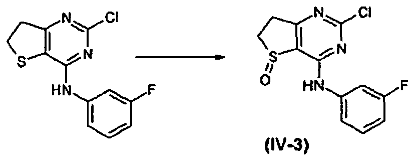

0,102 g S-(-)-1,1'-Bi-2-naphtol werden in 0,5 ml Chloroform unter Argon vorgelegt, dann 0,052 ml Titan(IV)-isopropylat und 0,064 ml Wasser zugegeben. Das Reaktionsgemisch wird 45 Minuten bei Raumtemperatur gerührt. Anschließend wird eine Suspension von 0,5 g **(III-3)** in 25 ml Chloroform zugegeben. Das Reaktionsgemisch wird auf -2°/-4°C abgekühlt und nach 20 Minuten werden 0,323 ml tert-Butylhydroperoxid 5-6 M in Decan zugetropft. Das Reaktionsgemisch wird bei -2/-4°C, bis keine weitere Umsetzung erfolgt, weitergerührt und mit Wasser versetzt. Das Produkt wird mit Dichlormethan extrahiert und chromatographisch (Kieselgel, Dichlormethan/Methanol 100/0 bis 95/5) gereinigt. 0,47 g **(IV-3)** werden als Feststoff erhalten. Analytische HPLC-MS (Methode A): RT = 1,15 min.

### 3.3 2-(4-Bromphenyl)-2,5-diazabicyclo[2.2.1]heptan (V-2)

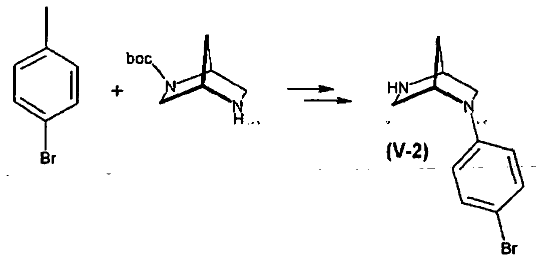

5,5 g 2,5-Diazabicyclo[2.2.1]heptan-2-BOC, 7,9 g 4-Bromjodbenzol, 0,77 g Tris(dibenzylidenaceton)dipalladium(0), 0,79 g rac-BINAP, 3,8 g Natrium-*tert*-Butoxid und. 10,4 g 1,4,7,10,13,16-Hexaoxacyclooctadecan werden in 55 ml wasserfreies und entgastes Tetrahydrofuran suspendiert und unter Argon bei Raumtemperatur, bis keine weitere Umsetzung erfolgt, gerührt. Das Reaktionsgemisch wird zur Trockne eingedampft und mit Wasser versetzt. Das Produkt wird mit Dichlormethan extrahiert und chromatographisch (Kieselgel Dichlormethan/Methanol 100/0 bis 0/100) gereinigt. 2,28 g 5-(4-Bromphenyl)-2,5-diazabicyclo[2.2.1]heptan-2-BOC werden erhalten.

Das erhaltene Produkt und 8,6 ml Trifluoressigsäure werden in 54 ml Dichlormethan suspendiert und bei Raumtemperatur, bis keine weitere Umsetzung erfolgt, gerührt. Das Reaktionsgemisch wird dann zur Trockne eingedampft. Der Rückstand wird in Diethylether suspendiert und der Feststoff abgesaugt. 2,34 g **(V-2)** werden als Trifluoracetat erhalten. Analytische HPLC-MS (Methode A): RT = 1,06 min.

### 3.4 {2-[5-(4-Bromphenyl)-2,5-diazabicyclo[2.2.1]hept-2-yl]-5-oxo-6,7-dihydro-5H-5λ⁴-thieno[3,2-d]pyrimidin-4-yl}-(3-fluorphenyl)-amin (Beispiel 21)

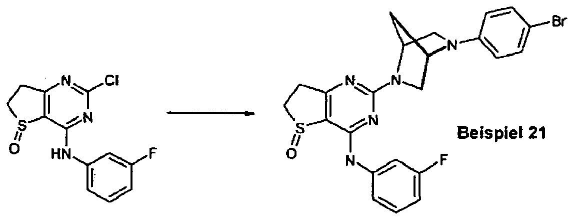

0,179 g **(IV-3)** wird in 1,6 ml N-Methyl-2-pyrrolidon (NMP) vorgelegt. 0,32 ml Diisopropylethylamin und 0,26 g **(V-2)** (siehe 3.3) werden zugefügt. Das Reaktionsgemisch wird in der Mikrowelle bei 120°C, bis keine weitere Umsetzung erfolgt, erhitzt. Das Produkt wird chromatographisch (präparative HPLC, Methode D) gereinigt. 0,13 g **Beispiel 21** werden als Feststoff erhalten. Analytische HPLC-MS (Methode A): RT = 1,34 min.

### 4. SYNTHESE VON (1-{2-[5-(4-FLUORPHENYL)-2,5-DIAZABICYCLO[2.2.1]HEPT-2-YL]-5-OXO-6,7-DIHYDRO-5H-5λ⁴-THIENO[3,2-d]PYRIMIDIN-4-YLAMINO}-CYCLOPROPYL)-METHANOL (BEISPIEL 24)

### 4.1 (1-Hydroxymethylcyclopropyl)-carbamidsäure tert-butylester:

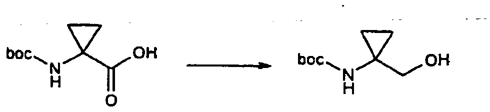

1 g 1-(BOC-amino)-cyclopropancarbonsäure wird in 20 ml Dimethoxyethan gelöst und auf - 70°C abgekühlt. Dann werden 0,65 ml N-Methylmorpholin zugegeben. 0,71 ml Isobutylchloroformiat in 5 ml Dimethoxyethan werden zugetropft. Das Reaktionsgemisch wird auf -5°C erwärmt. Der Niederschlag wird abgesaugt. Das Eluat wird auf -15°C abgekühlt und 0,303 g Natriumborhydrid werden langsam zugegeben. Das Reaktionsgemisch wird anschliessend 30 Minuten bei Raumtemperatur gerührt, mit Wasser versetzt und das Produkt mit Dichlormethan extrahiert. Die organische Phase wird getrocknet und zur Trockne eingedampft. 1,04 g Produkt werden als Feststoff erhalten. ¹H NMR (400 MHz, DMSO): 1,36 (9H, s); 0,61 (2H, t); 0,52 (2H, t).

### 4.2 1-Aminocyclopropanmethanol:

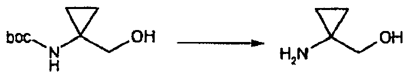

1,04 g (1-Hydroxymethylcyclopropyl)-carbamidsäure *tert*-butylester werden in 5 ml Dioxan vorgelegt. 2,5 ml HCl in Dioxan (4 mol/l) werden zugetropft. Das Reaktionsgemisch wird bei Raumtemperatur 15 h gerührt. Das Lösungsmittel wird zur Hälfte eingedampft und der ausgefallene Feststoff abgesaugt. 0,5 g Produkt werden als Hydrochlorid erhalten. ¹H NMR (400 MHz, DMSO): 5,27 (1H, t); 0,91 (2H, t); 0,71 (2H, t).

### 4.3 [1-(2-Chlor-6,7-dihydrothieno[3,2-d]pyrimidin-4-ylamino)-cyclopropyl]-methanol (III-4):

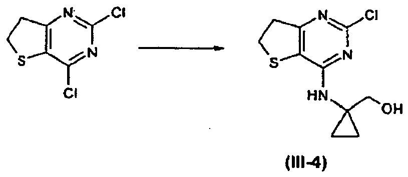

1,4 g **(II)** werden in 10 ml Dioxan vorgelegt, erst werden 3,6 ml Diisopropylethylamin, dann 1 g 1-Aminocyclopropanmethanol (siehe 4.2) zugegeben. Das Reaktionsgemisch wird bei 160°C, bis keine weitere Umsetzung erfolgt, erhitzt und nach Abkühlen eingedampft. Der Rückstand wird mit Cyclohexan/Essigester (8:2) im Ultraschallbad behandelt und der Feststoff abgesaugt und getrocknet. 1,24 g **(III-4)** werden als Feststoff erhalten. Analytische HPLC-MS (Methode A): RT = 1,01 min.

### 4.4 [1-(2-Chlor-5-oxo-6,7-dihydro-5H-5λ⁴-thieno[3,2-d]pyrimidin-4-ylamino)-cyclopropyl]-methanol (IV-4):

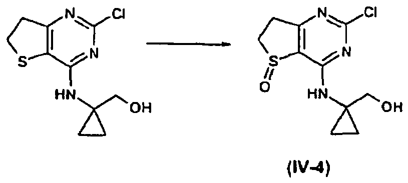

0,28 g S-(-)-1,1'-Bi-2-naphtol werden in 20 ml Chloroform unter Argon vorgelegt, dann 0,14 ml Titan(IV)-isopropylat und 0,17 ml Wasser zugegeben. Das Reaktionsgemisch wird 1 Stunde bei Raumtemperatur gerührt. Anschließend wird eine Suspension von 1,2 g **(III-4)** in 40 ml Dichlormethan und 2 ml Methanol zugegeben. Das Reaktionsgemisch wird auf -5°C abgekühlt und nach 30 Minuten werden 0,91 ml tert-Butylhydroperoxid 5-6 M in Decan zugetropft. Das Reaktionsgemisch wird bei -5°C, bis keine weitere Umsetzung erfolgt, weitergerührt und mit NH₄OH basisch gestellt. Die Wasserphase wird mit Dichlormethan gewaschen und gefriergetrocknet. 1 g **(IV-4)** wird als Feststoff erhalten. Analytische HPLC-MS (Methode A) RT = 0,85 min.

### 4.5 (1-{2-[5-(4-Fluorphenyl)-2,5-diazabicyclo[2.2.1]hept-2-yl]-5-oxo-6,7-dihydro-5H-5λ⁴-thieno[3,2-d]pyrimidin-4-ylamino}-cyclopropyl)-methanol (Beispiel 24)

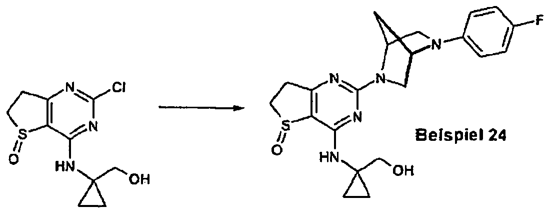

Ausgehend von 0,1 g **(IV-4)** (siehe 4.4) und 0,115 g-(4-Fluorphenyl)-2,5-diazabicyclo[2.2.1]heptan werden 0,142 g **Beispiel 24** analog zu Beispiel 21 (siehe 3.4) hergestellt. Das Produkt wird chromatographisch (präparative HPLC, Methode D) gereinigt. 0,142 g **Beispiel 24** werden als Feststoff erhalten. Analytische HPLC-MS (Methode A): RT = 1,08 min.

### 5. SYNTHESE VON (1-{2-[3-(4-BROMPHENYL)-3,8-DIAZABICYCLO[3.2.1]OCT-8-YL]-5-OXO-6,7-DIHYDRO-5H-5λ⁴-THIENO[3,2-d]PYRIMIDIN-4-YLAMINO}-CYCLOPROPYL)-METHANOL TRIFLUORACETAT (BEISPIEL 28)

### 5.1 3-(4-Bromphenyl)-3,8-diazabicyclo[3.2.1]octan (V-3)

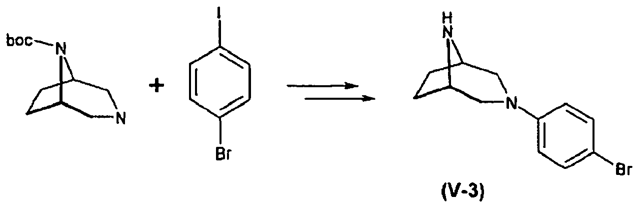

0,300 g 8-BOC-3,8-Diazabicyclo[3.2.1]octan, 0,402 g 4-Bromjodbenzol, 0,035 g Tris(dibenzylidenaceton)dipalladium(0), 0,064 g Xantphos und 0,660 g Cäsiumcarbonat werden in 6 ml wasserfreies und entgastes Dioxan und unter Argon 42 Stunden bei 110°C erhitzt. Das Reaktionsgemisch wird über Celite filtriert und das Eluat mit einer gesättigten Natriumchlorid Lösung versetzt. Das Produkt wird mit Essigester extrahiert. 0,610 g (1 R,5S)-3-(4-Brom-phenyl)-3,8-diazabicyclo[3.2.1]octane-8-BOC werden erhalten.

Das erhaltene Produkt wird analog zu **(V-2)** (siehe 3.3) weiter bearbeitet. 0,520 g **(V-3)** werden als Trifluoracetat erhalten. Analytische HPLC-MS (Methode B): RT= 1,26 min.

### 5.2 (1-{2-[3-(4-Bromphenyl)-3,8-diazabicyclo[3.2.1]oct-8-yl]-5-oxo-6,7-dihydro-5H-5λ⁴-thieno[3,2-d]pyrimidin-4-ylamino}-cyclopropyl)-methanol Trifluoracetat (Beispiel 28)

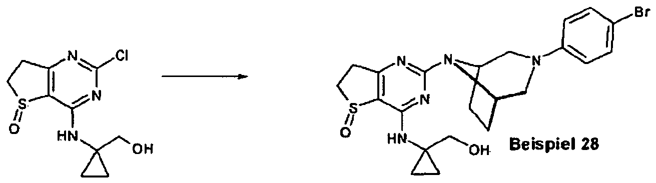

Ausgehend von 0,1 g **(IV-4)** (siehe 4.4) und 0,18 g **(V-3)** (siehe 5.1) werden 0,052 g **Beispiel 28** als Trifluoracetat analog zu Beispiel 21 (siehe 3.4) hergestellt. Das Produkt wird chromatographisch (präparative HPLC, Methode E) gereinigt. Analytische HPLC-MS (Methode B): RT = 1,37 min.

### 6. SYNTHESE VON [2-(5-BENZOOXAZOL-2-YL-2,5-DIAZABICYCLO[2.2.1]HEPT-2-YL)-5-OXO-6,7-DIHYDRO-5H-5λ⁴-THIENO[3,2-d]PYRIMIDIN-4-YL]-(TETRAHYDROPYRAN-4-YL)-AMIN (BEISPIEL 29)

### 6.1 (2-Chlor-6,7-dihydrothieno[3,2-d]pyrimidin-4-yl)-(tetrahydropyran-4-yl)-amin (III-5):

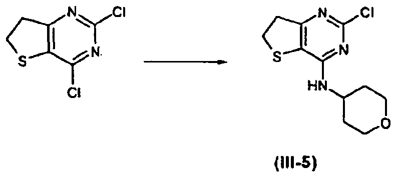

0,68 g **(II)** werden in 6 ml Dioxan vorgelegt, anschliessend werden 1,72 ml Diisopropylethylamin und dann 0,6 g 4-Aminotetrahydropyran zugegeben. Das Reaktionsgemisch wird bei 130°C, bis keine weitere Umsetzung erfolgt, erhitzt und nach Abkühlen eingedampft. Das Produkt wird mit Wasser im Ultraschallbad behandelt, abgesaugt und getrocknet 0,66 g **(III-5)** werden als Feststoff erhalten. Analytische HPLC-MS (Methode A): RT = 1,08 min.

### 6.2 (2-Chlor-5-oxo-6,7-dihydro-5H-5λ⁴-thieno[3,2-d]pyrimidin-4-yl)-(tetrahydropyran-4-yl)-amin (IV-5):

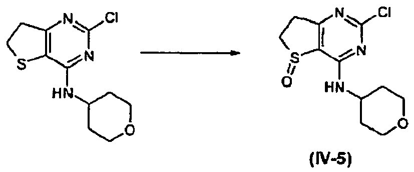

0,14 g S-(-)-1,1'-Bi-2-naphtol werden in 5 ml Chloroform unter Argon vorgelegt, dann 0,072 ml Titan(IV)-isopropylat und 0,087 ml Wasser zugegeben. Das Reaktionsgemisch wird 45 Minuten bei Raumtemperatur gerührt. Anschließend wird eine Suspension von 0,66 g **(III-5)** in 25 ml Chloroform zugegeben. Das Reaktionsgemisch wird auf -10°C abgekühlt und nach 60 Minuten werden 0,444 ml tert-Butylhydroperoxid 5-6 M in Decan zugetropft. Das Reaktionsgemisch wird bei -10 bis -4°C, bis keine weitere Umsetzung erfolgt, weitergerührt und mit Wasser versetzt. Das Produkt wird mit Dichlormethan extrahiert und chromatographisch (Kieselgel, Essigester/Methanol 100/0 bis 80/20) gereinigt. 0,42 g **(IV-5)** werden als Feststoff erhalten. Analytische HPLC-MS (Methode A): RT = 0,94 min.

### 6.3 (1S,4S)-2-(2,5-Diazabicyclo[2.2.1]hept-2-yl)-benzooxazol (V-4)

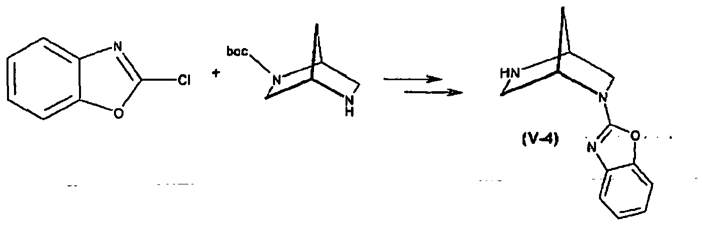

0,145 ml 2-Chlorbenzoxazol werden in 5 ml Chloroform vorgelegt, erst werden 0,225 ml Diisopropylethylamin, dann 0,250 g (1S,4S)-2,5-Diazabicyclo[2.2.1]heptan-2-BOC zugegeben. Das Reaktionsgemisch wird bei Raumtemperatur 15 Stunden gerührt, dann mit Wasser versetzt und das Produkt mit Chloroform extrahiert. 0,370 g **(V-4)** werden als Feststoff erhalten. Das erhaltene Produkt wird analog zu **(V-2)** (siehe 3.3) weiter bearbeitet. 0,320 g **(V-4)** werden als Trifluoracetat erhalten. Analytische HPLC-MS (Methode B): RT= 1,02 min.

### 6.4 [2-(5-Benzooxazol-2-yl-2,5-diazabicyclo[2.2.1]hept-2-yl)-5-oxo-6,7-dihydro-5H-5λ⁴-thieno[3,2-d]pyrimidin-4-yl]-(tetrahydropyran-4-yl)-amin (Beispiel 29)

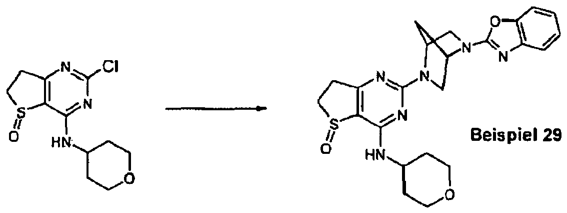

Ausgehend von 0,17 g **(IV-5)** (siehe 6.2) und 0,205 g **(V-4)** (siehe 6.3) werden 0,183 g **Beispiel 29** analog zu Beispiel 21 (siehe 3.4) hergestellt. Analytische HPLC-MS (Methode A): RT = 1,05 min.

### 7. SYNTHESE VON {1-[2-(5,6-DIHYDRO-8H-[1,2,4]TRIAZOLO[4,3-a]PYRAZIN-7-YL)-5-OXO-6,7-DIHYDRO-5H-5λ⁴-THIENO[3,2-d]PYRIMIDIN-4-YLAMINO]-CYCLOPROPYL}-METHANOL TRIFLUORACETAT (BEISPIEL 33)

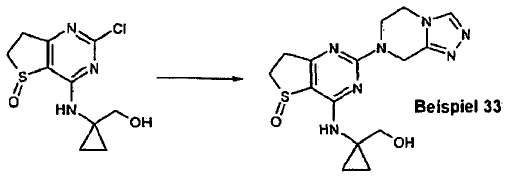

**(IV-4)** (siehe 4.4) (0,1 mmol) wird in 750 µl N-Methyl-2-pyrrolidon (NMP) und 50 µl Diisopropylethylamin vorgelegt, mit einer Lösung von 5,6,7,8-Tetrahydro-[1,2,4]triazolo[4,3-a]pyrazin (0,1 mmol) in 400µl NMP versetzt und 30 min bei 120°C in der Mikrowelle erhitzt. Anschliessend werden 600 µL DMF zugesetzt, die Reaktionslösung über präparative HPLC-MS (Methode A) aufgereinigt und die Produktfraktionen gefriergetrocknet. Beispiel 33 wird als Trifluoracetat erhalten. Analytische HPLC-MS (Methode C): RT = 1,62 min.

### 8. SYNTHESE VON [2-(5,6-DIHYDRO-8H-[1,2,4]TRIAZOLO[4,3-a]PYRAZIN-7-YL)-5-OXO-6,7-DIHYDRO-5H-5λ⁴-TH[ENO[3,2-d]PYRIMIDIN-4-YL]-(TETRAHYDRO-PYRAN-4-YL)-AMIN TRIFLUORACETAT (BEISPIEL 37)

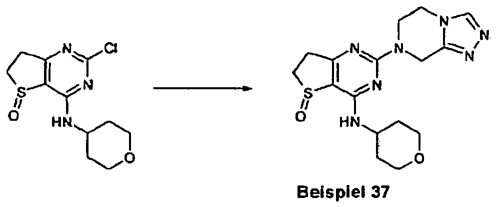

Ausgehend von **(IV-5)** (siehe 6.2) und 5,6,7,8-Tetrahydro-[1,2,4]triazolo[4,3-*a*]pyrazin wird **Beispiel 37** als Trifluoracetat analog zu Beispiel 33 (siehe 7) hergestellt und aufgereinigt. Analytische HPLC-MS (Methode C): RT = 1,35 min.

### 9. SYNTHESE VON (3-FLUORPHENYL)-{2-[3-(4-FLUORPHENYL)-5,6-DIHYDRO-8H-[1,2,4]TRIAZOLO[4,3-a]PYRAZIN-7-YL]-5-OXO-6,7-DIHYDRO-5H-5λ⁴-THIENO[3,2-d]PYRIMIDIN -4-YL}-AMIN TRIFLUORACETAT (BEISPIEL 39)

### 9.1 3-(4-Fluorphenyl)-5,6,7,8-tetrahydro-[1,2,4]triazolo[4,3-a]pyrazin (V-5)

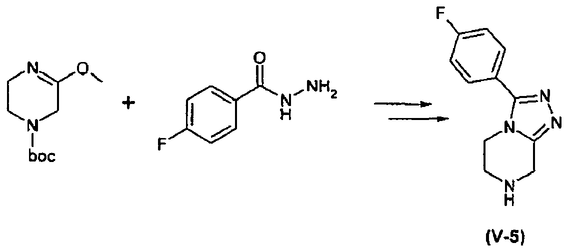

2,9 g 5-Methoxy-3,6-dihydro-2*H*-pyrazin-1-BOC (EP1477490), 2,1 g 4-Fluorbenzhydrazid werden in 50 ml Ethanol und 50 ml Eisessig suspendiert. Das Reaktionsgemisch wird bei 70°C, bis keine weitere Umsetzung erfolgt, erhitzt, dann zur Trockne eingedampft. Der Rückstand wird in 10 ml Dichlormethan und 10 ml Trifluoressigsäure suspendiert, 4 Stunden bei 40°C gerührt und zur Trockne eingedampft. Der Rückstand wird in Diethylether suspendiert und der Feststoff abgesaugt. 3,1 g **(V-5)** werden als Trifluoracetat erhalten. ¹H. NMR (400 MHz, DMSO): 4,61 (2H, s); 4,34 (2H, t); 3,61 (2H, t).

### 9.2 (3-Fluorphenyl)-{2-[3-(4-fluorphenyl)-5,6-dihydro-8H-[1,2,4]triazolo[4,3-a]pyrazin-7-yl]-5-oxo-6,7-dihydro-5H-5λ⁴-thieno[3,2-d]pyrimidin-4-yl}-amin (Beispiel 39)

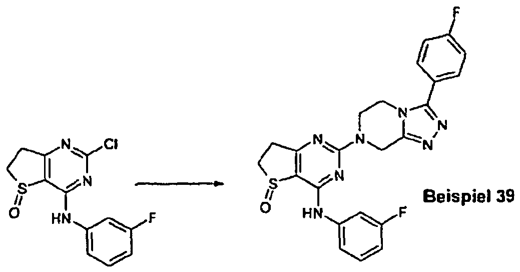

Ausgehend von **(IV-3)** (siehe 3.2) und **(V-5)** (siehe 9.1) wird **Beispiel 39** als Trifluoracetat analog zu Beispiel 33 (siehe7) hergestellt und aufgereinigt. Analytische HPLC-MS (Methode C): RT = 1,89 min.

### 10. SYNTHESE VON {2-[3-(3,4-DICHLORBENZYL)-5,6-DIHYDRO-8H-[1,2,4]TRIAZOLO[4,3-a]PYRAZIN-7-YL]-5-OXO-6,7-DIHYDRO-5H-5λ⁴-THIENO[3,2-d]PYRIMIDIN-4-YL}-(3-FLUORPHENYL)-AMIN TRIFLUORACETAT (BEISPIEL 40)

### 10.1 3-(3,4-Dichlorbenzyl)-5,6,7,8-tetrahydro-[1,2,4]triazolo[4,3-a]pyrazin (V-6)

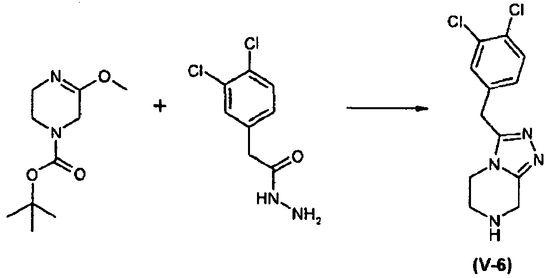

Ausgehend von 2,9 g 5-Methoxy-3,6-dihydro-2*H*-pyrazin-1-BOC (EP1477490), und 3 g (3,4-Dichlorphenyl)-essigsäure hydrazid werden analog zu **(V-5)** (siehe 9.1) 3,9 g **(V-6)** als Trifluoracetat erhalten. ¹H NMR (400 MHz, DMSO): 4,51 (2H, s); 4,22 (2H, s); 4,03 (2H, t); 3,57 (2H, t).

### 10.2 {2-[3-(3,4-Dichlorbenzyl)-5,6-dihydro-8H-[1,2,4]triazolo[4,3-a]pyrazin-7-yl]-5-oxo-6,7-dihydro-5H-5λ⁴-thieno[3,2-d]pyrimidin-4-yl}-(3-fluorphenyl)-amin (Beispiel 40)

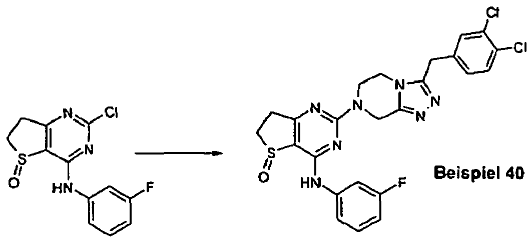

Ausgehend von **(IV-3)** (siehe 3.2) und **(V-6)** (siehe 10.1) wird **Beispiel 40** als Trifluoracetat analog zu Beispiel 33 (siehe 7.) hergestellt und aufgereinigt. Analytische HPLC-MS (Methode C): RT = 2,02 min.

### 11. SYNTHESE VON [2-(5,6-DIHYDRO-8H-[1,2,4]TRIAZOLO[4,3-a]PYRAZIN-7-YL)-5-OXO-6,7-DIHYDRO-5H-5λ⁴-THIENO[3,2-d]PYRIMIDIN-4-YL]-(3-FLUORPHENYL)-AMIN TRIFLUORACETAT (BEISPIEL 41)

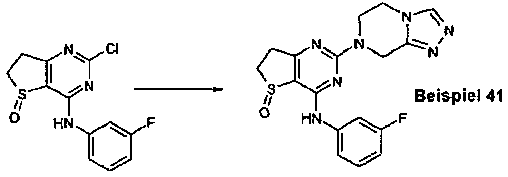

Ausgehend von **(IV-3)** (siehe 3.2) und 5,6,7,8-Tetrahydro-[1,2,4]triazolo[4,-*a*]pyrazin wird **Beispiel 41** als Trifluoracetat analog zu Beispiel 33 (siehe 7.) hergestellt und aufgereinigt. Analytische HPLC-MS (Methode C): RT = 1,6 min.

### 12. SYNTHESE VON (R)-2-[2-(5,6-DIHYDRO-8H-[1,2,4]TRIAZOLO[4,3-a]PYRAZIN-7-YL)-5-OXO-6,7-DIHYDRO-5H-5λ⁴-THIENO[3,2-d]PYRIMIDIN-4-YLAMINO]-3-METHYLBUTAN-1-OL TRIFLUORACETAT (BEISPIEL 45)

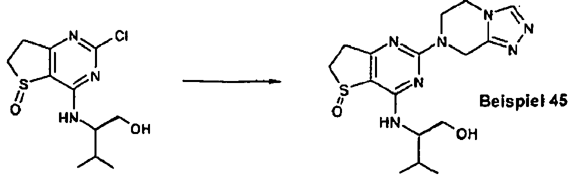

Ausgehend von **(IV-1)** (siehe 1.2) und 5,6,7,8-Tetrahydro[1,2,4]triazolo[4,3-*a*]pyrazin wird **Beispiel 45** als Trifluoracetat analog zu Beispiel 33 (siehe 7.) hergestellt und aufgereinigt. Analytische HPLC-MS (Methode C): RT = 1,35 min.

### 13. SYNTHESE VON MORPHOLIN-4-YL-{7-[5-OXO-4-(TETRAHYDROPYRAN-4-YLAMINO)-6,7-DIHYDRO-5H-5λ⁴-THIENO[3,2-d]PYRIMIDIN-2-YL]-5,6,7,8-TETRAHYDROIMIDAZO[1,2-a]PYRAZIN-2-YL}-METHANON (BEISPIEL 48)

### 13.1 7-[5-Oxo-4-(tetrahydropyran-4-ylamino)-6,7-dihydro-5H-5λ⁴-thieno[3,2-d]pyrimidin-2-yl]-5,6,7,8-tetrahydroimidazo[1,2-a]pyrazine-2-carbonsäure

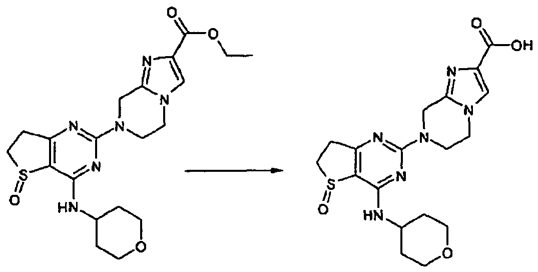

1,18 g **Beispiel 34** werden in 34,7 ml Ethanol suspendiert und 9 ml NaOH 1N zugegeben. Das Reaktionsgemisch wird 12 Stunden bei Raumtemperatur gerührt, mit 1N HCl neutralisiert und zur Trockne eingedampft. Das Produkt wird über präparative HPLC (Methode B) gereinigt. 550 mg Produkt werden als Feststoff erhalten. Analytische HPLC-MS (Methode D): RT = 0.36 min.

### 13.2 Morpholin-4-yl-{7-[5-oxo-4-(tetrahydropyran-4-ylamino)-6,7-dihydro-5H-5λ⁴-thieno[3,2-d]pyrimidin-2-yl]-5,6,7,8-tetrahydroimidazo[1,2-a]pyrazin-2-yl}-methanon

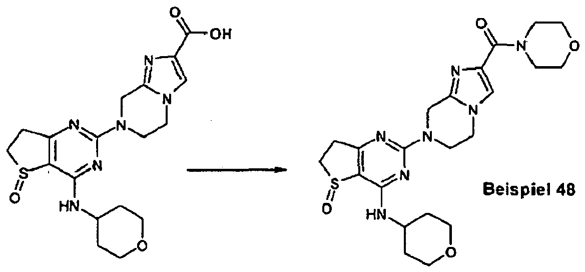

50 mg 7-[5-Oxo-4-(tetrahydropyran-4-ylamino)-6,7-dihydro-5H-5λ⁴-thieno[3,2-*d*]pyrimidin-2-yl]-5,6,7,8-tetrahydroimidazo[1,2-a]pyrazine-2-carbonsäure (siehe 13.1) werden in 2 ml. Dimethylformamid vorgelegt, dann 66 µl Diisopropylethylamin und 54,2 mg O-(7-Azabenzotriazol-1-yl-)-N,N,N',N'-tetramethyluroniumhexafluorphosphat (HATU) zugegeben. Nach 15 min wird eine Suspension von 11,4 mg Morpholin in 300 µl Dimethylformamid zugegeben. Das Reaktionsgemisch wird bei Raumtemperatur, bis keine weitere Reaktion erfolgt, gerührt und das Produkt direkt über präparative HPLC (Methode B) gereinigt. 40 mg

### Beispiel 48 werden als Feststoff erhalten. Analytische HPLC-MS (Methode D): RT = 0,79 min.

### CHROMATOGRAPHIE METHODEN

Die nach den obigen Synthese-Schema hergestellten Beispielverbindungen wurden durch folgende chromatographische Methoden, die - sofern sie durchgeführt wurden - im einzelnen in den Tabellen B, C und D angegeben sind, charakterisiert.

### Analytische HPLC-MS, Methode A

Waters ZMD Masse Spektrometer (positive Ionisation (ESI+)), Alliance 2690/2695 HPLC (Diodenarraydetektor, Wellenlängenbereich: 210 to 500 nm), Waters 2700 Autosampler, Waters 996/2996.
A: Wasser mit 0.10% TFA
B: Acetonitril mit 0.10% TFA

| Zeit in min | %A | %B | Flussrate in ml/min |
|---|---|---|---|
| 0.00 | 95 | 5 | 2.50 |
| 0.20 | 95 | 5 | 2.50 |
| 1.50 | 2 | 98 | 2.50 |
| 1.70 | 2 | 98 | 2.50 |
| 1.90 | 95 | 5 | 2.50 |
| 2.20 | 95 | 5 | 2.50 |

Als stationäre Phase dient eine Säule Merck Chromolith^{™} Flash RP-18e, 4.6 mm x 25 mm (Säulentemperatur: konstant bei 25°C).

### Analytische HPLC-MS, Methode B

Waters ZMD Masse Spektrometer (positive Ionisation (ESI+)), Alliance 2690/2695 HPLC (Diodenarraydetektor, Wellenlängenbereich: 210 to 500 nm), Waters 2700 Autosampler, Waters 996/2996.
A: Wasser mit 0.10% TFA
B: Acetonitril mit 0.10% TFA

| Zeit in min | %A | %B | Flussrate in ml/min |
|---|---|---|---|
| 0.00 | 95 | 5 | 2.80 |
| 0.30 | 95 | 5 | 2.80 |
| 1.60 | 2 | 98 | 2.80 |
| 1.90 | 2 | 98 | 2.80 |
| 2.00 | 95 | 5 | 2.50 |

Als stationäre Phase dient eine Säule Merck Chromolith^{™} Flash RP-18e, 3 mm x 100 mm (Säulentemperatur: konstant bei 25°C).

### Analytische HPLC-MS, Methode C:

Waters ZQ2000 Massenspektrometer (positive Ionisation (ESI+)), HP1100 HPLC (DAD, Wellenlängenbereich: 210 to 500 nm), and Gilson 215 Autosampler.
A: Wasser mit 0.10% TFA
B: Acetonitril mit 0.10°% TFA

| Zeit in min | %A | %B | Flussrate in ml/min |
|---|---|---|---|
| 0.00 | 95 | 5 | 1.50 |
| 2.00 | 0 | 100 | 1.50 |
| 2.50 | 0 | 100 | 1.50 |
| 2.60 | 95 | 5 | 1.50 |

Als stationäre Phase dient eine Säule Sunfire C18, 4,6 X 50mm, 3,5 µm, Säulentemperatur 40°C.

### Analytische HPLC-MS, Methode D

Waters ZMD Masse Spektrometer (positive Ionisation (ESI+)), Alliance 2690/2695 HPLC (Diodenarraydetektor, Wellenlängenbereich: 210 to 500 nm), Waters 2700 Autosampler, Waters 996/2996.
A: Wasser mit 0.10% NH₃
B: Acetonitril mit 0.10% NH₃

| Zeit in min | %A | %B | Flussrate in ml/min |
|---|---|---|---|
| 0.00 | 95 | 5 | 3.00 |
| 0.20 | 95 | 5 | 3.00 |
| 1.50 | 2 | 98 | 3.00 |
| 1.90 | 2 | 98 | 3.00 |
| 2.00 | 2 | 98 | 3.00 |

Als stationäre Phase dient Waters, X-Bridge, C18, 3,5 nm, 4,6 X 20 mm. Raumtemperatur

### Präparative HPLC-MS, Methode A

Waters ZQ2000 Masse Spektrometer (positive Ionisation (ESI+)), HP1100 HPLC (DAD, Wellenlängenbereich: 210 - 500 nm), and Gilson 215 Autosampler.
A: Wasser mit 0.10% TFA
B: Acetonitril

| Zeit in min | %A | %B | Flussrate in ml/min |
|---|---|---|---|
| 0.00 | 90 | 10 | 50 |
| 1.50 | 90 | 10 | 50 |
| 8.00 | 40 | 60 | 50 |
| 10.00 | 40 | 60 | 50 |
| 11.00 | 90 | 10 | 50 |

Als stationäre Phase dient eine Säule Sunfire C18, 30 X 100 mm, 5 µm, Raumtemperatur.

### Präparative HPLC, Methode A

Gilson HPLC mit Gilson UV-VIS-155 Detektor, Sampling Injektor 231 XL.

Als Wellenlänge wird das substanzspezifische UV-Maximum angegeben.
A: Wasser mit 0.13% TFA
B: Acetonitril mit 0,1% TFA

| Zeit in min | %A | %B | Flussrate in ml/min |
|---|---|---|---|
| 0.00 | 90 | 10 | 120 |
| 3.60 | 90 | 10 | 120 |
| 17.80 | 0 | 100 | 120 |
| 22.00 | 0 | 100 | 120 |
| 23.00 | 90 | 10 | 120 |
| 26.50 | 90 | 10 | 120 |

Als stationäre Phase dient eine Säule Microsorb RP 18, 8 µm, 41,4 X 250 mm, Raumtemperatur.

### Präparative HPLC, Methode B

Gilson HPLC mit Gilson UV-VIS-155 Detektor, Sampling Injektor 231 XL.

Als Wellenlänge wird das substanzspezifische UV-Maximum angegeben.
A: Wasser mit 0.15% Ammoniak 35%
B: Acetonitril mit 0,15% Ammoniak 35%

| Zeit in min | %A | %B | Flussrate in ml/min |
|---|---|---|---|
| 0.00 | 90 | 10 | 120 |
| 3.00 | 90 | 10 | 120 |
| 15.00 | 0 | 100 | 120 |
| 18.00 | 0 | 100 | 120 |
| 18.50 | 90 | 10 | 120 |
| 21.00 | 90 | 10 | 120 |

Als stationäre Phase dient eine Säule Varian Xbridge C 18, 5 µm, 50 X 162 mm, Raumtemperatur.

### Präparative HPLC, Methode C

Gilson HPLC mit Gilson UV-VIS-155 Detektor, Sampling Injektor 231 XL.

Als Wellenlänge wird das substanzspezifische UV-Maximum angegeben.
A: Wasser mit 0.13% TFA
B: Acetonitril mit 0, 1 % TFA

| Zeit in min | %A | %B | Flussrate in ml/min |
|---|---|---|---|
| 0.00 | 95 | 5 | 165 |
| 1.30 | 95 | 5 | 165 |
| 8.90 | 2 | 98 | 165 |
| 10.00 | 2 | 98 | 165 |
| 10.50 | 95 | 5 | 165 |
| 11.60 | 95 | 5 | 165 |

Als stationäre Phase dient eine Säule Microsorb RP 18, 8 µm, 50 X 65 mm, Raumtemperatur.

### Präparative HPLC, Methode D

Gilson HPLC mit Gilson UV-VIS-155 Detektor, Sampling Injektor 231 XL.

Als Wellenlänge wird das substanzspezifische UV-Maximum angegeben.
A: Wasser mit 0.1 % Ammoniak 35%ig
B: Acetonitril

| Zeit in min | %A | %B | Flussrate in ml/min |
|---|---|---|---|
| 0.00 | 95 | 5 | 180 |
| 1.40 | 95 | 5 | 180 |
| 17.00 | 2 | 98 | 180 |
| 18.50 | 2 | 98 | 180 |
| 18.70 | 95 | 5 | 180 |
| 20.-50 | 95 | 5 | 180 |

Als stationäre Phase dient eine Säule Pursuit XRS RP 18, 10 µm, 50 X 150 mm, Raumtemperatur.

### Präparative HPLC, Methode E

Gilson HPLC mit Gilson UV-VIS-155 Detektor, Sampling Injektor 231 XL.

Als Wellenlänge wird das substanzspezifische UV-Maximum angegeben.
A: Wasser mit 0.1 % TFA
B: Acetonitril mit 0,13% TFA

| Zeit in min | %A | %B | Flussrate in ml/min |
|---|---|---|---|
| 0.00 | 95 | 5 | 180 |
| 1.40 | 95 | 5 | 180 |
| 17.00 | 2 | 98 | 180 |
| 18.50 | 2 | 98 | 180 |
| 18.70 | 95 | 5 | 180 |
| 20.-50 | 95 | 5 | 180 |

Als stationäre Phase dient eine Säule Microsorb RP 18, 100A, 8 µm, 50 X 150 mm, Raumtemperatur.

### BEISPIELE

Die folgenden Beispiele wurden analog den oben gezeigten Synthesevorschriften herstellt (je nach Kennzeichnung in der Tabelle). Diese Verbindungen sind als PDE4-Inhibitoren geeignet und besitzen IC₅₀-Werte kleiner oder gleich 1 µmol. Die Inhibitionen (in %) bei 1 µM der einzelnen Beispiel-Substanzen sind in den nachfolgenden Beispiel-Tabellen eingefügt und wurden wie folgt bestimmt:

Bei der Durchführung des Scintilation Proximity (SPA) Assays (GE Healthcare, Nr. TRKQ7090) werden die unterschiedlichen Affinitäten des cyklischen 3'-5'-Adenosinmonophosphates (cAMP, niedrige Affinität) und des linearen 5'-Adenosinmonophosphates (AMP, hohe Affinität) an Yttrium-Silicat-Scintillatorbeads ausgenutzt. Die cAMP spezifische Phosphodiesterase (PDE) PDE4B spaltet die 3'-Phosphoesterbindung des Tritiummarkierten [H3]-cAMP zum [H3]-5'-AMP. Dieses [H3]-AMP lagert sich aufgrund der höheren Affinität zu den Scintillatorbeads an diese an und verursacht Scintillationsereignisse (Lichtblitze) welche in einem *Wallac Microbeta Scintillation Counter* gemessen werden.

Der Versuch startet mit einer einstündigen Inkubation von [H3]-cAMP mit dem PDE4B Enzym in Assaypuffer bei 30°C, jeweils einmal mit der zu testenden Beispiel-Substanz (in einer Konzentation von 1µM) und einmal ohne die zu testende Beispiel-Substanz .

Nach dieser Inkubation wird die Reaktion durch Zugabe der Beads gestoppt. Die Beads erhalten in den folgenden 45 Minuten Gelegenheit, sich abzusetzen, danach wird im Scintillation Counter gemessen. Ist die Substanz in der Lage, die enzymatische Aktivität der PDE4B zu hemmen, so entsteht während der Inkubationsphase weniger [H3]-AMP und es sind weniger Scintillationsereignisse messbar. Ausgedrückt werden diese Ergebnisse als Prozent Inhibition bei einer Konzentration der Test-Substanz von 1µM.

Bei den Beispielen handelt es sich um Verbindungen der folgenden Formel 1, 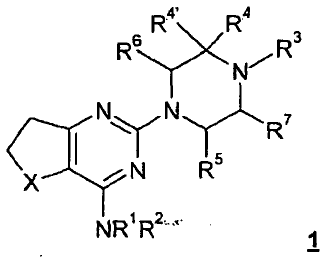 mit den in den folgenden Tabellen A und B bezeichneten Eigenschaften:

**Tabelle A: Chemische Strukturen der Beispiel-Substanzen 1 - 29**

| # | Struktur | R¹ | R² | R³ | R⁴ | R^{4'} | R⁵ | R⁶ | R⁷ | % Inhibition PDE4B @ 1 µm |
|---|---|---|---|---|---|---|---|---|---|---|
| 1 | 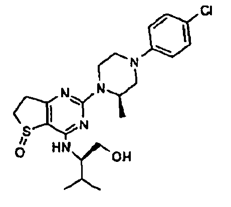 | H | 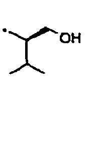 | 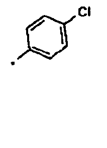 | H | H | CH₃ | H | H | 84 |
| 2 | 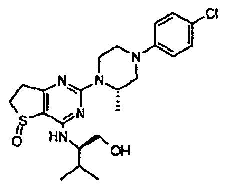 | H | 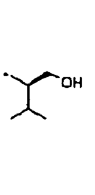 | 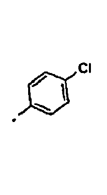 | H | H | CH₃ | H | H | 94 |
| 3 | 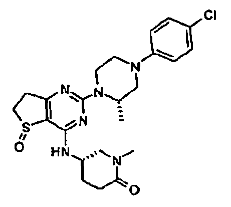 | H | 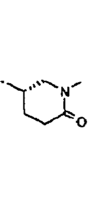 | 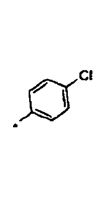 | H | H | CH₃ | H | H | 96 |
| 4 | 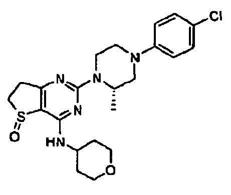 | H | 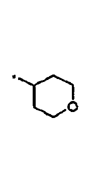 | 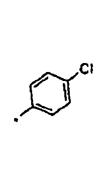 | H | H | CH₃ | H | H | 95 |
| 5 | 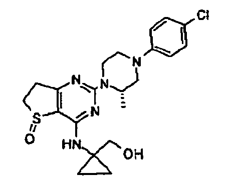 | H | 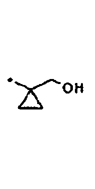 | 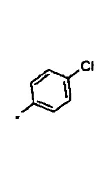 | H | H | CH₃ | H | H | 95 |
| 6 | 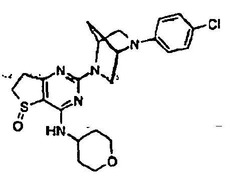 | H | 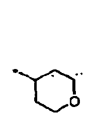 | 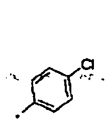 | H | H | H | bilden gemeinsam -CH₂- | | 95 |

| # | Struktur | R¹ | R² | R³ | R⁴ | R⁴' | R⁵ | R⁶ | R⁷ | % Inhibition PDE4B @ 1 µM |
|---|---|---|---|---|---|---|---|---|---|---|
| 7 | 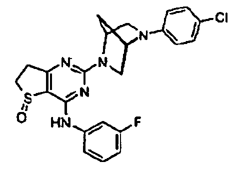 | H | 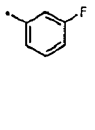 | 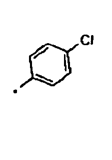 | H | H | H | bilden gemeinsam -CH₂- | | 95 |
| 8 | 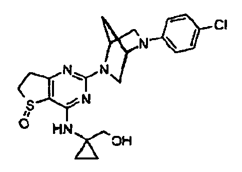 | H | 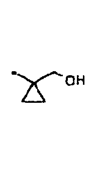 | 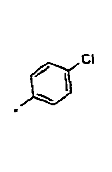 | H | H | H | bilden gemeinsam -CH₂- | | 95 |
| 9 | 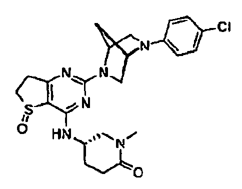 | H | 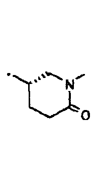 | 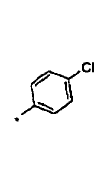 | H | H | H | bilden gemeinsam -CH₂- | | 96 |
| 10 | 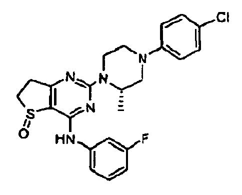 | H | 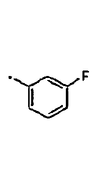 | 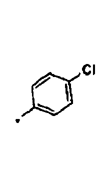 | H | H | CH₃ | H | H | 94 |
| 11 | 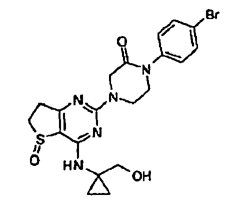 | H | 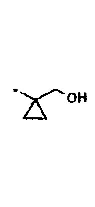 | 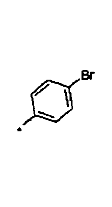 | bilden gemeinsam eine Oxo-Gruppe | | H | H | H | 90 |
| 12 | 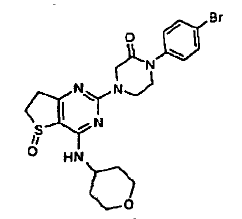 | H | 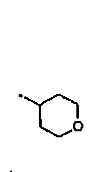 | 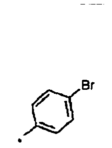 | bilden gemeinsam eine Oxo-Gruppe | | H | H | H | 93 |
| 13 | 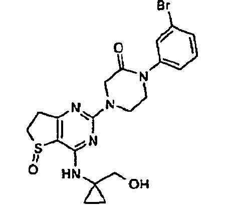 | H | 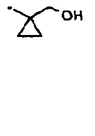 | 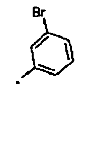 | bilden gemeinsam eine Oxo-Gruppe | | H | H | H | 19 |
| 14 | 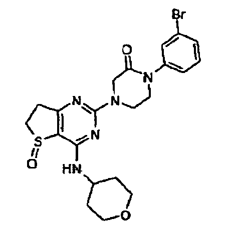 | H | 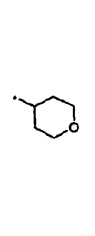 | 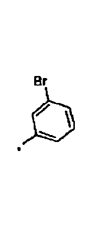 | bilden gemeinsam eine Oxo-Gruppe | | H | H | H | 92 |
| 15 | 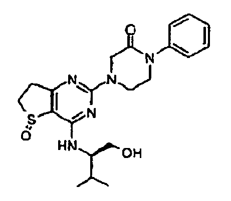 | H | 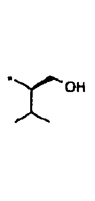 | 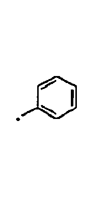 | bilden gemeinsam eine Oxo-Gruppe | | H | H | H | 77 |
| 16 | 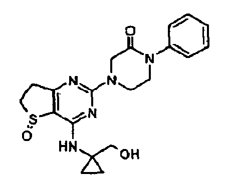 | H | 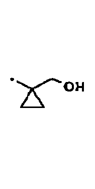 | 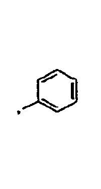 | bilden gemeinsam eine Oxo-Gruppe | | H | H | H | 86 |
| 17 | 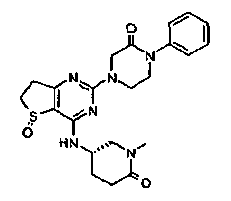 | H | 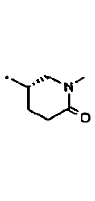 | 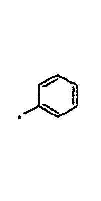 | bilden gemeinsam eine Oxo-Gruppe | | H | H | H | 96 |
| 18 | 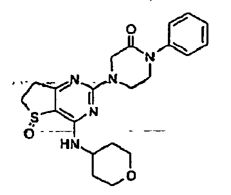 | H | 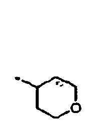 | 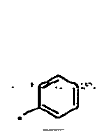 | bilden gemeinsam eine Oxo-Gruppe | | H | H | H | 93 |
| 19 | 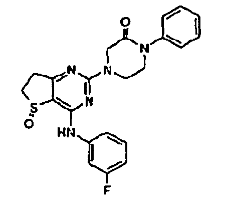 | H | 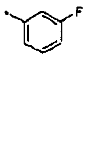 | 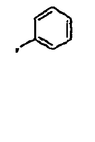 | bilden gemeinsam eine Oxo-Gruppe | | H | H | H | 95 |
| 20 | 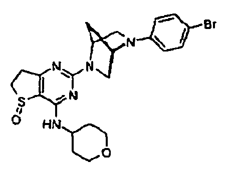 | H | 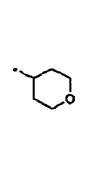 | 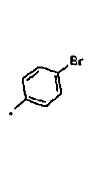 | H | H | H | bilden gemeinsam -CH₂- | | 95 |
| 21 | 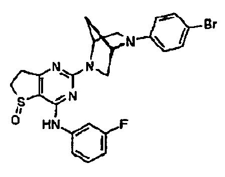 | H | 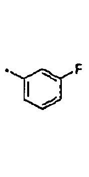 | 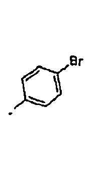 | H | H | H | bilden gemeinsam -CH₂- | | 95 |
| 22 | 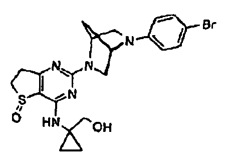 | H | 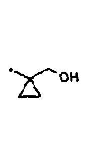 | 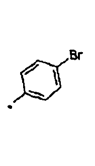 | H | H | H | bilden gemeinsam -CH₂- | | 95 |
| 23 | 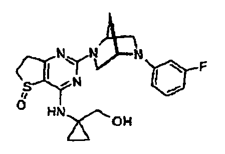 | H | 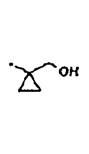 | 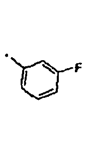 | H | H | H | bilden gemeinsam -CH₂- | | 93 |
| 24 | 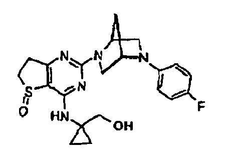 | H | 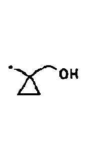 | 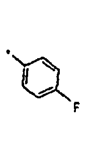 | H | H | H | bilden gemeinsam -CH₂- | | 93 |
| 25 | 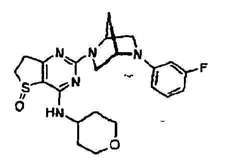 | H | 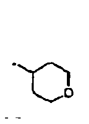 | 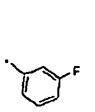 | H | H | H | bilden gemeinsam -CH₂- | | 94 |
| 26 | 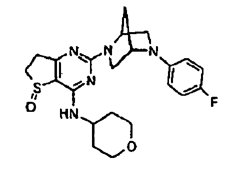 | H | 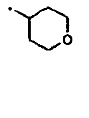 | 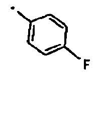 | H | H | H | bilden gemeinsam -CH₂- | | 94 |
| 27 | 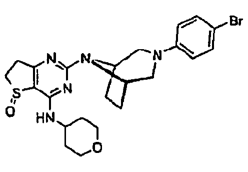 | H | 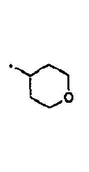 | 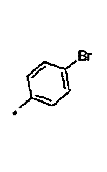 | H | H | bilden gemeinsam -CH₂-CH₂- | | H | 97 |
| 28 | 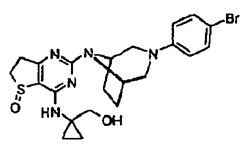 | H | 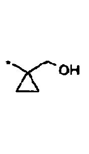 | 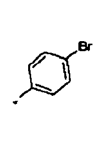 | H | H | bilden gemeinsam -CH₂-CH₂- | | H | 97 |
| 29 | 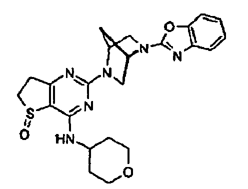 | H | 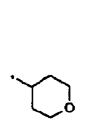 | 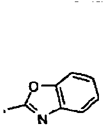 | H | H | H | bilden gemeinsam -CH₂-CH₂- | | 97 |

In der folgenden Tabelle B werden Detail-Informationen zu den chemischen Synthesen und der Analytik der einzelnen Beispiel-Substanzen 1-29 zusammengefasst.

**Tabelle B: Detail-Informationen zu den Herstellungen der einzelnen Beispiel-Substanzen 1-29**

| # | Struktur | Herstellung analog zu #* | nicht käuflicher Arylpiperazin-Baustein **(V)** | Literatur zur Herstellung des nicht käuflichen Arylpiperazin-Bausteins **(V)** | Analytische HPLC-MS, RT [min], Methode |
|---|---|---|---|---|---|
| 1 | 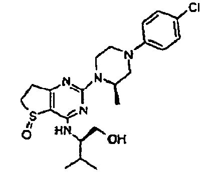 | siehe experim. Teil | | | 1,26 Methode A |
| 2 | 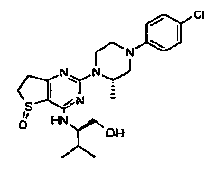 | 1 | 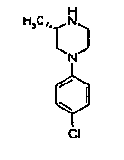 | Tet. Asymm. 2002, 2303 | 1,20 Methode A |
| 3 | 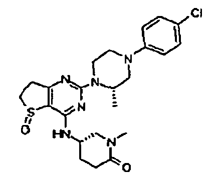 | 9 | 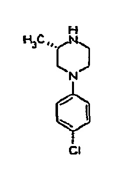 | Tet. Asymm. 2002, 2303 | 1,21 Methode A |
| 4 | 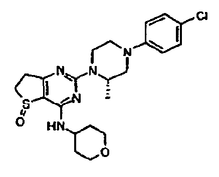 | 29 | 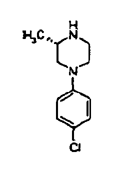 | Tet. Asymm. 2002, 2304 | 1,27 Methode A |
| 5 | 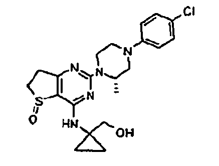 | 24 | 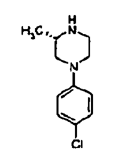 | Tet. Asymm. 2002, 2305 | 1,24 Methode A |
| 6 | 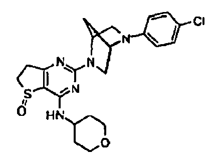 | 29 | | | 1,16 Methode A |
| 7 | 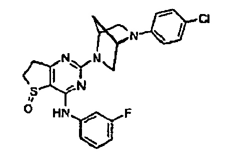 | 21 | | | 1,32 Methode A |
| 8 | 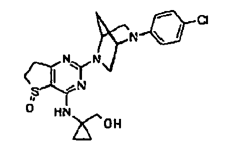 | 24 | | | 1,10 Methode A |
| 9 | 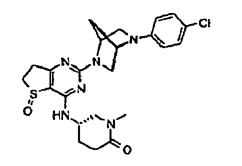 | siehe experim. Teil | | | 1,13 Methode A |
| 10 | 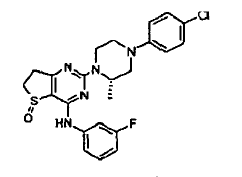 | 21 | 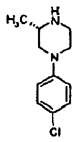 | Tet. Asymm. 2002, 2305 | 1,47 Methode A |
| 11 | 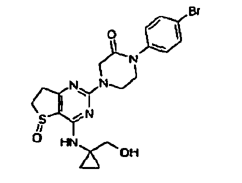 | 24 | | | 1,00 Methode D |
| 12 | 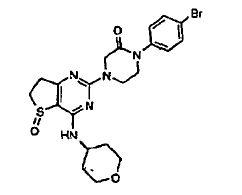 | 29 | | | 1,04 Methode D |
| 13 | 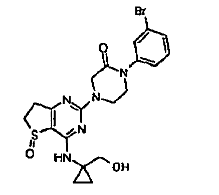 | 24 | | | 1,00 Methode D |
| 14 | 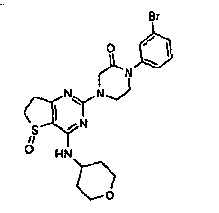 | 29 | | | 1,04 Methode D |
| 15 | 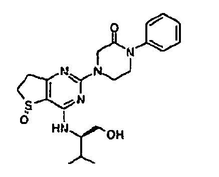 | 1 | | | 1,61 Methode C |
| 16 | 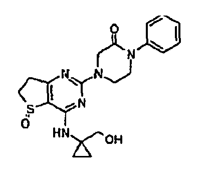 | 24 | | | 1,61 Methode C |
| 17 | 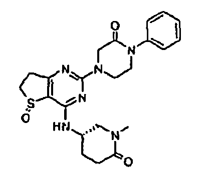 | 9 | | | 1,61 Methode C |
| 18 | 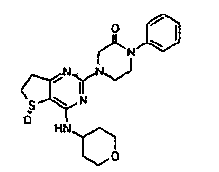 | 29 | | | 1,61 Methode C |
| 19 | 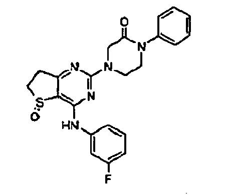 | 21 | | | 1,61 Methode C |
| 20 | 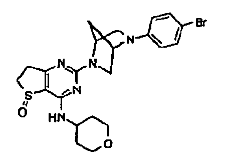 | 29 | 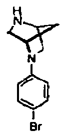 | siehe experim. Teil § 3.3, Baustein **(V-2)** | 1,19 Methode A |
| 21 | 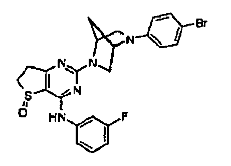 | siehe experim. Teil | | | 1,19 Methode A |
| 22 | 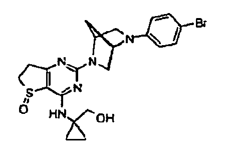 | 24 | 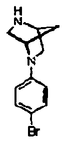 | siehe experim. Teil § 3.3, Baustein **(V-2)** | 1,16 Methode A |
| 23 | 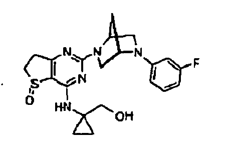 | 24 | | | 1,10 Methode A |
| 24 | 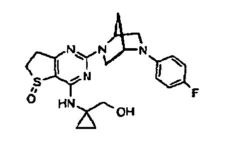 | siehe experim. Teil | | | 1,08 Methode A |
| 25 | 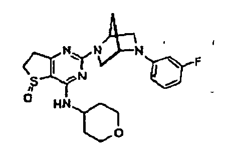 | 29 | | | 1,16 Methode A |
| 26 | 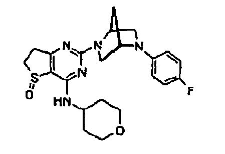 | 29 | | | 1,14 Methode A |
| 27 | 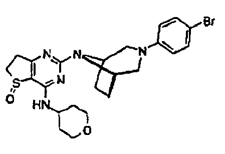 | 29 | 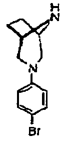 | siehe experim. Teil § 5.1, Baustein **(V-3)** | 1,40 Methode B |
| 28 | 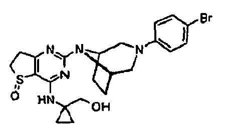 | siehe experim. Teil | | | 1,37 Methode B |
| 29 | 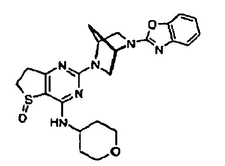 | siehe experim. Teil | | | 1,05 Methode A |

| | | | | | |
|---|---|---|---|---|---|
| * Das Beispiel kann analog hergestellt und aufgereinigt werden. | | | | | |

Bei den Beispielen handelt es sich weiterhin um folgende Verbindungen 30 - 45 der allgemeinen Formel 1, die in Tabelle C aufgezählt sind:

**Tabelle C: Chemische Strukturen und Details zur Herstellung der Beispiel-Substanzen 30-45**

| # | Struktur | Herstellung analog zu #* | nicht käuflicher Arylpiperazin-Baustein **(V)** | Literatur zur Herstellung des nicht käuflichen Arylpiperazin-Bausteins **(V)** | Analytische HPLC-MS, RT [min], Methode | % Inhibition PDE4B @ 1 µM |
|---|---|---|---|---|---|---|
| 30 | 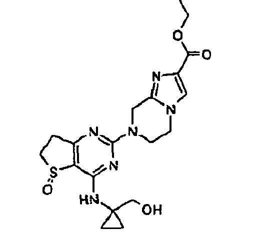 | 33 | | | 1,48 Methode C | 95 |
| 31 | 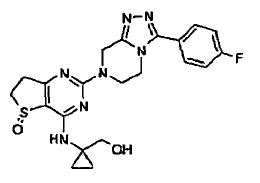 | 33 | 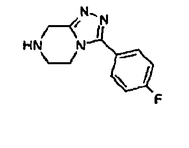 | siehe experim. Teil § 9.1, Baustein **(V.5)** | 1,61 Methode C | 93 |
| 32 | 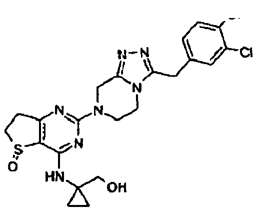 | 33 | 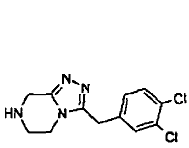 | siehe experim. Teil § 10.1, Baustein **(V-6)** | 1,76 Methode C | 91 |
| 33 | 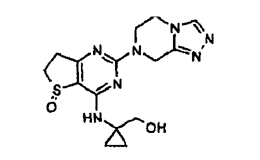 | siehe experim. Teil | | | 1,62 Methode C | 75 |
| 34 | 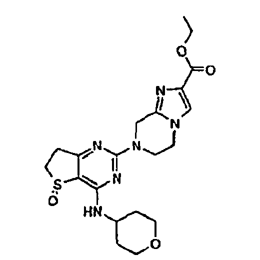 | 37 | | | 1,51 Methode C | 95 |
| 35 | 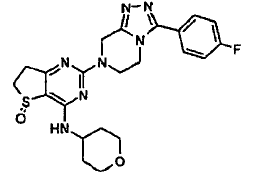 | 37 | 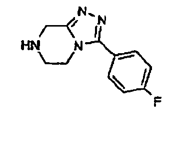 | siehe experim. Teil § 9.1, Baustein **(V-5)** | 1,65 Methode C | 94 |
| 36 | 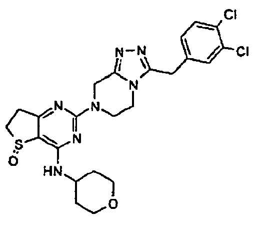 | 37 | 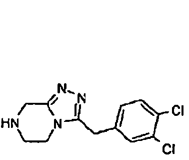 | siehe experim. Teil § 10.1, Baustein **(V-6)** | 1,79 Methode C | 93 |
| 37 | 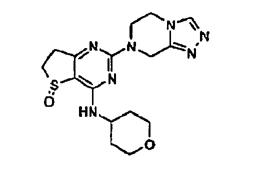 | siehe experim. Teil | | | 1,35 Methode C | 87 |
| 38 | 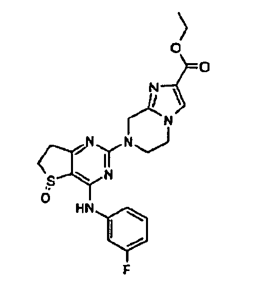 | 39 | | | 1,74 Methode C | 95 |
| 39 | 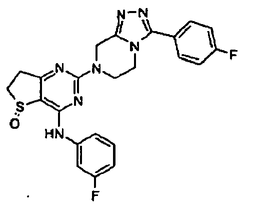 | siehe experim. Teil | | | 1,89 Methode C | 95 |
| 40 | 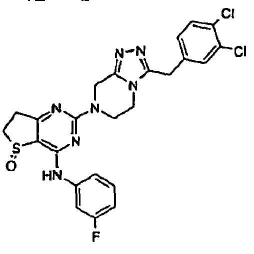 | siehe experim. Teil | | | 2,02 Methode C | 94 |
| 41 | 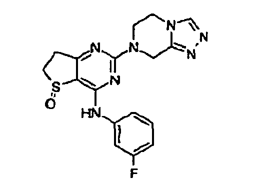 | siehe experim. Teil | | | 1,6 Methode C | 90 |
| 42 | 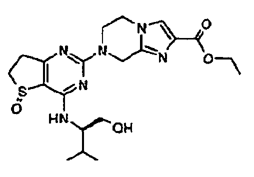 | 45 | | | 1,53 Methode C | 94 |
| 43 | 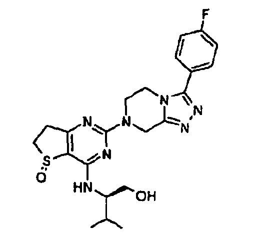 | 45 | 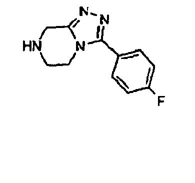 | siehe experim. Teil § 9.1, Baustein **(V-5)** | 1,64 Methode C | 88 |
| 44 | 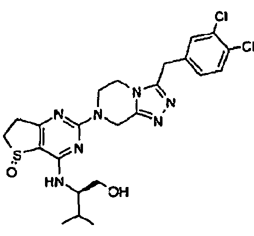 | 45 | 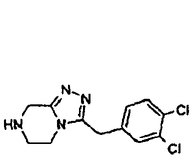 | siehe experim. Teil § 10.1, Baustein **(V-6)** | 1,79 Methode C | 78 |
| 45 | 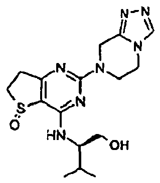 | siehe experim. Teil | | | 1,35 Methode C | 55 |

| | | | | | | |
|---|---|---|---|---|---|---|
| * Das Beispiel kann analog hergestellt und aufgereinigt werden. | | | | | | |

Bei den Beispielen handelt es sich weiterhin um folgende Verbindungen 46 - 57 der allgemeinen Formel 1, die in Tabelle D aufgezählt sind:

**Tabelle D: Chemische Strukturen und Details zur Herstellung der Beispiel-Substanzen 46 - 57**

| # | Struktur | Herstellung analog zu #* | nicht käufliche Arylpiperidin-Baustein **(V)** | Literatur zur Herstellung des Bausteins **(V)** | Analytische HPLC-MS, RT [min], Methode | % Inhibition PDE4B @ 1 µM |
|---|---|---|---|---|---|---|
| 46 | 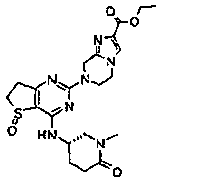 | 9 | | | 1,05 Methode B | 97 |
| 47 | 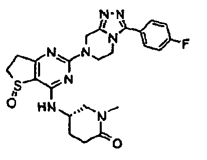 | 9 | 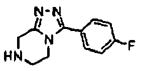 | siehe experim. Teil § 9.1, Baustein **(V-5)** | 1,12 Methode B | 96 |
| 48 | 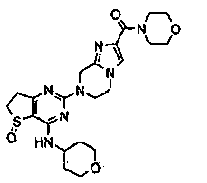 | siehe experim. Teil | | | 0,79 Methode D | 93 |
| 49 | 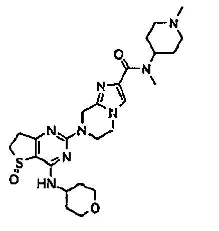 | 48 | | | 0,81 Methode D | 93 |
| 50 | 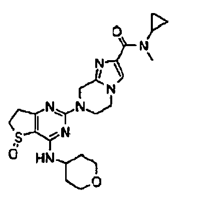 | 48 | | | 0,83 Methode D | 93 |
| 51 | 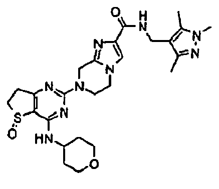 | 48 | | | 0,84 Methode D | 94 |
| 52 | 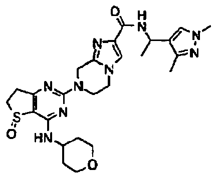 | 48 | | | 1,09 Methode B | 93 |
| 53 | 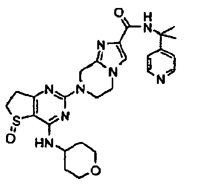 | 48 | | | 1,06 Methode B | 94 |
| 54 | 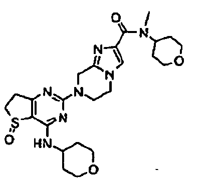 | 48 | | | 1,07 Methode B | 94 |
| 55 | 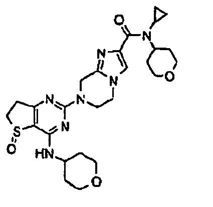 | 48 | | | 1,11 Methode B | 93 |
| 56 | 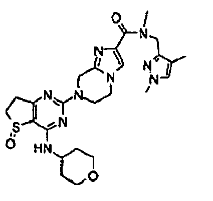 | 48 | | | 1,07 Methode B | 94 |
| 57 | 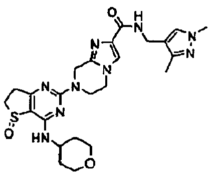 | 48 | | | 1,05 Methode B | 94 |

| | | | | | | |
|---|---|---|---|---|---|---|
| * Das Beispiel kann analog hergestellt und aufgereinigt werden. | | | | | | |

### INDIKATIONSGEBIETE

Wie gefunden wurde, zeichnen sich die Verbindungen der Formel **1** durch vielfältige Anwendungsmöglichkeiten auf therapeutischem Gebiet aus. Hervorzuheben sind solche Anwendungsmöglichkeiten, für welche die erfindungsgemäßen Verbindungen der Formel **1** aufgrund ihrer pharmazeutischen Wirksamkeit als PDE4-Inhibitor bevorzugt zur Anwendung gelangen können. Beispielhaft genannt seinen Atemwegs- oder gastrointestinalen Erkrankungen oder Beschwerden, entzündliche Erkrankungen der Gelenke, der Haut oder der Augen, Krebserkrankungen, sowie Erkrankungen des periphären oder zentralen Nervensystems.

Hierbei bevorzugt genannt sei die Vorbeugung und Behandlung von Atemwegs- oder Lungenerkrankungen, welche mit einer erhöhten Schleimproduktion, Entzündungen und/oder obstruktiven Erkrankungen der Atemwege einher gehen. Beispielhaft hierfür seinen genannt, akute, allergische oder chronische Bronchitis, chronisch obstruktive Bronchitis (COPD), Husten, Lungenemphysem, allergische oder nicht-allergische Rhinitis oder Sinusitis, chronische Rhinitis oder Sinusitis, Asthma, Alveolitis, Farmers' Krankheit, hyperreaktive Atemwege, infektiöse Bronchitis oder Pneumonitis, pediatrisches Asthma, Bronchiectasien, Lungenfibrose, ARDS (akutes Atemnotsyndrom des Erwachsenen), Bronchialödem, Lungenödem, Bronchitis, Pneumonie oder interstitielle Pneumonie ausgelöst durch verschiedene Ursachen wie Aspiration, Inhalation von toxischen Gasen oder Bronchitis, Pneumonie oder interstitielle Pneumonie ausgelöst durch Herzinsuffizienz, Bestrahlung, Chemotherapie zystische Fibrose oder Mukoviszidose, alpha1-Antitrypsin-Mangel.

Ebenfalls bevorzugt genannt sei die Behandlung von entzündlichen Erkrankungen des Gastrointestinaltraktes. Beispielhaft hierfür seinen genannt, akute oder chronische entzündliche Veränderungen bei Gallenblasenentzündung, Morbus Crohn, Colitis ulcerosa, entzündliche Pseudopolypen, juvenile Polypen, Colitis cystica profunda, Pneumatosis cystoides intestinales, Erkrankungen der Gallengänge und Gallenblase, z.B. Gallensteine und Konglomerate, zur Behandlung von entzündlichen Erkrankungen der Gelenke wie rheumatoide Arthritis oder entzündliche Erkrankungen der Haut und der Augen.

Ebenfalls bevorzugt genannt sei die Behandlung von Krebserkrankungen. Beispielhaft hierfür seinen genannt alle Formen von akuten und chronischen Leukämien wie, akute lymphatische und akute myeloische Leukämie, chronisch lymphatische und chronisch myeloische Leukämie, sowie Knochentumoren wie das Osteosarkom und sowie alle Arten von Gliomen wie Oligodendrogliom und Glioblastom.

Des Weiteren bevorzugt genannt sei die Vorbeugung und Behandlung von Erkrankungen des periphären oder zentralen Nervensystems. Beispielhaft hierfür seien genannt Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Hirn-Trauma.

Besonders bevorzugt betrifft die vorliegende Erfindung die Verwendung von Verbindungen der Formel **1** zur Herstellung eines Arzneimittels zur Behandlung entzündlicher oder obstruktiver Erkrankungen der oberen und unteren Atmungsorgane einschließlich der Lunge wie beispielsweise allergische Rhinitis, chronische Rhinitis, Bronchiectasis, zystische Fibrose, idiopathische Lungenfibrose, fibrosierende Alveolitis, COPD, chronische Bronchitis, chronischer Sinusitis, Asthma, Morbus Crohn, Colitis ulcerosa, insbesondere COPD, chronische Bronchitis und Asthma.

Am meisten bevorzugt ist die Verwendung der Verbindungen der Formel **1** zur Behandlung von entzündlichen und obstruktiven Erkrankungen wie COPD, chronische Bronchitis, chronischer Sinusitis, Asthma, Morbus Crohn, Colitis ulcerosa, insbesondere COPD, chronische Bronchitis und Asthma.

Ebenfalls bevorzugt ist die Verwendung der Verbindungen der Formel **1** zur Behandlung von Erkrankungen des periphären oder zentralen Nervensystems wie Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Hirn-Trauma.

Ein herausragender Aspekt der vorliegenden Erfindung ist das reduzierte Profil an Nebenwirkungen. Darunter wird im Rahmen der Erfindung verstanden, eine Dosis einer pharmazeutischen Zusammensetzung verabreichen zu können, ohne beim Patienten Erbrechen, bevorzugt Übelkeit, besonders bevorzugt Unwohlsein auszulösen. Höchst bevorzugt ist die Verabreichung einer therapeutisch wirksamen Substanzmengen, ohne Emesis oder Nausea auszulösen, in jedem Stadium des Krankheitsverlaufs.

### KOMBINATIONEN

Die Verbindungen der Formel **1** können allein oder in Kombination mit anderen erfindungsgemäßen Wirkstoffen der Formel **1** zur Anwendung gelangen. Gegebenenfalls können die Verbindungen der Formel **1** auch in Kombination mit weiteren pharmakologisch aktiven Wirkstoffen eingesetzt werden. Bevorzugt gelangen hierbei solche Wirkstoffe zur Anwendung, die beispielsweise ausgewählt sind aus der Gruppe bestehend aus Betamimetika, Anticholinergika, Corticosteroiden, weiteren PDE4-Inhibitoren, LTD4-Antagonisten, EGFR-Hemmern, MRP4-Inhibitoren, Dopamin-Agonisten, H1-Antihistaminika, PAF-Antagonisten und PI3-Kinase Inhibitoren oder zwei- oder dreifach Kombinationen davon, wie beispielsweise Kombinationen von Verbindungen der Formel **1** mit ein oder zwei Verbindungen aus der Gruppe bestehend aus
- Betamimetika, Corticosteroiden, PDE4-Inhibitoren, EGFR-Hemmer und LTD4-Antagonisten,
- Anticholinergika, Betamimetika, Corticosteroiden, PDE4-Inhibitoren, EGFR-Hemmern und LTD4-Antagonisten,
- PDE4-Inhibitoren, Corticosteroiden, EGFR-Hemmern und LTD4-Antagonisten
- EGFR-Hemmern, PDE4-inhibitoren und LTD4-Antagonisten
- EGFR-Hemmern und LTD4-Antagonisten
- CCR3-Inhibitoren, iNOS-Inhibitoren (inducible nitric oxide synthase-Inhibitoren), (6R)-L-erythro-5,6,7,8-tetrahydrobiopterin (im folgenden "BH4" genannt) und dessen Derivate wie in WO 2006/120176 genannt und SYK-Inhibitoren (spleen tyrosine kinase-Inhibitoren)
- Anticholinergika, Betamimetika, Corticosteroiden, PDE4-Inhibitoren und MRP4-Inhibitoren.

Auch die Kombinationen dreier Wirkstoffe je einer der o.g. Verbindungsklassen ist Bestandteil der Erfindung.

Als Betamimetika gelangen hierbei vorzugsweise Verbindungen zur Anwendung, die ausgewählt sind aus der Gruppe bestehend aus Albuterol, Bambuterol, Bitolterol, Broxaterol, Carbuterol, Clenbuterol, Fenoterol, Formoterol, Arformoterol, Zinterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmeterol, Salmefamol, Soterenol, Sulphonterol, Tiaramide, Terbutaline, Tolubuterol, CHF-1035, HOKU-81, KUL-1248, 3-(4-{6-[2-Hydroxy-2-(4-hydroxy-3-hydroxymethyl-phenyl)-ethylamino]-hexyloxy}-butyl)-benzyl-sulfonamid, 5-[2-(5,6-Diethyl-indan-2-ylamino)-1-hydroxy-ethyl]-8-hydroxy-1H-quinolin-2-on, 4-Hydroxy-7-[2-{[2-{[3-(2-phenylethoxy)propyl]sulphonyl}ethyl]-amino}ethyl]-2(3H)-benzothiazolon, 1-(2-Fluoro-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-Hydroxy-8-(1-hydroxy-2-isopropylaminobutyl)-2H-1,4-benzoxazin-3-(4H)-on, 1-(4-Amino-3-chloro-5-trifluormethylphenyl)-2-tert.-butylamino)ethanol, 6-Hydroxy-8-{1-hydroxy-2-[2-(4-methoxyphenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropyl-phenyl)-1,1dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxy-phenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 1-(4-Ethoxy-carbonylamino-3-cyano-5-fluorophenyl)-2-(tert.-butylamino)ethanol, gegebenenfalls in Form ihrer Racemate, Enantiomere, Diasteromere und gegebenenfalls in Form ihrer pharmakologisch verträglichen Säureadditionssalze, Solvate oder Hydrate.

Bevorzugt sind die Betamimetika ausgewählt aus der Gruppe bestehend aus Bambuterol, Bitolterol, Carbuterol, Clenbuterol, Fenoterol, Formoterol, Hexoprenaline, Ibuterol, Pirbuterol, Procaterol, Reproterol, Salmeterol, Sulphonterol, Terbutaline, Tolubuterol, 3-(4-{6-[2-Hydroxy-2-(4-hydroxy-3-hydroxymethyl-phenyl)-ethylamino]-hexyloxy}-butyl)-benzenesulfoneamide, 5-[2-(5,6-Diethyl-indan-2-ylamino)-1-hydroxy-ethyl]-8-hydroxy-1H-quinolin-2-on , 4-hydroxy-7-[2-{[2-{[3-(2-phenylethoxy)propyl]sulphonyl}ethyl]-amino}ethyl]-2(3H)-benzothiazolone, 1-(2-fluoro-4-hydroxyphenyl)-2-[4-(1-benzimidazolyl)-2-methyl-2-butylamino]ethanol, 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-hydroxy-8-(1-hydroxy-2-isopropylaminobutyl)-2H-1,4-benzoxazin-3-(4H)-on, 1-(4-amino-3-chloro-5-trifluormethylphenyl)-2-tert.-butylamino)ethanol, 6-Hydroxy-8-{1-hydroxy-2-[2-(4-methoxy-phenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxyethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropyl-phenyl)-1,1dimethyl-ethylamino]-ethyl}-4H-benzo[1,4]oxazin-3-on, 8-{2-[2-(4-Ethylphenyl)-1,1-dimethyl-ethylamino]-1-hydroxy-ethyl}-6-hydroxy-4H-benzo[1,4]oxazin-3-on, 8-{2-[2-(4-Ethoxy-phenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 1-(4-ethoxycarbonylamino-3-cyano-5-fluorophenyl)-2-(tert.-butylamino)ethanol, gegebenenfalls in Form ihrer Racemate, Enantiomere, Diasteromere und gegebenenfalls in Form ihrer pharmakologisch verträglichen Säureadditionssalze, Solvate oder Hydrate.

Besonders bevorzugte Betamimetika sind ausgewählt aus der Gruppe bestehend aus Fenoterol, Formoterol, Salmeterol, 3-(4-{6-[2-Hydroxy-2-(4-hydroxy-3-hydroxymethyl-phenyl)-ethylamino]-hexyloxy}-butyl)-benzenesulfoneamid, 5-[2-(5,6-Diethyl-indan-2-ylamino)-1-hydroxy-ethyl]-8-hydroxy-1H-quinolin-2-on, 1-[3-(4-Methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino]ethanol, 6-Hydroxy-8-{1-hydroxy-2-[2-(4-methoxyphenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropyl-phenyl)-1,1dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxy-phenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2 -methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, gegebenenfalls in Form ihrer Racemate, Enantiomere, Diasteromere und gegebenenfalls in Form ihrer pharmakologisch verträglichen Säureadditionssalze, Solvate oder Hydrate.

Von diesen Betamimetika sind erfindungsgemäß besonders bevorzugt Formoterol, Salmeterol, 3-(4-{6-[2-Hydroxy-2-(4-hydroxy-3-hydroxymethyl-phenyl)-ethylamino]-hexyloxy}-butyl)-benzenesulfoneamide, 6-Hydroxy-8-{1-hydroxy-2-[2-(4-methoxy-phenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester}-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxyethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropyl-phenyl)-1,1dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxyphenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 5-[2-(5,6-Diethyl-indan-2-ylamino)-1-hydroxy-ethyl]-8-hydroxy-1H-quinolin-2-on, gegebenenfalls in Form ihrer Racemate, Enantiomere, Diasteromere und gegebenenfalls in Form ihrer pharmakologisch verträglichen Säureadditionssalze, Solvate oder Hydrate.

Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden. Von den vorstehend genannten Säureadditionssalzen sind die Salze der Chlorwasserstoffsäure, der Methansulfonsäure, der Benzoesäure und der Essigsäure erfindungsgemäß besonders bevorzugt.

Als Anticholinergika gelangen hierbei vorzugsweise Verbindungen zur Anwendung, die ausgewählt sind aus der Gruppe bestehend aus Tiotropiumsalzen, Oxitropiumsalzen, Flutropiumsalzen, Ipratropiumsalzen, Glycopyrroniumsalzen, Trospiumsalzen 2,2-Diphenylpropionsäuretropenolester-methobromid, 2,2-Diphenylpropionsäurescopinestermethobromid, 2-Fluor-2,2-Diphenylessigsäurescopinester-methobromid, 2-Fluor-2,2-Diphenylessigsäuretropenolester-methobromid, 3,3',4,4'-Tetrafluorbenzilsäuretropenolester-Methobromid, 3,3',4,4'-Tetrafluorbenzilsäurescopinester-Methobromid, 4,4'-Difluorbenzilsäuretropenolester-Methobromid, 4,4'-Difluorbenzilsäurescopinester-Methobromid, 3,3'-Difluorbenzilsäuretropenolester-Methobromid, 3,3'-Difluorbenzilsäurescopinester-Methobromid, 9-Hydroxy-fluoren-9-carbonsäuretropenolester -Methobromid, 9-Fluor-fluoren-9-carbonsäuretropenolester -Methobromid, 9-Hydroxy-fluoren-9-carbonsäure-scopinester -Methobromid, 9-Fluor-fluoren-9-carbonsäurescopinester Methobromid, 9-Methyl-fluoren-9-carbonsäuretropenolester Methobromid, 9-Methyl-fluoren-9-carbonsäure-scopinester Methobromid, Benzilsäurecyclopropyltropinester-Methobromid, 2,2-Diphenylpropionsäurecyclopropyltropinester -Methobromid, 9-Hydroxy-xanthen-9-carbonsäure-cyclopropyltropinesterMethobromid, 9-Methyl-fluoren-9-carbonsäurecyclopropyltropinester-Methobromid, 9-Methyl-xanthen-9-carbonsäurecyclopropyltropinester -Methobromid, 9-Hydroxy-fluoren-9-carbonsäurecyclopropyltropinester -Methobromid, 4,4'-Difluorbenzilsäuremethylestercyclopropyltropinester -Methobromid, 9-Hydroxy-xanthen-9-carbonsäure-tropenolester -Methobromid, 9-Hydroxy-xanthen-9-carbonsäurescopinester Methobromid, 9-Methyl-xanthen-9-carbonsäuretropenolester -Methobromid, 9-Methyl-xanthen-9-carbonsäurescopinester -Methobromid, 9-Ethyl-xanthen-9-carbonsäuretropenolester Methobromid, 9-Difluormethyl-xanthen-9-carbonsäuretropenolester -Methobromid. 9-Hydroxymethyl-xanthen-9-carbonsäurescopinester -Methobromid, gegebenenfalls in Form ihrer Solvate oder Hydrate.

In den vorstehend genannten Salzen stellen die Kationen Tiotropium, Oxitropium, Flutropium, Ipratropium, Glycopyrronium und Trospium die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodid und Methansulfonat besonders bevorzugt.

Von besonderer Bedeutung ist das Tiotropiumbromid. Im Falle des Tiotropiumbromids enthalten die erfindungsgemäßen Arzneimittelkombinationen dieses bevorzugt in Form des kristallinen Tiotropiumbromid Monohydrats, welches aus der WO 02/30928 bekannt ist. Wird das Tiotropiumbromid in den erfindungsgemäßen Arzneimittelkombinationen in wasserfreier Form eingesetzt, so gelangt bevorzugt das wasserfreie kristalline Tiotropiumbromid zur Anwendung, welches aus der WO 03/000265 bekannt ist.

Als Corticosteroide gelangen hierbei vorzugsweise Verbindungen zur Anwendung, die ausgewählt sind aus der Gruppe bestehend aus Prednisolon, Prednison, Butixocortpropionat, Flunisolid, Beclomethason, Triamcinolon, Budesonid, Fluticason, Mometason, Ciclesonid, Rofleponid, Dexamethason, Betamethason, Deflazacort,RPR-106541, NS-126, 6,9-Difluoro-17-[(2-furanylcarbonyl)oxy]-11-hydroxy-16-methyl-3-oxo-androsta-1,4-dien-17-carbothionsäure (S)-fluoromethylester und 6,9-Difluoro-11-hydroxy-16-methyl-3-oxo-17-propionyloxy-androsta-1,4-dien-17-carbothionsäure (S)-(2-oxo-tetrahydrofuran-3S-yl)ester, gegebenenfalls in Form ihrer Racemate, Enantiomere oder Diastereomere und gegebenenfalls in Form ihrer Salze und Derivate, ihrer Solvate und/oder Hydrate.

Besonders bevorzugt ist das Steroid ausgewählt aus der Gruppe bestehend aus Flunisolid, Beclomethason, Triamcinolon, Budesonid, Fluticason, Mometason, Ciclesonid, Rofleponid, Dexamethason,NS-126, 6,9-Difluoro-17-[(2-furanylcarbonyl)oxy]-11-hydroxy-16-methyl-3-oxo-androsta-1,4-dien-17-carbothionsäure (S)-fluoromethylester und 6,9-Difluoro-11-hydroxy-16-methyl-3-oxo-17-propionyloxy-androsta-1,4-dien-17-carbothionsäure (S)-(2-oxo-tetrahydro-furan-3S-yl)ester, gegebenenfalls in Form ihrer Racemate, Enantiomere oder Diastereomere und gegebenenfalls in Form ihrer Salze und Derivate, ihrer Solvate und/oder Hydrate.

Besonders bevorzugt ist das Steroid ausgewählt aus der Gruppe bestehend aus Budesonid, Fluticason, Mometason, Ciclesonid und 6,9-Difluoro-17-[(2-furanylcarbonyl)oxy]-11-hydroxy-16-methyl-3-oxo-androsta-1,4-dien-17-carbothionsäure (S)-fluoromethylester, gegebenenfalls in Form ihrer Racemate, Enantiomere oder Diastereomere und gegebenenfalls in Form ihrer Salze und Derivate, ihrer Solvate und/oder Hydrate.

Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Propionate, Dihydrogenphosphate, Palmitate, Pivalate oder auch Furoate.

Als weitere PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4396 (Sch-351591), AWD-12-281 (GW-842470), NCS-613, CDP-840, D-4418, PD-168787, T-440, T-2585, V-11294A, CI-1018, CDC-801, CDC-3052, D-22888, YM-58997, Z-15370,N-(3,5-Dichloro-1-oxo-pyridin-4-yl)-4-difluoromethoxy-3-cyclopropylmethoxybenzamid, (-)p-[(4aR*,10bS*)-9-Ethoxy-1,2,3,4,4a,10b-hexahydro-8-methoxy-2-methyl-benzo[s][1,6]naphthyridin-6-yl]-N,N-diisopropylbenzamid, (R)-(+)-1-(4-Bromobenzyl)-4-[(3-cyclopentyloxy)-4-methoxyphenyl]-2-pyrrolidon, 3-(Cyclopentyloxy-4-methoxyphenyl)-1-(4-N'-[N-2-cyano-S-methyl-isothioureido]benzyl)-2-pyrrolidon, cis[4-Cyano-4-(3-cyclopentyloxy-4-methoxyphenyl)cyclohexan-1-carbonsäure], 2-carbomethoxy-4-cyano-4-(3-cyclopropylmethoxy-4-difluoromethoxyphenyl)cyclohexan-1-on, cis[4-Cyano-4-(3-cyclopropylmethoxy-4-difluoromethoxyphenyl)cyclohexan-1-ol], (R)-(+)-Ethyl[4-(3-cyclopentyloxy-4-methoxyphenyl)pyrrolidin-2-yliden]acetat, (S)-(-)-Ethyl[4-(3-cyclopentyloxy-4-methoxyphenyl)pyrrolidin-2-yliden]acetat, 9-Cyclopentyl-5,6-dihydro-7-ethyl-3-(2-thienyl)-9H-pyrazolo[3,4-c]-1,2,4-triazolo[4,3-a]pyridin und 9-Cyclopentyl-5,6-dihydro-7-ethyl-3-(tert-butyl)-9H-pyrazolo[3,4-c]-1,2,4-triazolo[4,3-a]pyridin, gegebenenfalls in Form ihrer Racemate, Enantiomere oder Diastereomere und gegebenenfalls in Form ihrer pharmakologisch verträglichen Säureadditionssalze, Solvate und/oder Hydrate.

Besonders bevorzugt ist der PDE4-Inhibitor ausgewählt aus der Gruppe bestehend aus Enprofyllin, Roflumilast, Ariflo (Cilomilast), Arofyllin, Atizoram,AWD-12-281 (GW-842470), T-440, T-2585, PD-168787, V-11294A, CI-1018, CDC-801, D-22888, YM-58997, Z-15370, N-(3,5-Dichloro-1-oxo-pyridin-4-yl)-4-difluoromethoxy-3-cyclopropylmethoxybenzamid, Cis[4-Cyano-4-(3-cyclopentyloxy-4-methoxyphenyl)cyclohexan-1-carbonsäure], 2-Carbomethoxy-4-cyano-4-(3-cyclopropylmethoxy-4-difluoromethoxyphenyl)cyclohexan-1-on, Cis[4-cyano-4-(3-cyclopropylmethoxy-4-difluoromethoxyphenyl)cyclohexan-1-ol], 9-Cyclopentyl-5,6-dihydro-7-ethyl-3-(2-thienyl)-9H-pyrazolo[3,4-c]-1,2,4-triazolo[4,3-a]pyridin und 9-Cyclopentyl-5,6-dihydro-7-ethyl-3-(tert-butyl)-9H-pyrazolo[3,4-c]-1,2,4-triazolo[4,3-a]pyridin, gegebenenfalls in Form ihrer Racemate, Enantiomere oder Diastereomere und gegebenenfalls in Form ihrer pharmakologisch verträglichen Säureadditionssalze, Solvate und/oder Hydrate.

Besonders bevorzugt ist der PDE4-Inhibitor ausgewählt aus der Gruppe bestehend aus Roflumilast, Ariflo (Cilomilast), Arofyllin,AWD-12-281 (GW-842470), 2-Carbomethoxy-4-cyano-4-(3-cyclopropylmethoxy-4-difluoromethoxyphenyl)cyclyhexan-1-on, Cis[4-cyano-4-(3-cyclopropylmethoxy-4-difluoromethoxyphenyl)cyclohexan-1-ol], Atizoram, Z-15370, 9-Cyclopentyl-5,6-dihydro-7-ethyl-3-(2-thienyl)-9H-pyrazolo[3,4-c]-1,2,4-triazolo[4,3-a]pyridin und 9-Cyclopentyl-5,6-dihydro-7-ethyl-3-(tert-butyl)-9H-pyrazolo[3,4-c]-1,2,4-triazolo[4,3-a]pyridin gegebenenfalls in Form ihrer Racemate, Enantiomere oder Diastereomere und gegebenenfalls in Form ihrer pharmakologisch verträglichen Säureadditionssalze, Solvate und/oder Hydrate.

Unter Säureadditionssalzen mit pharmakologisch verträglichen Säuren zu deren Bildung die o.g. PDE4-inhibitoren gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321, 1-(((R)-(3-(2-(6,7-Difluoro-2-quinolinyl)ethenyl)phenyl)-3-(2-(2-hydroxy-2-propyl)phenyl)thio)-methylcyclopropan-essigsäure, 1-(((1(R)-3(3-(2-(2,3-Dichlorothieno[3,2-b]pyridin-5-yl)-(E)-ethenyl)phenyl)-3-(2-(1-hydroxy-1-methylethyl)phenyl)propyl)thio)methyl)cyclopropan-essigsäure und [2-[[2-(4-tert-Butyl-2-thiazolyl)-5-benzofuranyl]oxymethyl]phenyl]essigsäure gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze- und Derivate, ihrer Solvate und/oder Hydrate.

Bevorzugt ist der LTD4-Antagonist ausgewählt aus der Gruppe bestehend aus Montelukast, Pranlukast, Zafirlukast, MCC-847 (ZD-3523), MN-001, MEN-91507 (LM-1507), VUF-5078, VUF-K-8707 und L-733321, gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

Besonders bevorzugt ist der LTD4-Antagonist ausgewählt aus der Gruppe bestehend aus Montelukast, Pranlukast, Zafirlukast, MCC-847 (ZD-3523), MN-001 und MEN-91507 (LM-1507) gegebenenfalls in Form ihrer Racemate, Enantiomere oder Diastereomere, gegebenenfalls in Form ihrer pharmakoligisch verträglichen Säureadditionssalze sowie gegebenenfalls in Form ihrer Salze und Derivate, ihrer Solvate und/oder Hydrate.

Unter Säureadditionssalzen mit pharmakologisch verträglichen Säuren zu deren Bildung die LTD4-Antagonisten gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden. Unter Salzen oder Derivaten zu deren Bildung die LTD4-antagonisten gegebenenfalls in der Lage sind, werden beispielsweise verstanden : Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluorphenyl)amino]-6-{[4-(N,N-diethylamino)-1-oxo-2-buten-1-yl]amino}-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopentyloxy-chinazolin, 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin, 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin, 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin, 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin, 4-[(3-Ethinyl-phenyl)amino]-6,7-bis-(2-methoxy-ethoxy)-chinazolin, 4-[(3-Chlor-4-fluorphenyl)amino]-7-[3-(morpholin-4-yl)-propyloxy]-6-[(vinylcarbonyl)amino]-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-{[3-Chlor-4-(3-fluor-benzyloxy)-phenyl]amino}-6-(5-{[(2-methansulfonyl-ethyl)amino]methyl}-furan-2-yl)chinazolin, 4-[(R)-(1-Phenylethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-[2-(2,2-dimethyl-6-oxo-morpholin-4-yl)-ethoxy]-7-methoxy-chinazolin, 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin, 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluorphenyl)amino]-6-[1-(tert.-butyloxycarbonyl)-piperidin-4-yloxy]-7-methoxy-chinazolin, 4-[(3-Chlor-4-fluor-phenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-(1-methyl-piperidin-4-yloxy)-7-methoxy-chinazolin, 4-[(3-Chlor-4-fluor-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(methoxymethyl)carbonyl]-piperidin-4-yloxy}-7-methoxy-chinazolin, 4-[(3-Chlor-4-fluorphenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluorphenyl)amino]-6-(tetrahydropyran-4-yloxy)-7-ethoxy-chinazolin, 4-[(3-Chlor-4-fluorphenyl}amino]-6-((S)-tetrahydrofuran-3-yloxy)-7-hydroxy-chinazolin, 4-[(3-Chlor-4-fluorphenyl)amino]-6-(tetrahydropyran-4-yloxy)-7-(2-methoxy-ethoxy)-chinazolin, 4-[(3-Chlor-4-fluor-phenyl)amino]-6-{trans-4-[(dimethylamino)sulfonylamino]-cyclohexan-1-yloxy}-7-methoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluorphenyl)amino]-6-{trans-4-[(morpholin-4-yl)sulfonylamino]-cyclohexan-1-yloxy}-7-methoxy-chinazolin, 4-[(3-Chlor-4-fluor-phenyl)amino]-6-(tetrahydropyran-4-yloxy)-7-(2-acetylamino-ethoxy)-chinazolin, 4-[(3-Chlor-4-fluor-phenyl)amino]-6-(tetrahydropyran-4-yloxy)-7-(2-methansulfonylamino-ethoxy)-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluor-phenyl)amino]-6-(1-aminocarbonylmethyl-piperidin-4-yloxy)-7-methoxy-chinazolin, 4-[(3-Chlor-4-fluorphenyl)amino]-6-(cis-4-{N-[(tetrahydropyran-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluorphenyl)amino]-6-(cis-4-{N-[(morpholin-4-yl)sulfonyl]-N-methyl-amino}-cyclohexan-1-yloxy)-7-methoxy-chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluor-phenyl)amino]-6-(1-methansulfonyl-piperidin-4-yloxy)-7-ethoxy-chinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluorphenyl)amino]-6-[1-(2-methoxy-acetyl)-piperidin-4-yloxy]-7-(2-methoxy-ethoxy)-chinazolin, 4-[(3-Chlor-4-fluor-phenyl)amino]-6-(cis-4-acetylamino-cyclohexan-1-yloxy)-7-methoxy-chinazolin, 4-[(3-Ethinyl-phenyl)amino]-6-[1-(tert.-butyloxycarbonyl)-piperidin-4-yloxy]-7-methoxy-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-(cis-4-{N-[(4-methyl-piperazin-1-yl)carbonyl]-N-methyl-amino)-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Chlor-4-fluorphenyl)amino]-6{1-[(morpholin-4-yl)carbonyl]-piperidin-4-yloxy}-7-(2-methoxy-ethoxy)-chinazolin, 4-[(3-Ethinyl-phenyl)amino]-6-(1-acetyl-piperidin-4-yloxy)-7-methoxy-chinazolin 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Chlor-4-fluor-phenyl)amino]-6-(1-isopropyloxycarbonyl-piperidin-4-yloxy)-7-methoxy-chinazolin, 4-[(3-Chlor-4-fluor-phenyl)amino]-6-(cis-4-methylamino-cyclohexan-1-yloxy)-7-methoxy-chinazolin, 4-[(3-Chlor-4-fluor-phenyl)amino]-6-{cis-4-[N-(2-methoxy-acetyl)-N-methyl-amino]-cyclohexan-1-yloxy}-7-methoxy-chinazolin, 4-[(3-Ethinyl-phenyl)amino]-6-(piperidin-4-yloxy)-7-methoxy-chinazolin, 4-[(3-Ethinyl-phenyl)amino]-6-[1-(2-methoxyacetyl)-piperidin-4-yloxy]-7-methoxy-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(cis-2,6-dimethyl-morpholin-4-yl)carbonyl]-piperidin-4-yloxy}-7-methoxy-chinazolin, 4-[(3-Chlor-4-fluor-phenyl)amino]-6-{1-[(2-methyl-morpholin-4-yl)carbonyl]-piperidin-4-yloxy}-7-methoxy-chinazolin, 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin, 4-[(3-Chlor-4-fluorphenyl)amino]-6-{1-[(N-methyl-N-2-methoxyethyl-amino)carbonyl]-piperidin-4-yloxy}-7-methoxy-chinazolin, 4-[(3-Chlor-4-fluor-phenyl)amino]-6-(1-ethyl-piperidin-4-yloxy)-7-methoxy-chinazolin, 4-[(3-Chlor-4-fluor-phenyl)amino]-6-{1-[(2-methoxyethyl)carbonyl]-piperidin-4-yloxy}-7-methoxy-chinazolin, 4-[(3-Chlor-4-fluor-phenyl)amino]-6-{1-[(3-methoxypropyl-amino)-carbonyl]-piperidin-4-yloxy}-7-methoxy-chinazolin, 4-[(3-Chlor-4-fluorphenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-hinazolin, 4-[(3-Chlor-4-fluor-phenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, Cetuximab, Trastuzumab, ABX-EGF und Mab ICR-62, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Bevorzugte EGFR-Hemmer sind ausgewählt aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluorphenyl)amino]-6-{[4-(N,N-diethylamino)-1-oxo-2-buten-1-yl]-amino}-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopentyloxy-chinazolin, 4-[(R)-(1-Phenylethyl)amino]-6{[4-(N,N-bis-(2-methoxy-ethyl)-amino)-1-oxo-2-buten-1-yl]amino}-7-cyclopropylmethoxy-chinazolin, 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin, 4-[(R)-(1-Phenylethyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin, 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin, 4-[(3-Ethinyl-phenyl)amino]-6,7-bis-(2-methoxy-ethoxy)-chinazolin, 4-[(3-Chlor-4-fluorphenyl)amino]-7-[3-(morpholin-4-yl)-propyloxy]-6-[(vinylcarbonyl)amino]-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-{[3-Chlor-4-(3-fluor-benzyloxy)-phenyl]amino}-6-(5-{[(2-methansulfonyl-ethyl)amino]methyl}-furan-2-yl)chinazolin, 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-[2-(2,2-dimethyl-6-oxo-morpholin-4-yl)-ethoxy]-7-methoxy-chinazolin, 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin, 4-[(3-Chlor-4-fluorphenyl)amino]-7-[2-(2,2-dimethyl-6-oxo-morpholin-4-yl)-ethoxy]-6-[(S)-(tetrahydrofuran-2-yl)methoxy]-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluor-phenyl)amino]-6-[1-(tert.-butyloxycarbonyl)-piperidin-4-yloxy]-7-methoxy-chinazolin, 4-[(3-Chlor-4-fluor-phenyl)amino]-6-(trans-4-amino-cyclohexan-1-yloxy)-7-methoxy-chinazolin, 4-[(3-Chlor-4-fluorphenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-(1-methyl-piperidin-4-yloxy)-7-methoxy-chinazolin, 4-[(3-Chlor-4-fluor-phenyl)amino]-6-{1-[(morpholin-4-yl)carbonyl]-piperidin-4-yloxy}-methoxy-chinazolin, 4-[(3-Chlor-4-fluor-phenyl)amino]-6-{1-[(methoxymethyl)carbonyl]-piperidin-4-yl-oxy}-7-methoxy-chinazolin, 4-[(3-Chlor-4-fluor-phenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-((S)-tetrahydrofuran-3-yloxy)-7-hydroxy-chinazolin, 4-[(3-Chlor-4-fluor-phenyl)amino]-6-(tetrahydropyran-4-yloxy)-7-(2-methoxy-ethoxy)-chinazolin, 4-[(3-Chlor-4-fluor-phenyl)amino]-6-{trans-4-[(dimethylamino)sulfonylamino]-cyclohexan-1-yloxy}-7-methoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluor-phenyl)amino]-6-{trans-4-[(morpholin-4-yl)sulfonylamino]-cyclohexan-1-yloxy}-7-methoxy-chinazolin, 4-[(3-Chlor-4-fluorphenyl)amino]-6-(tetrahydropyran-4-yloxy)-7-(2-acetylamino-ethoxy)-chinazolin, 4-[(3-Chlor-4-fluor-phenyl)amino]-6-(tetrahydropyran-4-yloxy)-7-(2-methansulfonylamino-ethoxy)-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluor-phenyl)amino]-6-(1-aminocarbonylmethyl-piperidin-4-yloxy)-7-methoxy-chinazolin, 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluor phenyl)amino]-6-(1-methansulfonyl-piperidin-4-yloxy)-7-ethoxy-chinazolin, 4-[(3-Chlor-4-fluorphenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxy-acetyl)-piperidin-4-yloxy]-7-(2-methoxy-ethoxy)-chinazolin, 4-[(3-Chlor-4-fluor-phenyl)amino]-6-(cis-4-acetylamino-cyclohexan-1-yloxy)-7-methoxy-chinazolin, 4-[(3-Ethinyl-phenyl)amino]-6-[1-(tert.-butyloxycarbonyl)-piperidin-4-yloxy]-7-methoxy-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-(cis-4-{N-[(4-methyl-piperazin-1-yl)carbonyl]-N-methyl-amino}cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluorphenyl)amino]-6-{1-[2-(2-oxopyrrolidin-1-yl)ethyl]-piperidin-4-yloxy}-7-methoxy-chinazolin, 4-[(3-Chlor-4-fluor-phenyl)amino]-6-{1-[(morpholin-4-yl)carbonyl]-piperidin-4-yloxy}-7-(2-methoxy-ethoxy)-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Chlor-4-fluor-phenyl)amino]-6-(1-isopropyloxycarbonyl-piperidin-4-yloxy)-7-methoxy-chinazolin, 4-[(3-Chlor-4-fluor-phenyl)amino]-6-(cis-4-methylamino-cyclohexan-1-yloxy)-7-methoxy-chinazolin, 4-[(3-Chlor-4-fluor-phenyl)amino]-6-{cis-4-[N-(2-methoxy-acetyl)-N-methyl-amino]-cyclohexan-1-yloxy}-7-methoxy-chinazolin, 4-[(3-Ethinyl-phenyl)amino]-6-(piperidin-4-yloxy)-7-methoxy-chinazolin, 4-[(3-Ethinyl-phenyl)amino]-6-[1-(2-methoxy-acetyl)-piperidin-4-yloxy]-7-methoxy-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluorphenyl)amino]-6-{1-[(cis-2,6-dimethyl-morpholin-4-yl)carbonyl]-piperidin-4-yloxy}-7-methoxy-chinazolin, 4-[(3-Chlor-4-fluor-phenyl)amino]-6-{1-[(2-methyl-morpholin-4-yl)carbonyl]-piperidin-4-yloxy}-7-methoxy-chinazolin, 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluor-phenyl)amino]-6-(1-ethyl-piperidin-4-yloxy)-7-methoxy-chinazolin, 4-[(3-Chlor-4-fluor-phenyl)amino]-6-{1-[(2-methoxyethyl)carbonyl]-piperidin-4-yloxy}-7-methoxy-chinazolin, 4-[(3-Chlor-4-fluor-phenyl)amino]-6-{1-[(3-methoxypropyl-amino)-carbonyl]-piperidin-4-yloxy}-7-methoxy-chinazolin, 4-[(3-Chlor-4-fluorphenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, und Cetuximab, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Besonders bevorzugt gelangen im Rahmen der vorliegenden Erfindung diejenigen EGFR-Hemmer zur Anwendung, die ausgewählt sind aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxyl-chinazolin, 4-[(3-Ethinyl-phenyl)amino]-6,7-bis-(2-methoxy-ethoxy)-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluorphenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-{1-[(morpholin-4-yl)carbonyl]-piperidin-4-yl-oxy}-7-methoxy-chinazolin, 4-[(3-Chlor-4-fluor-phenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-{trans-4-[(morpholin-4-yl)carbonylamino]₋cyclohexan-1-yloxy}-7-methoxy-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluorphenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxy-acetyl)-piperidin-4-yloxy]-7-(2-methoxy-ethoxy)-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluor-phenyl)amino]-6-(1-ethyl-piperidin-4-yloxy)-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin, 4-[(3-Chlor-4-fluorphenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluorphenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, und 4-[(3-Chlor-4-fluor-phenyl)amino]-6-{1-[(2-methoxyethyl)carbonyl]-piperidin-4-yloxy}-7-methoxy-chinazolin, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Erfindungsgemäß besonders bevorzugt sind als EGFR-Hemmer diejenigen Verbindungen, die ausgewählt sind aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(3-Ethinyl-phenyl)amino]-6,7-bis-(2-methoxy-ethoxy)-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin,4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluorphenyl)amino)-6-(trans-4-methansulfonylamino-cyclohexan-1-yloxy)-7-methoxy-chinazolin, 4-[(3-Chlor-4-fluor-phenyl)amino]-6-(tetrahydropyran-3-yioxy)-7-methoxy-chinazolin, 4-[(3-Chlor-4-fluor-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(2-methoxyethyl)carbonyl]-piperidin-4-yloxy}-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin, 4-[(3-Chlor-4-fluorphenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluorphenyl)amino]-6-(1-methansulfonyl-piperidin-4-yloxy)-7-methoxy-chinazolin und 4-[(3-Chlor-4-fluor-phenyl)amino]-6-(1-cyano-piperidin-4-yloxyy-7-methoxy-chinazolin gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Unter Säureadditionssalzen mit pharmakologisch verträglichen Säuren zu deren Bildung die EGFR-Hemmer gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan. Eine Bezugnahme auf die vorstehend genannten Dopamin-Agonisten schließt im Rahmen der vorliegenden Erfindung eine Bezugnahme auf deren gegebenenfalls existierende pharmakologisch verträgliche Säureadditionssalze und gegebenenfalls deren Hydrate ein. Unter den physiologisch verträglichen Säureadditionssalzen, die von den vorstehend genannten Dopamin-Agonisten gebildet werden können, werden beispielsweise pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure sind.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin. Eine Bezugnahme auf die vorstehend genannten H1-Antihistaminika schließt im Rahmen der vorliegenden Erfindung eine Bezugnahme auf deren gegebenenfalls existierende pharmakologisch verträgliche Säureadditionssalze ein.

Als PAF-Antagonisten gelangen hierbei vorzugsweise Verbindungen zur Anwendung, die ausgewählt sind aus der Gruppe bestehend aus 4-(2-Chlorphenyl)-9-methyl-2-[3(4-morpholinyl)-3-propanon-1-yl]-6H-thieno-[3,2-f]-[1,2,4]triazolo[4,3-a][1,4]diazepin, 6-(2-Chlorphenyl)-8,9-dihydro-1-methyl-8-[(4-morpholinyl)carbonyl]-4H,7H-cyclo-penta-[4,5]thieno-[3,2-f][1,2,4]triazola[4,3-a][1,4]diazepin.

Als MRP4-Inhibitoren gelangen hierbei vorzugsweise Verbindungen zur Anwendung, die ausgewählt sind aus der Gruppe bestehend aus *N*-Acetyl-dinitrophenyl-Cysteine, cGMP, Cholate, Diclofenac, Dehydroepiandrosterone 3-glucuronide, Dehydroepiandrosterone 3-sulphate, Dilazep, Dinitrophenyl-S-glutathione, Estradiol 17-beta-glucuronide, Estradiol 3,17-disulphate, Estradiol 3-glucuronide, Estradiol 3-sulphate, Estrone 3-sulphate, Flurbiprofen, Folate, N5-formyl-tetrahydrofolate, Glycocholate, Glycolithocholic acid sulphate, Ibuprofen, Indomethacin, Indoprofen, Ketoprofen, Lithocholic acid sulphate, Methotrexate, MK571 ((*E*)-3-[[[3-[2-(7-Chloro-2-quinolinyl)ethenyl]phenyl]-[[3-dimethylamino)-3-oxopropyl]thio]methyl]thio]-propanoic acid), alpha-Naphthyl-beta-D-glucuronide, Nitrobenzyl mercaptopurine riboside, Probenecid, PSC833, Sildenafil, Sulfinpyrazone, Taurochenodeoxycholate, Taurocholate, Taurodeoxycholate, Taurolithocholate, Taurolithocholic acid sulphate, Topotecan, Trequinsin und Zaprinast, Dipyridamol, gegebenenfalls in Form ihrer Racemate, Enantiomere, Diastereomere und ihrer pharmakologisch verträglichen Säure-Additionssalze und Hydrate.

Vorzugsweise bezieht sich die Erfindung auf die Verwendung von MRP4-Inhibitoren zur Herstellung einer pharmazeutischen Zusammensetzung zur Behandlung von Atemwegserkrankungen enthaltend die erfindungsgemäßen PDE4B-Inhibitoren und MRP4-Inhibitoren, wobei die MRP4-Inhibitoren vorzugsweise ausgewählt sind aus der Gruppe bestehend aus N-Acetyl-dinitrophenyl-Cysteine, Dehydroepiandrosterone 3-sulphate, Dilazep, Dinitrophenyl-S-glutathione, Estradiol 3,17-disulphate, Flurbiprofen, Glycocholate, Glycolithocholic acid sulphate, Ibuprofen, Indomethacin, Indoprofen, Lithocholic acid sulphate, MK571, PSC833, Sildenafil, Taurochenodeoxycholate, Taurocholate, Taurolithocholate, Taurolithocholic acid sulphate, Trequinsin und Zaprinast, Dipyridamol, gegebenenfalls in Form ihrer Racemate, Enantiomere, Diastereomere und ihrer pharmakologisch verträglichen Säure-Additionssalze und Hydrate.

Stärker bevorzugt bezieht sich die Erfindung auf die Verwendung von MRP4-Inhibitoren zur Herstellung einer pharmazeutischen Zusammensetzung zur Behandlung von Atemwegserkrankungen enthaltend die erfindungsgemäßen PDE4B-Inhibitoren und MRP4-Inhibitoren, wobei die MRP4-Inhibitoren vorzugsweise ausgewählt sind aus der Gruppe bestehend aus Dehydroepiandrosterone 3-sulphate, Estradiol 3,17-disulphate, Flurbiprofen, Indomethacin, Indoprofen, MK571, Taurocholate, gegebenenfalls in Form ihrer Racemate, Enantiomere, Diastereomere und ihrer pharmakologisch verträglichen Säure-Additionssalze und Hydrate. Die Trennung von Enantiomeren aus den Racematen kann durch bekannte Verfahren nach dem Stand der Technik durchgeführt werden (z.B. durch Chromatographie an chiralen Phasen-etc.).

Mit Säure-Additionssatzen mit pharmakologisch verträglichen Säuren sind z.B. Salze ausgewählt aus der Gruppe bestehend aus Hydrochloriden, Hydrobromiden, Hydroiodiden, Hydrosulphaten, Hydrophosphaten, Hydromethanesulphonaten, Hydronitraten, Hydromaleaten, Hydroacetaten, Hydrobenzoaten, Hydrocitraten, Hydrofumaraten, Hydrotartraten, Hydrooxalaten, Hydrosuccinaten, Hydrobenzoaten and Hydro-p-toluenesulphonaten, vorzugsweise Hydrochloride, Hydrobromide, Hydrosulphate, Hydrophosphate, Hydrofumarate and Hydromethanesulphonate gemeint.

Ein weiterer Gegenstand der Erfindung sind pharmazeutische Zubereitungen, die Dreifachkombinationen von den erfindungsgemäßen PDE4B-Inhibitoren, von MRP4-Inhibitoren und einer weiteren aktiven Substanz wie z.B. einem Anticholinergikum, einem Steroid, einem LTD4-Antagonist oder einem Betamimetikurn enthalten, sowie deren Herstellung und deren Verwendung zur Behandlung von Atemwegserkrankungen.

Als iNOS-Inhibitoren gelangen hierbei vorzugsweise Verbindungen zur Anwendung, die ausgewählt sind aus der Gruppe bestehend aus: S-(2-Aminoethyl)isothiohamstoff, Aminoguanidin, 2-Aminomethylpyridin, AMT, L-Canavanin, 2-Iminopiperidin, S-Isopropylisothioharnstoff, S-Methylisothioharnstoff, S-Ethylisothioharnstoff, S-Methyltiocitrullin, S-Ethylthiocitrullin, L-NA (N^{ω}-Nitro-L-arginin), L-NAME (N^{ω}-Nitro-L-argininmethylester), L-NMMA (N^{G}-Monomethyl-L-arginin), L-NIO (N^{ω}-Iminoethyl-L-ornithin), L-NIL (N^{ω}-Iminoethyl-lysin), (S)-6-Acetimidoylamino-2-amino-hexanoic acid (1H-tetrazol-5-yl)-amid (SC-51) (J. Med. Chem. 2002, 45, 1686-1689), 1400W, (S)-4-(2-Acetimidoylamino-ethylsulfanyl)-2-amino-buttersäure (GW274150) (Bioorg. Med. Chem. Lett. 2000, 10, 597-600), 2-[2-(4-Methoxy-pyridin-2-yl)-ethyl]-3*H*-imidazo[4,5-*b*]pyridin (BYK191023) (Mol. Pharmacol. 2006, 69, 328-337), 2-((R)-3-Amino-1-phenyl-propoxy)-4-chlor-5-fluorbenzonitril (WO 01/62704), 2-((1R,3S)-3-Amino-4-hydroxy-1-thiazol-5-yl-butylsulfanyl)-6-trifluoromethyl-nicotinonitril (WO 2004/041794), 2-((1R,3S)-3-Amino-4-hydroxy-1-thiazol-5-yl-butylsulfanyl)-4-chlor-benzonitril (WO 2004/041794), 2-((1R,3S)-3-Amino-4-hydroxy-1-thiazol-5-yl-butylsulfanyl)-5-chlor-benzonitril (WO 2004/041794), (2S,4R)-2-Amino-4-(2-chlor-5-trifluoromethyl-phenylsulfanyl)-4-thiazol-5-yl-butan-1-ol (WO 2004/041794), 2-((1R,3S)-3-Amino-4-hydroxy-1-thiazol-5-yl-butylsulfanyl)-5-chlor-nicotinonitril (WO 2004/041794), 4-((S)-3-Amino-4-hydroxy-1-phenyl-butylsulfanyl)-6-methoxy-nicotinonitril (WO 02/090332), substituierte 3-Phenyl-3,4-dihydro-1-isoquinolinamin wie z.B. AR-C102222 (J. Med. Chem. 2003, 46, 913-916), (1S,5S,6R)-7-Chlor-5-methyl-2-aza-bicyclo[4.1.0]hept-2-en-3-ylamin (ONO-1714) *(*Biochem. Biophys. Res. Commun. 2000, 270, 663-667), (4R,5R)-5-Ethyl-4-methyl-thiazolidin-2-ylideneamin *(*Bioorg. Med. Chem. 2004, 12, 4101), (4R,5R)-5-Ethyl-4-methyl-selenazolidin-2-ylideneamin (Bioorg. Med. Chem. Lett. 2005, 15, 1361), 4-Aminotetrahydrobiopterin (Curr. Drug Metabol. 2002, 3, 119-121), (E)-3-(4-Chlor-phenyl)-N-(1-{2-oxo-2-[4-(6-trifluomethyl-pyrimidin-4-yloxy)-piperidin-1-yl]-ethylcarbamoyl}-2-pyridin-2-yl-ethyl)-acrylamid (FR260330) (Eur. J. Pharmacol. 2005, 509, 71-76), 3-(2,4-Difluor-phenyl)-6-[2-(4-imidazol-1-ylmethyl-phenoxy)-ethoxy]-2-phenyl-pyridin (PPA250) (J. Pharmacol. Exp. Ther. 2002, 303, 52-57), 3-{[(Benzo[1,3]dioxol-5-ylmethyl)-carbamoyl]-methyl}-4-(2-imidazol-1-yl-pyrimidin-4-yl)-piperazin-1-carbonsäuremethylester (BBS-1) (Drugs Future 2004, 29, 45-52), (R)-1-(2-Imidazol-1-yl-6-methyl-pyrimidin-4-yl)-pyrrolidin-2-carbonsäure (2-benzo[1,3]dioxol-5-yl-ethyl)-amid (BBS-2) (Drugs Future 2004, 29, 45-52) und deren pharmazeutischen Salze, Prodrugs oder Solvate.

Als iNOS-Inhibitoren im Rahmen der vorliegenden Erfindung können weiterhin antisense-Oligonucleotide, insbesondere solche antisense-Oligonucleotide, die iNOS-kodierende Nukleinsäuren binden, eingesetzt werden. Z.B. werden in WO 01/52902 antisense-Oligonucleotide, insbesondere antisense-Oligonucleotide, die iNOS kodierende Nukleinsäuren binden, zur Modulierung der Expression von iNOS beschrieben. Solche iNOS-antisense-Oligonucleotide wie insbesondere in WO 01/52902 beschrieben können daher auch aufgrund ihrer ähnlichen Wirkung wie die iNOS-Inhibitoren mit den PDE4-Inhibitoren der vorliegenden Erfindung kombiniert werden.

Als SYK-Inhibitoren gelangen hierbei vorzugsweise Verbindungen zur Anwendung, die ausgewählt sind aus der Gruppe bestehend aus: 2-[(2-aminoethyl)amino]-4-[(3-bromophenyl)amino]-5-Pyrimidinecarboxamide; 2-[[7-(3,4-dimethoxyphenyl)imidazo[1,2-c]pyrrimidin-5-yl]amino]-3-Pyridinecarboxamide; 6-[[5-fluoro-2-[3,4,5-trimethoxyphenyl)amino]-4-pyrimidinyl]amino]-2,2-dimethyl-2H-Pyrido[3,2-b]-1,4-oxazin-3(4H)-one; N-[3-bromo-7-(4-methoxyphenyl)-1,6-naphthyridin-5-yl]-1,3-Propanediamine 7-(4-methoxyphenyl)-N-methyl-1,6-Naphthyridin-5-amine; N-[7-(4-methoxyphenyl)-1,6-naphthyridin-5-yl]-1,3-Propanediamine; N-[7-(2-thienyl)-1,6-naphthyridin-5-yl-1,3-Propanediamine; N-[7-[4-(dimethylamino)phenyl]-1,6-naphthyridin-5-yl]-1,2-Ethanediamine; N-[7-(4-methoxyphenyl)-2-(trifluoromethyl)-1,6-naphthyridin-5-yl]- 1,3-Propanediamine; N-[7-(4-methoxyphenyl)-3-phenyl-1,6-naphthyridin-5-yl]-1,3-Propanediamine; N-(7-phenyl-1,6-naphthyridin-5-yl)-1,3-Propanediamine; N-[7-(3-fluorophenyl)-1,6-naphthyridin-5-yl]-1,3-Propanediamine; N-[7-(3-chlorophenyl)-1,6-naphthyridin-5-yl]-1,3-Propanediamine; N-[7-[3-(trifluoromethoxy)phenyl]-1,6-naphthyridin-5yl]-1,3-Propanediamine ; N-[7-(4-fluorophenyl)-1,6-naphthyridin-5-yl]-1,3-Propanediamine; N-[7-(4-fluorophenyl)-1,6-naphthyridin-5-yl]-1,3-Propanediamine; N-[7-(4-chlorophenyl)-1,6-naphthyridin-5-yl]-1,3-Propanediamine; N-[7-(4'-methyl[1,1'-biphenyl]-4-yl)-1,6-naphthyridin-1,3-Propanediamine; N-[7-[4-(dimethylamino)phenyl]-1,6-naphthyridin-5-yl]-1,3-Propanediamine; N-[7-[4-(diethylamino)phenyl]-1,6-naphthyridin-5-yl]-1,3-Propanediamine; N-[7-[4-(4-morpholinyl)phenyl]-1,6-naphthyridin-5-yl]-1,3-Propanediamine; N-[7-[4-[[2-(dimethylamino)ethyl]methylamino]phenyl]-1,6-naphthyridin-5-yl]-1,3-Propanediamine; N-[7-(4-bromophenyl)-1,6-naphthyridin-5-yl]-1,3-Propanediamine; N-[7-(4-methylphenyl)-1,6-naphthyridin-5-yl]-1,3-Propanediamine; N-[7-[4-(methylthio)phenyl]-1,6-naphthyridin-5-yl]-1,3-Propanediamine ; N-[7-[4-(1-methylethyl)phenyl]-1,6-naphthyridin-5-yl]-1,3-Propanediamine; 7-[4-(dimethylamino)phenyl]-N-methyl-1,6-Naphthyridin-5-amine; 7-[4-(dimethylamino)phenyl]-N,N-dimethyl-1,6-Naphthyridin-5-amine; N-[7-[4-(dimethylamino)phenyl]-1,6-naphthyridin-5-yl]-1,4-Butanediamine; N-[7-[4-(dimethylamino)phenyl]-1,6-naphthyridin-5-yl]-1,5-Pentanediamine; 3-[[7-[4-(dimethylamino)phenyl]-1,6-naphthyridin-5-yl]oxy]-1-Propanol; 4-[5-(4-aminobutoxy)-1,6-naphthyridin-7-yl]-N,N-dimethyl-Benzenamine; 4-[[7-[4-(dimethylamino)phenyl]-1,6-naphthyridin-5-yl]amino]-1-Butanol; N-[7-[4-(dimethylamino)phenyl]-1,6-naphthyridin-5-yl]-N-methyl-1,3-Propanediamine; N-[7-[4-(dimethylamino)phenyl]-1,6-naphthyridin-5-yl]-N'-methyl-1,3-Propanediamine; N-[7-[4-(dimethylamino)phenyl]-1,6-naphthyridin-5-yl]-N,N'-dimethyl-1,3-Propanediamine; 1-amino-3-[[7-[4-(dimethylamino)phenyl]-1,6-naphthyridin-5-yl]amino]-2-Propanol; N-[7-[4-(dimethylamino)phenyl]-1,6-naphthyridin-5-yl]-2,2-dimethyl-1,3-Propanediamine; 7-[4-(dimethylamino)phenyl]-N-(3-pyridinylmethyl)-1,6-Naphthyridin-5-amine; N-[(2-aminophenyl)methyl]-7-[4-(dimethylamino)phenyl]-1,6-Naphthyridin-5-amine; N-[7-[6-(dimethylamino)[1,1'-biphen]-3-yl]-1,6-naphthyridin-5-yl]-1,3-Propanediamine, N-[7-[3-chloro-4-(diethylamino)phenyl]-1,6-naphthyridin-5-yl]-1,3-Propanediamine; N-[7-[4-(dimethylamino)-3-methoxyphenyl]-1,6-naphthyridin-5-yl]-1,3-Propanediamine; N-[7-[4-(diethylamino)phenyl]-3-methyl-1,6-naphthyridin-5-yl]-1,3-Propanediamine; N-[7-(3'-fluoro[1,1'-biphenyl]-3-yl)-1,6-naphthyridin-5-yl]-1,2-Ethanediamine, N-[7-(4-methoxyphenyl)-1,6-naphihyridin-5-yl]-1,6-Naphthyridine-1,3-Propanediamine; N,N'-bis(3-aminopropyl)-7-(4-methoxyphenyl)-2,5-diamine; N-[7-(4-methoxyphenyl)-2-(phenylmethoxy)-1,6-naphthyridin-5-yl]-1,6-Naphthyridine-1,3-Propanediamine; N5-(3-aminopropyl)-7-(4-methoxyphenyl)-N2-(phenylmethyl)-2,5-diamine; N-[7-(2-naphthalenyl)-1,6-naphthyridin-5-yl]-1,3-Propanediamine; N-[7-(2'-fluoro[1,1'-biphenyl]-4-yl)-1,6-naphthyridin-5-yl]-1,3-Propanediamine; N-[7-(3,4,5-trimethoxyphenyl)-1,6-naphthyridin-5-yl]-1,3-Propanediamine; N-[7-(3,4-dimethylphenyl)-1,6-naphthyridin-5-yl]-1,3-Propanediamine; 1-amino-3-[[7-(2-naphthalenyl)-1,6-naphthyridin-5-yl]amino]-2-Propanol; 1-amino-3-[[7-(2'-fluoro[1,1'-biphenyl]-4-yl)-1,6-naphthyridin-5-yl]amino]-2-Propanol; 1-amino-3-[17-(4'-methoxy[1,1'-biphenyl]-4-yl)-1,6-naphthyridin-5-yl]amino]-2-Propanol; 1-amino-3-[[7-(3,4,5-trimethoxyphenyl)-1,6-naphthyridin-5-yl]amino]-2-Propanol; 1-amino-3-[[7-(4-bromophenyl)-1,6-naphthyridin-5-yl]amino]-2-Propanol; N-[7-(4'-methoxy[1,1'-biphenyl]-4-yl)-1,6-naphthyridin-5-yl]-2,2-dimethyl-1,3-Propanediamine; 1-[[7-[4-(dimethylamino)phenyl]-1,6-naphthyridin-5-yl]amino]-2-Propanol; 2-[[2-[[7-[4-(dimethylamino)phenyl]-1,6-naphthyridin-5-yl]amino]ethyl]thio]-Ethanol; 7-[4-(dimethylamino)phenyl]-N-(3-methyl-5-isoxazolyl)-1,6-Naphthyridin-5-amine; 7-[4-(dimethylamino)phenyl]-N-4-pyrimidinyl-1,6-Naphthyridin-5-amine; N-[7-[4-(dimethylamino)phenyl]-1,6-naphthyridin-5-yl]-1,3-Cyclohexanediamine; N,N-dimethyl-4-[5-(1-piperazinyl)-1,6-naphthyridin-7-yl]-Benzenamine; 4-[5-(2-methoxyethoxy)-1,6-naphthyridin-7-yl]-N,N-dimethyl-Benzenamine; 1-[7-[4-(dimethylamino)phenyl]-1,6-naphthyridin-5-yl]-4-Piperidinol; 1-[7-[4-(dimethylamino)phenyl]-1,6-naphthyridin-5-yl]-3-Pyrrolidinol-, 7-[4-(dimethylamino)phenyl]-N-(2-furanylmethyl)-1,6-Naphthyridin-5-amine; 7-[4-(dimethylamino)phenyl]-N-[3-(1H-imidazol-1-yl)propyl]-1,6-Naphthyridin-5-amine; 1-[7-[4-(dimethylamino)phenyl]-1,6-naphthyridin-5-yl]-4-Piperidinecarboxamide; 1-[3-[[7-[4-(dimethylamino)phenyl]-1,6-naphthyridin-5-yl]amino]propyl]-2-Pyrrolidinone; N-[3'-[5-[(3-aminopropyl)amino]-1,6-naphthyridin-7-yl][1,1'-biphenyl]-3-yl]-Acetamide; N-[7-(4'-fluoro[1,1'-biphenyl]-4-yl)-1,6-naphthyridin-5-yl]-1,3-Propanediamine; N-[4'-[5-[(3-aminopropyl)amino]-1,6-naphthyridin-7-yl][1,1'-biphenyl]-3-yl]-Acetamide; N-[7-[4-(1,3-benzodioxol-5-yl)phenyl]-1,6-naphthyridin-5-yl]-1,3-Propanediamine; N-[7-[4-(2-thienyl)phenyl]-1,6-naphthyridin-5-yl]-1,3-Propanediamine; N-[7-[4-fluoro-3-(trifluoromethyl)phenyl]-1,6-naphthyridin-5-yl]-1,3-Propanediamine; N-[7-[4-(3-pyridinyl)phenyl]-1,6-naphthyridin-5-yl]-1,3-Propanediamine; N-[7-(1,3-benzodioxol-5-yl)-1,6-naphthyridin-5-yl]-1,3-Propanediamine; N-[7-(6-methoxy-2-naphthalenyl)-1,6-naphthyridin-5-yl]-1,3-Propanediamine; 7-[4-(dimethylamino)phenyl]-N-(4-pyridinylmethyl)-1,6-Naphthridin-5-amine ; 3-[[7-[4-(dimethylamino)phenyl]-1,6-naphthyridin-5-yl]methylamino]-Propanenitrile; 7-[4-(dimethylamino)phenyl]-N-[1-(phenylmethyl)-4-piperidinyl]-1,6-Naphthyridin-5-amine; N-[7-[4-(dimethylamino)phenyl]-1,6-naphthyridin-5-yl]-1,2-Cyclohexanediamine, (1R,2S)-rel-. N-[7-[4-(dimethylamino)phenyl]-1,6-naphthyridin-5-yl]-1,2-Benzenedimethanamine; N-[7-[4-(diethylamino)phenyl]-1,6-naphthyridin-5-yl]-1,4-Butanediamine; N-[7-[3',5'-bis(trifluoromethyl)[1,1'-biphenyl]-4-yl]-1,6-naphthyridin-5-yl]-3-Propanediamine; N-[7-(3'-methoxy[1,1'-biphenyl]-4-yl)-1,6-naphthyridin-5-yl]-1,3-Propanediamine; N-[7-(3'-fluoro[1,1'-biphenyl]-4-yl)-1,6-naphthyridin-5-yl]-1,3-Propanediamine; 4-[[7-[4-(dimethylamino)phenyl]-1,6-naphthyridin-5-yl]oxy]-1-Butanol; N-[7-[4-(dimethylamino)phenyl]-1,6-naphthyridin-5-yl]-1,4-Cyclohexanediamine; 7-[4-(dimethylamino)phenyl]-N-(2,2,6,6-tetramethyl-4-piperidinyl)-1,6-Naphthyridin-5-amine; N-[7-[3-bromo-4-(dimethylamino)phenyl]-1,6-naphthyridin-5-yl]-1,3-Propanediamine; N-[7-(1-methyl-1H-indol-5-yl)-1,6-naphthyridin-5-yl]-1,3-Propanediamine; N-[7-[3-(trifluoromethyl)phenyl]-1,6-naphthyridin-5-yl]-1,3-Propanediamine; N-[7-[4-(trifluoromethyl)phenyl]-1,6-naphthyridin-5-yl]-1,3-Propanediamine; N-[7-(3-bromo-4-methoxyphenyl)-1,6-naphthyridin-5-yl]-1,3-Propanediamine; N-[7-[4-[[3-(dimethylamino)propyl]methylamino]phenyl]-1,6-naphthyridin-5-yl]-1,4-Cyclohexanediamine; N-[7-[4-[[2-(dimethylamino)ethyl]methylamino]phenyl]-1,6-naphthyridin-5-yl]-1,4-Cyclohexanediamine; N-[7-[4-(dimethylamino)-3-methoxyphenyl]-1,6-naphthyridin-5-yl]-1,4-Cyclohexanediamine; N-[7-[4-(4-morpholinyl)phenyl]-1,6-naphthyridin-5-yl]-1,4-Cyclohexanediamine; N-[7-[3-bromo-4-(4-morpholinyl)phenyl]-1,6-naphthyridin-5-yl]-1,4-Cyclohexanediamine; 4-[[7-[4-[[2-(dimethylamino)ethyl]methylamino]phenyl]-1,6-naphthyridin-5-yl]oxy]-Cyclohexanol; N-[7-[3-bromo-4-(4-morpholinyl)phenyl]-1,6-naphthyridin-5-yl]-1,3-Propanediamine; N,N-dimethyl-4-[5-(4-methyl-1-piperazinyl)-1,6-naphthyridin-7-yl]-Benzenamine; 4-[[7-[4-[[3-(dimethylamino)propyl]methylamino]phenyl]-1,6-naphthyridin-5-yl]oxy]-Cyclohexanol; N-[7-[4-[[2-(dimethylamino)ethyl]methylamino]phenyl]-1,6-naphthyridin-5-yl]-1,4-Butanediamine; [3-[[5-[(3-aminopropyl)amino]-7-(4-methoxyphenyl)-1,6-naphthyridin-2-yl]amino]propyl]-Carbamic acid-1,1-dimethylethyl ester.

### DARREICHUNGSFORMEN

Geeignete Anwendungsformen sind beispielsweise Tabletten, Kapseln, Lösungen, Säfte, Emulsionen oder Inhalationspulver oder -aerosole. Hierbei soll der Anteil der pharmazeutisch wirksamen Verbindung(en) jeweils im Bereich von 0, 1 bis 90 Gew.%, bevorzugt 0, 5 bis 50 Gew.-% der Gesamtzusammensetzung liegen, d.h. in Mengen die ausreichend sind, um den unten angegebenen Dosierungsbereich zu erreichen.

Die orale Gabe kann in Form einer Tablette, als Pulver, als Pulver in einer Kapsel (z.B. Hartgelatinekapsel), als Lösung oder Suspension erfolgen. Im Fall einer inhalativen Gabe kann die Wirkstoffkombination als Pulver, als wässrige oder wässrig-ethanolische Lösung oder mittels einer Treibgasformulierung erfolgen.

Bevorzugt sind deshalb pharmazeutische Formulierungen gekennzeichnet durch den Gehalt an einer oder mehrerer Verbindungen der Formel 1 gemäß der obigen bevorzugten Ausführungsformen.

Besonders bevorzugt ist es, wenn die Verbindungen der Formel 1 oral verabreicht werden, besonders bevorzugt ist es, wenn die Verabreichung ein oder zweimal täglich erfolgt. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein Geschmack verbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmacksaufbesserern oder Farbstoffen versetzt werden.

Ebenfalls bevorzugt ist es, wenn die Verbindungen der Formel 1 inhalativ verabreicht werden, besonders bevorzugt ist es, wenn die Verabreichung ein oder zweimal täglich erfolgt. Hierzu müssen die Verbindungen der Formel 1 in inhalierbaren Darreichungsformen bereitgestellt werden. Als inhalierbare Darreichungsformen kommen Inhalationspulver, treibgashaltige Dosieraerosole oder treibgasfreie Inhalationslösungen in Betracht, die gegebenenfalls im Gemisch mit gebräuchlichen physiologisch verträglichen Hilfsstoffen vorliegen.

Im Rahmen der vorliegenden Erfindung sind von dem Begriff treibgasfreie Inhalationslösungen auch Konzentrate oder sterile, gebrauchsfertige Inhalationslösungen umfasst. Die im Rahmen der vorliegenden Erfindung einsetzbaren Darreichungsformen werden im nachfolgenden Teil der Beschreibung detailliert beschrieben.

### Inhalationspulver

Sind die Verbindungen der Formel **1** im Gemisch mit physiologisch unbedenklichen Hilfsstoffen enthalten, können zur Darstellung der erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung. Verfahren zur Herstellung der erfindungsgemäßen Inhalationspulver durch Mahlen und Mikronisieren sowie durch abschließendes Mischen der Bestandteile sind aus dem Stand der Technik bekannt.

### Treibgashaltige Inhalationsaerosole

Die im Rahmen der erfindungsgemäßen Verwendung einsetzbaren treibgashaltigen Inhalationsaerosole können die Verbindungen der Formel **1** im Treibgas gelöst oder in dispergierter Form enthalten. Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie bevorzugt fluorierten Derivaten des Methans, Ethans, Propans, Butans, Cyclopropans oder Cyclobutans. Die vorstehend genannten Treibgase können dabei allein oder in Mischungen derselben zur Verwendung kommen. Besonders bevorzugte Treibgase sind fluorierte Alkanderivate ausgewählt aus TG134a (1, 1, 1, 2-Tetrafluorethan), TG227 (1, 1, 1, 2, 3, 3, 3-Heptafluorpropan) und Mischungen derselben. Die im Rahmen der erfindungsgemäßen Verwendung einsetzbaren treibgashaltigen Inhalationsaerosole können ferner weitere Bestandteile wie Co-Solventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

### Treibgasfreie Inhalationslösungen

Die erfindungsgemäße Verwendung von Verbindungen der Formel **1** erfolgt bevorzugt zur Herstellung von treibgasfreien Inhalationslösungen und Inhalationssuspensionen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Die Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

Den im Rahmen der erfindungsgemäßen Verwendung einsetzbaren treibgasfreien Inhalationslösungen können Co-Solventien und/oder weitere Hilfsstoffe zugesetzt werden. Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester.

Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien. Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine. Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration.

Für die oben beschriebenen Behandlungsformen werden gebrauchsfertige Packungen eines Medikaments zur Behandlung von Atemwegserkrankungen, beinhaltend eine beigelegte Beschreibung, welche beispielsweise die Worte Atemwegserkrankung, COPD oder Asthma enthalten, Dihydrothienopyrimidin und ein oder mehrere Kombinationspartner ausgewählt aus der oben beschriebenen Gruppe, bereit gestellt.

## Patentansprüche

1. Verbindungen der Formel **1** 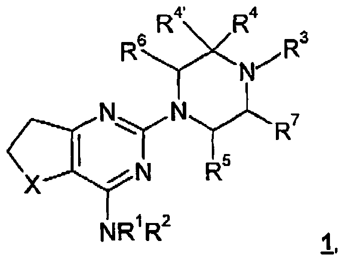 worin
**X** SO oder SO₂,
**R¹** H, C₁₋₈-Alkyl,
**R²** H ist oder ein Rest ausgewählt aus der Gruppe bestehend aus C₁₋₁₀-Alkyl und C₂₋₆-Alkenyl ist, der gegebenenfalls durch einen oder mehrere Reste ausgewählt aus Halogen und C₁₋₃-Fluoroalkyl substituiert sein kann oder der gegebenenfalls durch einen oder mehrere Reste ausgewählt aus der Gruppe bestehend aus OR²¹, COOR^{2.1}, CONR^{2.2}R^{2.3}, SR²¹, SO-R²¹, SO₂-R^{2.1}, C₆₋₁₀-Aryl, einem Het, einem Hetaryl, einem mono- oder bicyclischem C₃₋₁₀-Cycloalkyl, CH₂-NR^{2.2}R^{2.3} und NR^{2.2}R^{2.3} substituiert sein kann, welcher wiederum gegebenenfalls durch einen oder mehrere Reste ausgewählt aus der Gruppe bestehend aus OH, Halogen, OR^{2.1}, Oxo, CF₃, CHF₂, CH₂F, C₁₋₆-Alkyl, C₁₋₆-Alkanol, C₆₋₁₀-Aryl, COOR^{2.1}, CH₂-NR^{2.2}R^{2.3} und NR^{2.2}R^{2.3} substituiert sein kann,
wobei **R^{2.1}** H ist oder ein Rest ausgewählt ist aus der Gruppe bestehend aus C₁₋₆-Alkyl, C₁₋₆-Alkanol, C₁₋₃-Fluoroalkyl, mono- oder bicyclisches C₃₋₁₀ Cycloalkyl, C₆₋₁₀₋Aryl-C₁₋₆-alkylen, Hetaryl-C₁₋₆-alkylen, Het-C₁₋₆-alkylen, C₃₋₁₀-Cycloalkyl-C₁₋₆-alkylen, ein mono- oder bicyclisches C₆₋₁₀-Aryl, ein Hetaryl und ein Het, der Gegebenenfalls durch einen oder mehrere Reste ausgewählt aus der Gruppe bestehend aus OH, Q-(C₁₋₃-Alkyl), Halogen, C₁₋₆-Alkyl und C₆₋₁₀-Aryl substituiert sein kann,
wobei **R^{2.2}** und **R^{2.3}** unabhängig voneinander H sind oder ein Rest ausgewählt sind aus der Gruppe bestehend aus C₁₋₆-Alkyl, mono- oder bicyclisches C₃₋₁₀-Cycloalkyl, C₆₋₁₀-Aryl-C₁₋₆-alkylen, Hetaryl-C₁₋₆-alkylen, mono- oder bicyclisches C₆₋₁₀-Aryl, ein Het ein Hetaryl, CO-NH₂, CO-NH(CH₃), CO-N(CH₃)₂, SO₂-(C₁₋₂-Alkyl), CO-R^{2.1} und COOR^{2.1},
der gegebenenfalls durch einen oder mehrere Reste ausgewählt aus der Gruppe bestehend aus OH, Halogen, C₁₋₆-Alkyl, C₆₋₁₀-Aryl und COOR^{2.1} substituiert sein kann,
wobei
**Het** ein drei- bis elfgliedriger, mono- oder bicyclischer, gesättigter oder teilweise gesättigter, gegebenenfalls annellierter oder gegebenenfalls überbrückter Heterocyclus ist, der 1, 2, 3 oder 4 Heteroatome unabhängig voneinander ausgewählt aus der Gruppe bestehend aus N, S oder O enthält, und wobei
**Hetaryl** ein fünf- bis zehngliedriger, mono- oder bicyclisches, gegebenenfalls annelliertes Heteroaryl ist, das 1, 2, 3 oder 4 Heteroatome unabhängig voneinander ausgewählt aus der Gruppe bestehend aus N, S oder O enthält,
und wobei
**Cycloalkyl** gesättigt oder teilweise gesättigt sein kann,
oder
**R²** ein mono- oder polycyclisches C₉₋₁₀ Cycloalkyl, das gegebenenfalls einfach oder mehrfach über C₁₋₃-Alkylgruppen verbrückt sein kann und das gegebenenfalls mit einem Rest ausgewählt aus der Gruppe bestehend aus verzweigtes oder unverzweigtes C₁₋₆₋Alkanol, C₆₋₃-Fluoroalkyl, C₁₋₃-alkylen-OR^{2.1}, OR^{2.1}, COOR^{2.1}, SO₂-NR^{2.2}R^{2.3}, Het, C₆₋₁₀-Aryl, C₁₋₆-Alkyl, C₆₋₁₀-Aryl-C₁₋₆-alkylen, Hetaryl-C₁-6-alkylen, mono- oder bicyclisches C₃₋₁₀ Cycloalkyl und NR^{2.2}R^{2.3} substituiert sein kann, der gegebenenfalls durch einen oder mehrere Reste ausgewählt aus der Gruppe bestehend aus OH, OR^{2.1}, Oxo, Halogen, CF₃, CHF₂, CH₂F, C₁₋₆-Alkyl, C₆₋₁₀-Aryl und NR^{2.2}R^{2.3} substituiert sein kann,
oder
**R²** ein mono- oder polycyclisches C₆₋₁₀-Aryl, das gegebenenfalls durch OH, SH oder Halogen oder durch einen oder mehrere Reste ausgewählt aus der Gruppe bestehend aus OR^{2.1}, COOR^{2.1}, NR^{2.2}R^{2.3}, CH₂-NR^{2.2}R^{2.3}, C₃₋₁₀-Cycloalkyl, Het, C₁₋₆-Alkyl, C₁₋₃-Fluoroalkyl, C₆₋₁₀-Aryl-C₁₋₆-alkylen, Het-C₁₋₆-alkylen, Hetaryl-C₁₋₆-alkylen, C₆₋₁₀-Aryl, SO₂-CH₃, SO₂-CH₂CH₃ und SO₂-NR^{2.2}R^{2.3} substituiert sein kann, der wiederum gegebenenfalls durch einen oder mehrere oder mehrere Reste ausgewählt aus der Gruppe bestehend aus OH, OR^{2.1}, Oxo, Halogen, CF₃, CHF₂, CH₂F, C₁₋₆-Alkyl, C₆₋₁₀-Aryl und NR^{2.2}R^{2.3} substituiert sein kann,
oder
**R²** ein Rest ausgewählt aus einer Gruppe bestehend aus einem Het und einem Hetaryl, welcher gegebenenfalls durch einen oder mehrere Reste ausgewählt aus der Gruppe Halogen, OH, Oxo, CF₃, CHF₂ und CH₂F oder durch einen oder mehrere Reste ausgewählt aus der Gruppe OR^{2.1}, C₁₋₃-alkylen-OR^{2.1}, SR^{2.1}, SO-R^{2.1} und SO₂-R^{2.1} COOR^{2.1}, COR^{2.1}, C₁₋₆-Alkanol, C₃₋₁₀-Cycloalkyl, C₆₋₁₀-Aryl, C₁₋₆-Alkyl, C₆₋₁₀-Aryl-C₁₋₆-alkylen, Hetaryl-C₁₋₆-alkylen, Het, Hetaryl, C₁₋₃-alkylen-OR^{2.1} und NR^{2.2}R^{2.3} substituiert sein kann,
der gegebenenfalls wiederum durch einen oder mehrere Reste ausgewählt aus der Gruppe bestehend aus OH, OR^{2.1}, Oxo, Halogen, CF₃, CHF₂, CH₂F, C₁₋₆-Alkyl, C₆₋₁₀-Aryl und NR^{2.2}R^{2.3} substituiert sein kann,
oder worin
**NR¹R²** gemeinsam einen heterocyclischen vier- bis siebengliedrigen Ring bedeutet, der gegebenenfalls überbrückt sein kann, der 1, 2 oder 3 Heteroatome ausgewählt aus der Gruppe bestehend aus N, O und S enthält und der gegebenenfalls durch einen oder mehrere Reste ausgewählt aus der Gruppe bestehend aus OH, OR^{2.1}, C₁₋₃-alkylen-O^{R.1}, Oxo, Halogen, C₁₋₆-Alkyl, C₆₋₁₀-Aryl, COOR^{2.1}, CH₂-NR^{2.2}-COO-R^{2.1}, CH₂-NR^{2.2}-CO-R^{2.1}, CH₂-NR²²-CO-CH₂-NR^{2.2}R^{2.3}, CH₂-NR^{2.2}-SO₂-C₁₋₃-Alkyl, CH₂-NR^{2.2}-SO₂-NR^{2.2}R^{2.3}, CH₂-NR^{2.2}-CO-NR^{2.2}R^{2.3}, CO-NR^{2.2}R^{2.3}, CH₂-NR^{2.2}R^{2.3} und NR^{2.2}R^{2.3} substituiert sein kann,
und worin
R³ ein Rest ausgewählt aus der Gruppe bestehend aus einem mono- oder bicyclischen C₆₋₁₀-Aryl, einem Het und einem Hetaryl ist, der gegebenenfalls mit einem oder mehreren Resten ausgewählt aus der Gruppe Halogen, -C₁₋₃-Alkyl, Cyclopropyl, -C₁₋₃-Fluoroalkyl, -C₁₋₃-alkylen-OR²¹, -OH und -O-C₁₋₃-Alkyl substituiert sein kann,
und worin
a) R³, R⁴, R^{4'} und R⁵ H sind und
**R⁶ und R⁷** gemeinsam eine Brücke ausgewählt aus der Gruppe bestehend aus Methylen, Ethylen und Propylen bilden, der gegebenenfalls mit einem Rest ausgewählt aus der Gruppe bestehend aus -CH₃, -OH, -F, -CF₃, -CHF₂, -CH₂F, -NH₂, -NH(C₁₋₃-Alkyl), -N(C₁₋₃-Alkyl)₂ und O-(C₁₋₃-Alkyl) substituiert sein kann,
oder wobei
b) R³, R⁴, R^{4'} und R⁷ H sind und
**R⁵ und R⁶** gemeinsam eine Brücke ausgewählt aus der Gruppe bestehend aus Methylen, Ethylen und Propylen bilden, der gegebenenfalls mit einem Rest ausgewählt aus der Gruppe bestehend aus CH₃, -OH, -F, -CF₃, -CHF₂, -CH₂F, -NH₂, -NH(C₁₋₃-Alkyl), -N(C₁₋₃-Alkyl)₂ und OCH₃ substituiert sein kann,
oder wobei
c) R⁵, R⁶, R^{4'} und R⁷ H sind und
**R³ und R⁴** gemeinsam mit dem C- und dem N-Atom des Piperazins einen gesättigten oder teilweise ungesättigten 5- oder 6-gliedrigen Heterocyclus enthaltend zwei oder drei, mindestens jedoch zwei Stickstoffatome bilden
oder wobei
d) R⁵, R⁶ uns R⁷ H sind und
**R³, R⁴ und R^{4'}** gemeinsam mit dem C- und dem N-Atom des Piperazins einen ungesättigten oder teilweise ungesättigten, 5- oder 6-gliedrigen Heterocyclus enthaltend zwei oder drei, mindestens jedoch zwei Stickstoffatome bilden,
wobei jeder dieser gesättigten, ungesättigten oder teilweise ungesättigten, 5- oder 6-gliedrigen Heterocyclen jeweils gegebenenfalls mit einem, zwei oder drei Resten ausgewählt aus der Gruppe bestehend aus Oxo, OH, Halogen, C₁₋₆-Alkyl, Cyclopropyl, C₁₋₃-Fluoroalkyl, C₁₋₃-alkylen-OR^{2.1}, C₁₋₆-Alkanol, COOR^{2.1}, C₆₋₁₀-Aryl, C₁₋₃-Alkylen-C₆₋₁₀-Aryl, Hetaryl, C₁₋₃-Alkylen-Hetaryl, Het, CO-Het, C₁₋₃-Alkylen-Het, -CO-N(C₁₋₃-Alkyl)-Het, -CO-NH-Het, -CO-O-N(C₁₋₃-Alkyl)-Het, -CO-O-NH-Het,
-CO-N(C₁₋₃-Alkyl)-C₃₋₇-Cycloalkyl,
-CO-NH-C₃₋₇-Cycloalkyl, -CO-O-N(C₁₋₃-Alkyl)-C₃₋₇-Cycloalkyl, -CO-O-NH-C₃₋₇-Cycloalkyl, -CO-NH-(C₁₋₂-Alkylen)-Hetaryl, -CO-N(C₁₋₃-Alkyl)-(C₁₋₂-Alkylen)-Hetaryl, -CO-NH-CH(C₁₋₃-Alkyl)-Hetaryl, -CO-NH-C(C₁₋₂-Alkyl)₂-Hetaryl, -CO-N(Cyclopropyl)-Het und -CO-N(Cyclopropyl)-Hetaryl substituiert sein kann, welcher wiederum gegebenenfalls mit einem oder mehreren Resten ausgewählt aus der Gruppe OH, Oxo, Halogen, C₁₋₆-Alkyl, C₁₋₃-Fluoroalkyl, C₁₋₆-Alkanol substituiert sein kann,
sowie pharmakologisch verträgliche Salze davon.

2. Verbindungen der Formel 1 nach Anspruch 1, wobei
X SO oder SO₂,
R¹ H
R² H ist oder C₁₋₁₀-Alkyl ist, das gegebenenfalls durch einen oder mehrere Reste ausgewählt aus Halogen und C₁₋₃-Fluoroalkyl substituiert sein kann oder der gegebenenfalls durch einen oder mehrere Reste ausgewählt aus der Gruppe bestehend aus OR^{2.1}, COOR^{2.1}, CONR^{2.2}R^{2.3}, SR^{2.1}, SO-R^{2.1}, SO₂-R^{2.1}, C₆₋₁₀-Aryl, einem Het, einem Hetaryl, einem mono- oder bicyclischem C₃₋₁₀-Cycloalkyl, CH₂-NR^{2.2}R^{2.3} und NR^{2.2}R^{2.3} substituiert sein kann, welcher wiederum gegebenenfalls durch einen oder mehrere Reste ausgewählt aus der Gruppe bestehend aus OH, Halogen, OR^{2.1}, Oxo, CF₃, CHF₂, CH₂F, C₁₋₆-Alkyl, C₁₋₆-Alkanol, C₆₋₁₀-Aryl, COOR^{2.1}, CH₂-NR^{2.2}R^{2.3} und NR^{2.2}R^{2.3} substituiert sein kann,
wobei
Het ein drei- bis siebengliedriger, monocyclischer, gesättigter oder teilweise gesättigter Heterocyclus oder ein sieben- bis elfgliedriger, bicyclischer, gesättigter oder teilweise gesättigter Heterocyclus ist, der 1, 2, 3 oder 4 Heteroatome unabhängig voneinander ausgewählt aus der Gruppe bestehend aus N, S oder O enthält, und wobei
Hetaryl ein fünf bis sechsgliedriges, monocyclisches, aromatisches Heteroaryl oder ein sieben- bis elfgliedriges, bicyclisches, aromatisches Heteroaryl ist, das jeweils 1, 2, 3 oder 4 Heteroatome unabhängig voneinander ausgewählt aus der Gruppe bestehend aus N, S oder O enthält,
und wobei
Cycloalkyl gesättigt oder teilweise gesättigt sein kann, wobei R^{2.1}, R^{2.2} und R^{2.3} wie in Anspruch 1 definiert ist
oder
R² ein mono- oder polycyclisches C₃₋₁₀ Cycloalkyl, das gegebenenfalls einfach oder mehrfach über C₁₋₃-Alkylgruppen verbrückt sein kann und das gegebenenfalls mit einem Rest ausgewählt aus der Gruppe bestehend aus verzweigtes oder unverzweigtes C₁₋₆-Alkanol, C₁₋₃-alkylen-OR^{2.1}, OR^{2.1}, C₁₋₃-Fluoroalkyl, COOR^{2.1}, SO₂-NR^{2.2}R^{2.3}, Het, C₆₋₁₀-Aryl, C₁₋₆-Alkyl, C₆₋₁₀-Aryl-C₁₋₆-alkylen, Hetaryl-C₁₋₆-alkylen, mono- oder bicyclisches C₃₋₁₀ Cycloalkyl und NR^{2.2}R^{2.3} substituiert sein kann, der gegebenenfalls durch einen oder mehrere Reste ausgewählt aus der Gruppe bestehend aus OH, OR^{2.1}, Oxo, Halogen, CF₃, CHF₂, CH₂F, C₁₋₆-Alkyl, C₆₋₁₀-Aryl und NR^{2.2}R^{2.3} substituiert sein kann,
oder
**R²** ein mono- oder polycyclisches C₆₋₁₀-Aryl, das gegebenenfalls durch OH, SH oder Halogen oder durch einen oder mehrere Reste ausgewählt aus der Gruppe bestehend aus OR^{2.1}, COOR^{2.1}, NR^{2.2}R^{2.3}, CH₂-NR^{2.2}R^{2.3}, C₃₋₁₀-Cycloalkyl, Het, C₁₋₆-Alkyl, C₁₋₃-Fluoroalkyl, C₆₋₁₀-Aryl-C₁₋₆-alkylen Het-C₁₋₆-alkylen, Hetaryl-C₁₋₆-alkylen, C₆₋₁₀-Aryl, SO₂-CH₃, SO₂-CH₂CH₃ und SO₂-NR^{2.2}R^{2.3} substituiert sein kann, der wiederum gegebenenfalls durch einen oder mehrere oder mehrere Reste ausgewählt aus der Gruppe bestehend aus OH, OR^{2.1}, Oxo, Halogen, CF₃, CHF₂, CH₂F, C₁₋₆-Alkyl, C₆₋₁₀-Aryl und NR^{2.2}R^{2.3} substituiert sein kann,
oder
**R²** ein Rest ausgewählt aus einer Gruppe bestehend aus einem Het und einem Hetaryl, welcher gegebenenfalls durch einen oder mehrere Reste ausgewählt aus der Gruppe Halogen, CF₃, CHF₂, CH₂F, OH und Oxo oder durch einen oder mehrere Reste ausgewählt aus der Gruppe OR^{2.1}, C₁₋₃-alkylen-OR^{2.1}, SR^{2.1}, SO-R^{2.1}, SO₂-R^{2.1}, COOR^{2.1}, COR^{2.1}, C₁₋₆-Alkanol, C₁₋₃-alkylen-OR^{2.1}, C₃₋₁₀-Cycloalkyl, C₆₋₁₀-Aryl, C₁₋₆-Alkyl, C₆₋₁₀-Aryl-C₁₋₆-alkylen, Hetaryl-C₁₋₆-alkylen, Het, Hetaryl und NR^{2.2}R^{2.3} substituiert sein kann,
der gegebenenfalls wiederum durch einen oder mehrere Reste ausgewählt aus der Gruppe bestehend aus OH, OR^{2.1}, Oxo, Halogen, CF₃, CHF₂, CH₂F, C₁₋₆-Alkyl, C₆₋₁₀-Aryl und NR^{2.2}R^{2.3} substituiert sein kann,
und worin
**R³** ein Rest ausgewählt aus der Gruppe bestehend aus einem mono- oder bicyclischen C₆₋₁₀-Aryl, einem Het und einem Hetaryl, der gegebenenfalls mit einem oder mehreren Resten ausgewählt aus der Gruppe Halogen, -C₁₋₃-Alkyl, Cyclopropyl, -C₁₋₃-Fluoroalkyl, -C₁₋₃-alkylen-OR^{2.1}. -OH und -O-C₁₋₃-Alkyl substituiert sein kann
und worin
a) R³, R⁴, R^{4'} und R⁵ H sind und
**R⁶ und R⁷** gemeinsam eine Brücke ausgewählt aus der Gruppe bestehend aus Methylen, Ethylen und Propylen bilden,
oder wobei
b) R³, R⁴, R^{4'} und R⁷ H sind und
**R⁶ und R⁶** gemeinsam eine Brücke ausgewählt aus der Gruppe bestehend aus Methylen, Ethylen und Propylen bilden,
oder wobei
d) R⁵, R⁶ und R⁷ H sind und
**R^{3,} R⁴ und R^{4'}** gemeinsam mit dem C- und dem N-Atom des Piperazins einen ungesättigten oder teilweise ungesättigten, 5- oder 6-gliedrigen Heterocyclus enthaltend zwei oder drei, mindestens jedoch zwei Stickstoffatome bilden, wobei dieser Heterocyclus gegebenenfalls mit einem, zwei oder drei Resten ausgewählt aus der Gruppe bestehend aus Oxo, OH, Halogen, C₁₋₆-Alkyl, Cyclopropyl, C₁₋₆-Fluoroalkyl, C₁₋₃-alkylen-OR^{2.1}, C₁₋₆-Alkanol, COOR^{2.1}, C₆₋₁₀-Aryl, C₁₋₃-Alkylen-C₆₋₁₀-Aryl, Hetaryl, C₁₋₃-Alkylen-Hetaryl, Het, CO-Het, C₁₋₃-Alkylen-Het, -CO-N(CH₃)-Het, -CO-NH-Het, -CO-N(CH₃)-C₃₋₇₋Cycloalkyl, -CO-NH-C₃₋₇-Cycloalkyl, -CO-NH-C₁₋₂-Alkylen-Hetaryl, -CO-N(CH₃)-C₁₋₂-Alkylen-Hetaryl, -CO-NH-CH(CH₃)-Hetaryl, -CO-NH-C(CH₃)₂-Hetaryl, -CO-N(Cyclopropyl)-Het und -CO-N(Cyclopropyl)-Hetaryl substituiert sein kann, welcher wiederum gegebenenfalls mit einem oder mehreren Resten ausgewählt aus der Gruppe OH, Oxo, Halogen, C₁₋₆-Alkyl, C₁₋₃-Fluoroalkyl, C₁₋₆-Alkanol substituiert sein kann,
sowie pharmakologisch verträgliche Salze davon.

3. Verbindungen der Formel 1 nach einem der Ansprüche 1 oder 2, wobei
**X** SO,
R¹ H
R² H ist oder C₁₋₁₀-Alkyl ist, der gegebenenfalls durch einen oder mehrere Reste ausgewählt aus Halogen und C₁₋₃-Fluoroalkyl substituiert sein kann oder der gegebenenfalls durch einen oder mehrere Reste ausgewählt aus der Gruppe bestehend aus OR^{2.1}, COOR^{2.1}, CONR^{2.2}R^{2.3}, SR^{2.1}, SO-R^{2.1}, SO₂-R^{2.1}**,** Phenyl, einem Het, einem Hetaryl, einem monocyclischem C₃₋₇-Cycloalkyl, CH₂-NR^{2.2}R^{2.3} und NR^{2.2}R^{2.3} substituiert sein kann, welcher wiederum gegebenenfalls durch einen oder mehrere Reste ausgewählt aus der Gruppe bestehend aus OH, Halogen, OR^{2.1}, Oxo, CF₃, CHF₂, CH₂F, C₁₋₆-Alkyl, C₁₋₆-Alkanol, Phenyl, COOR^{2.1}, CH₂-NR^{2.2}R^{2.3} und NR^{2.2}R^{2.3} substituiert sein kann,
wobei R^{2.1} H ist oder ein Rest ausgewählt ist aus der Gruppe bestehend aus C₁₋₈-Alkyl, C₁₋₆-Alkanol, C₁₋₃-Haloalkyl, monocyclisches C₃₋₇ Cycloalkyl, Phenyl-C₁₋₆-alkylen, Hetaryl-C₁₋₆-alkylen,
Het-C₁₋₆-alkylen, C₃₋₇-Cycloalkyl-C₁₋₆-alkylen, Phenyl, ein Hetaryl und ein Het, der gegebenenfalls durch einen oder mehrere Reste ausgewählt aus der Gruppe bestehend aus OH, Halogen, C₁₋₆-Alkyl, O-(C₁₋₃-Alkyl) und Phenyl substituiert sein kann,
wobei R^{2.2} und R^{2.3} unabhängig voneinander H sind oder ein Rest ausgewählt sind aus der Gruppe bestehend aus C₁₋₆-Alkyl, monocyclisches C₃₋₇ Cycloalkyl, Phenyl-C₁₋₃-alkylen, Hetaryl-C₁₋₃-alkylen, Phenyl, monocyclischer C₃₋₇-Heterocyclus, Hetaryl, CO-NH₂, CO-NHCH₃, CON(CH₃)₂, SO₂-(C₁-C₂-Alkyl), CO-R^{2.1} und COOR^{2.1}, der gegebenenfalls durch einen oder mehrere Reste ausgewählt aus der Gruppe bestehend aus OH, Halogen, C₁₋₆-Alkyl, Phenyl und COOR^{2.1} substituiert sein kann,
wobei
**Het** ein drei- bis siebengliedriger, monocyclischer, gesättigter-oder teilweise gesättigten Heterocyclus ist, der 1, 2 oder 3 Heteroatome unabhängig voneinander ausgewählt aus der Gruppe bestehend aus N, S oder O enthält,
und wobei
**Hetaryl** ein fünf bis sechsgliedriger, monocyclisches, aromatisches Heteroaryl ist, das 1, 2 oder 3 Heteroatome unabhängig voneinander ausgewählt aus der Gruppe bestehend aus N, S oder O enthält,
und wobei
**Cycloalkyl** gesättigt oder teilweise gesättigt sein kann,
ode**r**
**R²** ein monocyclisches C₃₋₇ Cycloalkyl, das gegebenenfalls mit einem Rest ausgewählt aus der Gruppe bestehend aus verzweigtes oder unverzweigtes C₁₋₆-Alkanol, C₁₋₃-Fluoroalkyl, C₁₋₃-alkylen-OR^{2.1}, OR^{2.1}, COOR^{2.1}, SO₂-NR^{2.2}R^{2.3}, Het, Phenyl, C₁₋₆-Alkyl, Phenyl-C₁₋₆-alkylen, Hetaryl-C₁₋₆-alkylen, monocyclisches C₃₋₇ Cycloalkyl und NR^{2.2}R^{2.3} substituiert sein kann, der gegebenenfalls durch einen oder mehrere Reste ausgewählt aus der Gruppe bestehend aus OH, OR^{2.1}, Oxo, Halogen, CF₃, CHF₂, CH₂F, C₁₋₆-Alkyl, Phenyl und NR^{2.2}R^{2.3} substituiert sein kann,
oder
**R²** ein Phenyl, das gegebenenfalls durch OH, SH oder Halogen oder durch einen oder mehrere Reste ausgewählt aus der Gruppe bestehend aus OR^{2.1}, COOR^{2.1}, NR^{2.2}R^{2.3}, CH₂-NR^{2.2}R^{2.3}, C₃₋₇-Cycloalkyl, Het, C₁₋₆-Alkyl, CF₃, CHF₂, CH₂F, Phenyl-C₁₋₆-alkylen, Het-C₁₋₆-alkylen, Hetaryl-C₁₋₆-alkylen, Phenyl, SO₂-CH₃, SO₂-CH₂CH₃ und SO₂-NR^{2.2}R^{2.3} substituiert sein kann, der wiederum gegebenenfalls durch einen oder mehrere oder mehrere Reste ausgewählt aus der Gruppe bestehend aus OH, OR^{2.1}, Oxo, Halogen, CF₃, CHF₂, CH₂F, C₁₋₆-Alkyl, Phenyl und NR^{2.2}R^{2.3} substituiert sein kann,
oder
**R²** ein Rest ausgewählt aus einer Gruppe bestehend aus einem Het und einem Hetaryl, welcher gegebenenfalls durch einen oder mehrere Reste ausgewählt aus der Gruppe Halogen, OH, Oxo, CF₃, CHF₂ und CH₂F oder durch einen oder mehrere Reste ausgewählt aus der Gruppe OR^{2.1}, C₁₋₃-alkylen-OR^{2.1}, SR^{2.1}, SO-R^{2.1}, SO₂-R^{2.1}, COOR^{2.1}, COR^{2.1}, C₁₋₆-Alkanol, C₃₋₁₀-Cycloalkyl, Phenyl, C₁₋₆-Alkyl, Phenyl-C₁₋₆-alkylen, Hetaryl-C₁₋₆-alkylen, Het, Hetaryl, C₁₋₆-Alkanol, C₁₋₃-alkylen-OR^{2.1} und NR^{2.2}R^{2.3} substituiert sein kann, der gegebenenfalls wiederum durch einen oder mehrere Reste ausgewählt aus der Gruppe bestehend aus OH, OR^{2.1}, Oxo, Halogen, CF₃, CHF₂, CH₂F, C₁₋₆-Alkyl, Phenyl und NR^{2.2}R^{2.3} substituiert sein kann,
und worin
**R³** ein Rest ausgewählt aus der Gruppe aus einem mono- oder bicyclischen C₆₋₁₀-Aryl, einem Het und einem Hetaryl, der gegebenenfalls mit einem oder mehreren Resten ausgewählt aus der Gruppe Halogen, C₁₋₃-Alkyl, Cyclopropyl, C₁₋₃-Fluoroalkyl, C₁₋₃-Alkanol, C₁₋₃-alkylen-OR^{2.1}, OH und O-C₁₋₃-Alkyl substituiert sein kann
und worin
a) R³, R⁴, R^{4'} und R⁵ H sind und
**R⁶ und R⁷** gemeinsam eine Brücke ausgewählt aus der Gruppe bestehend aus Methylen, Ethylen und Propylen bilden,
oder wobei
b) R³, R⁴, R^{4'} und R⁷ H sind und
**R⁵ und R⁶** gemeinsam eine Brücke ausgewählt aus der Gruppe bestehend aus Methylen, Ethylen und Propylen bilden,
oder wobei
d) R⁵, R⁶ und R⁷ H sind und
**R^{3,} R⁴ und R**^{**4**'} gemeinsam mit dem C- und dem N-Atom des Piperazins einen ungesättigten oder teilweise ungesättigten, 5- oder 6-gliedrige Heterocyclus enthaltend zwei oder drei, mindestens jedoch zwei Stickstoffatome bilden, wobei dieser Heterocyclus gegebenenfalls mit einem, zwei oder drei Resten ausgewählt aus der Gruppe bestehend aus Oxo, OH, Halogen, C₁₋₆-Alkyl, Cyclopropyl, C₁₋₃-Fluoroalkyl, C₁₋₃-alkylen-OR^{2.1}, C₁₋₆-Alkanol, COOR^{2.1}, C₆₋₁₀-Aryl, C₁₋₃-Alkylen-C₆₋₁₀-Aryl, Hetaryl, C₁₋₃-alkylen-Hetaryl, Het, CO-Het, C₁₋₃-alkylen-Het -CO-N(CH₃)-Het, -CO-NH-Het, -CO-O-N(CH₃)-Het, -CO-O-NH-Het, -CO-N(CH₃)C₃₋₇-Cycloalkyl, -CO-NH-C₃₋₇-Cycloalkyl, -CO-O-N(CH₃)-C₃₋₇-Cycloalkyl, -CO-O-NH-C₃₋₇-Cycloalkyl, -CO-NH-C₁₋₂-Alkylen-Hetaryl, -CO-N(CH₃)-C₁₋₂-Alkylen-Hetaryl, -CO-NH-CH(CH₃)-Hetaryl, -CO-NH-C(CH₃)₂-Hetaryl, -CO-N(Cyclopropyl)-Het und -CO-N(Cyclopropyl)-Hetaryl substituiert sein kann, welcher wiederum gegebenenfalls mit einem oder mehreren Resten ausgewählt aus der Gruppe OH, Oxo, Halogen, C₁₋₆-Alkyl, C₁₋₃-Fluoroalkyl und C₁₋₆-Alkanol substituiert sein kann,
sowie pharmakologisch verträgliche Salze davon.

4. Verbindungen der Formel **1** nach einem der Ansprüche 1 bis 3, wobei
X SO,
**R²** H ist oder C₁₋₆-Alkyl ist, welches gegebenenfalls durch F, CF₃, CHF₂ oder CH₂F, substituiert sein kann oder welches gegebenenfalls durch einen oder mehrere Reste ausgewählt aus der Gruppe bestehend aus OR^{2.1} COOR^{2.1}, CONR^{2.2}R^{2.3}, SR^{2.1}, SO-R^{2.1}, SO₂-R^{2.1}, Phenyl, einem Het, einem Hetaryl, einem monocyclischem C₃₋₇-Cycloalkyl, CH₂-NR^{2.2}R^{2.3} und NR^{2.2}R^{2.3} substituiert sein kann, welcher wiederum gegebenenfalls durch einen oder mehrere Reste ausgewählt aus der Gruppe bestehend aus OH, Halogen, CF₃, CHF₂, CH₂F, OR^{2.1}, Oxo, C₁₋₆-Alkyl, C₁₋₆-Alkanol, Phenyl, COOR^{2.1}, CH₂-NR^{2.2}R^{2.3} und NR^{2.2}R^{2.3} substituiert sein kann,
wobei **R^{2.1}** H ist oder ein Rest ausgewählt ist aus der Gruppe bestehend aus C₁₋₆-Alkyl, C₁₋₆-Alkanol, monocyclisches C₃₋₇ Cycloalkyl, Phenyl-CH₂-, Hetaryl-methylen,
Het-methylen, C₃₋₇-Cycloalkyl-methylen, Phenyl, ein Hetaryl und ein Het, der gegebenenfalls durch einen oder mehrere Reste ausgewählt aus der Gruppe bestehend aus OH, F, CI, Br, I, Methyl, Ethyl, Propyl, Isopropyl, O-Methyl, O-Ethyl, O-Propyl, O-Isopropyl und Phenyl substituiert sein kann,
wobei **R^{2.2}** und **R^{2.3}** unabhängig voneinander H sind oder ein Rest ausgewählt sind aus der Gruppe bestehend aus Methyl, Ethyl, Propyl, Isopropyl, monocyclisches C₃₋₇ Cycloalkyl, Phenyl-methylen, Hetaryl-methyten, Phenyl, Het, Hetaryl, CO-NH₂, CO-NHCH₃, CON(CH₃)₂, SO₂-(C₁-C₂-Alkyl), CO-R^{2.1} und COOR^{2.1}, der gegebenenfalls durch einen oder mehrere Reste ausgewählt aus der Gruppe bestehend aus OH, F, Cl, Br, Methyl, Ethyl, Phenyl und COOR^{2.1} substituiert sein kann,
und wobei **R¹, R³, R⁴, R^{4'}, R⁵, R⁶ und R⁷** wie in einem der Ansprüche 1 bis 3 definiert sind,
sowie pharmakologisch verträgliche Salze davon.

5. Verbindungen der Formel **1** nach einem der Ansprüche 1 bis 3, wobei
**R²** ein Rest nach Formel **2** 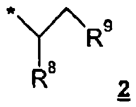 ist,
worin **R⁹** OH oder NH₂ ist und
worin **R⁸** ein Rest ausgewählt aus der Gruppe bestehend aus C₁₋₆-Alkyl, Hetaryl und Phenyl, der gegebenenfalls mit einem oder mehreren Resten ausgewählt aus der Gruppe bestehend aus OH, F, Br, OR^{2.1}, Oxo, Methyl, Ethyl, C₁₋₂-Alkanol, Phenyl, COOR^{2.1}, CH₂-NR^{2.2}R^{2.3} und NR^{2.2}R^{2.3} substituiert sein kann,
sowie pharmakologisch verträgliche Salze davon.

6. Verbindungen der Formel **1** nach Anspruch 5, wobei
**R²** ein Rest nach Formel **2** 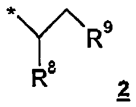 ist,
worin **R⁹** OH oder NH₂ ist und
worin **R⁸** Methyl, Ethyl, Propyl, Isopropyl ist
sowie pharmakologisch verträgliche Salze davon.

7. Verbindungen der Formel **1** nach einem der Ansprüche 1 bis 3, wobei
**R²** ein monocyclischer drei-, vier-, fünf-, sechs oder siebengliedriger Cycloalkyl-Ring ist; der gegebenenfalls in spiro-Stellung mit einem Rest ausgewählt aus der Gruppe bestehend aus -CH₂-OR^{2.1}, verzweigtes oder unverzweigtes C₂₋₆-Alkylen-OR^{2.1}, Methyl, Ethyl, Propyl, isopropyl, Butyl, Isobutyl, Cyclopropyl, und -CF₃, CHF₂, CH₂F und C₂₋₄-Fluoroalkyl substituiert sein kann, wobei
**R^{2.1}** ausgewählt ist aus der Gruppe bestehend aus Methyl, Ethyl, Propyl, Isopropyl, Butyl, Isobutyl,
sowie pharmakologisch verträgliche Salze davon.

8. Verbindungen der Formel **1** nach einem der Ansprüche 1 bis 3, wobei
**R²** ein Phenyl, das gegebenenfalls in einer oder in beiden meta-Stellungen durch einen oder mehrere Reste ausgewählt aus der Gruppe bestehend aus Methyl, Ethyl, Propyl, Isopropyl, Cyclopropyl, F, Cl, Br, OH, OR^{2.1}, COOR^{2.1}, CF₃, CHF₂, CH₂F, NH₂, NH(CH₃) und N(CH₃)₂ substituiert ist, wobei R^{2.1} H, Methyl oder Ethyl sein kann,
sowie pharmakologisch verträgliche Salze davon.

9. Verbindungen der Formel **1** nach einem der Ansprüche 1 bis 3, wobei
**R²** ein Rest ausgewählt aus einer Gruppe bestehend aus monocyclischen, gesättigten drei-, vier-, fünf-, sechs- oder siebengliedrigem Heterocyclus mit 1, 2 oder 3 Heteroatomen jeweils ausgewählt aus der Gruppe bestehend aus N, O und S, der gegebenenfalls durch einen oder mehrere Reste ausgewählt aus der Gruppe Fluor, OH, CF₃, CHF₂, CH₂F und Oxo oder durch einen oder mehrere Reste ausgewählt aus der Gruppe OR^{2.1}, C₁₋₃-alkylen-OR^{2.1}, SR^{2.1}, SO-R^{2.1}, SO₂-R^{2.1}, COOR^{2.1}, COR^{2.1}, C₁₋₆-Alkanol, C₃₋₁₀-Cycloalkyl, Phenyl, C₁₋₆-Alkyl, Phenyl-C₁₋₆-alkylen, Hetaryl-C₁₋₆-alkylen, Het, Hetaryl und NR^{2.2} R^{2.3} substituiert sein kann, der gegebenenfalls wiederum durch einen oder mehrere Reste ausgewählt aus der Gruppe bestehend aus OH, OR^{2.1}, Oxo, F, CI, CF₃, CHF₂, CH₂F, C₁₋₆-Alkyl, Phenyl und NR^{2.2}R^{2.3} substituiert sein kann,
wobei **R^{2.1}, R^{2.2} und R^{2.3}** wie in Anspruch 1 bis 3 definiert sind,
sowie pharmakologisch verträgliche Salze davon.

10. Verbindungen der Formel **1** nach Anspruch 9, wobei
**R²** ein Rest ausgewählt aus-einer Gruppe bestehend aus einem monocyclischen, gesättigten sechsgliedrigem Heterocyclus mit mindestens einem Heteroatom jeweils ausgewählt aus der Gruppe bestehend aus N, O und S ist, der gegebenenfalls durch einen oder mehrere Reste ausgewählt aus der Gruppe F, CI, Br, CF₃, CHF₂, CH₂F, OH, Oxo, NH₂, NHCH₃, N(CH₃)₂, Methyl, Ethyl, Propyl, Isopropyl, Cyclopropyl, Methoxy und Ethoxy substituiert sein kann,
sowie pharmakologisch verträgliche Salze davon.

11. Verbindungen der Formel **1** nach Anspruch 10, wobei
**R²** ein Rest ausgewählt aus einer Gruppe bestehend aus Piperidin oder Tetrahydropyran, der gegebenenfalls durch einen oder mehrere Reste ausgewählt aus der Gruppe F, CI, Br, OH, CF₃, CHF₂, CH₂F, NH₂, NHCH₃, N(CH₃)₂, Oxo, Methyl und Methoxy substituiert sein kann,
sowie pharmakologisch verträgliche Salze davon.

12. Verbindungen der Formel **1** nach einem der Ansprüche 1 bis 3, wobei
**R³** ein Rest ausgewählt aus der Gruppe aus einem Phenyl, der gegebenenfalls mit einem oder mehreren Resten ausgewählt aus der Gruppe F, Cl, Br, Methyl, Ethyl, Propyl, Isopropyl, Cyclopropyl, C₁₋₃-Fluoroalkyl, C₁₋₃-Alkanol, OH und O-C₁₋₃-Alkyl substituiert sein kann,
sowie pharmakologisch verträgliche Salze davon.

13. Verbindungen der Formel **1** nach einem der Ansprüche 1 bis 3, wobei
**R³** ein Rest ausgewählt aus der Gruppe bestehend aus einem mono- oder bicyclischen, gesättigten, ungesättigten oder teilweise gesättigten drei- bis zehngliedrigem Heterocyclus mit 1 bis 3 Heteroatomen ausgewählt aus N, O und S, der gegebenenfalls mit einem oder mehreren Resten ausgewählt aus der Gruppe F, CI, Br, Methyl, Ethyl, Propyl, Isopropyl, Cyclopropyl, C₁₋₃-Fluoroalkyl, C₁₋₃-Alkanol, OH und O-C₁₋₃-Alkyl substituiert sein kann,
sowie pharmakologisch verträgliche Salze davon.

14. Verbindungen der Formel **1** nach einem der Ansprüche 1 bis 3, wobei
**R³ und R⁴** gemeinsam mit dem C- und dem N-Atom des Piperazins einen gesättigten oder teilweise ungesättigten 5- oder 6-gliedrigen Heterocyclus enthaltend zwei oder drei, mindestens jedoch zwei Stickstoffatome bilden
oder wobei
**R³, R⁴ und R^{4'}** gemeinsam mit dem C- und dem N-Atom des Piperazins einen ungesättigten oder teilweise ungesättigten, 5- oder 6-gliedrigen Heterocyclus enthaltend zwei oder drei, mindestens jedoch zwei Stickstoffatome bilden,
wobei dieser Heterocyclus gegebenenfalls mit einem, zwei oder drei Resten ausgewählt aus der Gruppe bestehend aus Oxo, OH, F, CI, Br, C₁₋₆-Alkyl, Cyclopropyl, C₁₋₃-Fluoroalkyl, C₁₋₆-Alkanol, COOR^{2.1}, Phenyl, C₁₋₃-Alkylen-Phenyl, Hetaryl, C₁₋₃-Alkylen-Hetaryl, Het, CO-Het, C₁₋₃-Alkylen-Het, -CO-N(CH₃)-Het, -CO-NH-Het, -CO-O-N(CH₃)-Het, -CO-O-NH-Het, -CO-N(CH₃)-C₃₋₇-Cycloalkyl, -CO-NH-C₃₋₇-Cycloalkyl, -CO-O-N(CH₃)-C₃₋₇-Cycloalkyl, -CO-O-NH-C₃₋₇-Cycloalkyl, -CO-NH-C₁₋₂-Alkylen-Hetaryl, -CO-N(CH₃)-C₁₋₂-Alkylen-Hetaryl, -CO-NH-CH(CH₃)-Hetaryl, -CO-NH-C(CH₃)₂-Hetaryl, -CO-N(Cyclopropyl)-Het und -CO-N(Cyclopropyl)-Hetaryl substituiert sein kann, welcher wiederum gegebenenfalls mit einem oder mehreren Resten ausgewählt aus der Gruppe OH, Oxo, F, CI, Br, C₁₋₆-Alkyl, C₁₋₃-Fluoroalkyl, C₁₋₆-Alkanol substituiert sein kann,
sowie pharmakologisch verträgliche Salze davon.

15. Verbindungen der Formel **1** nach Anspruch 14, wobei
**R³, R⁴ und R^{4'}** gemeinsam mit dem C- und dem N-Atom des Piperazins einen fünfgliedrigen Heterocyclus ausgewählt aus der Gruppe bestehend aus Imidazol, Pyrazol und Triazol bilden, der gegebenenfalls mit einem, zwei oder drei Resten ausgewählt aus der Gruppe bestehend aus Oxo, OH, F, CI, Br, C₁₋₃-Fluoroalkyl, Methyl, Ethyl, Propyl, Isopropyl, Cyclopropyl, COOR^{2.1}, Phenyl, C₁₋₃-Alkylen-Phenyl, Hetaryl, C₁₋₃-Alkylen-Hetaryl, Het, CO-Het, C₁₋₃-Alkylen-Het, -CO-N(CH₃)-Het. -CO-NH-Het, -CO-O-N(CH₃)-Het, -CO-O-NH-Het, -CO-N(CH₃)-C₃₋₇-Cycloalkyl,
-CO-NH-C₃₋₇-Cycloalkyl, -CO-O-N(CH₃)-C₃₋₇-Cycloalkyl, -CO-O-NH-C₃₋₇-Cycloalkyl, -CO-NH-C₁₋₂-Alkylen-Hetaryl, -CO-N(CH-₃)-C₁₋₂-Alkylen-Hetaryl, -CO-NH-CH(CH₃)-Hetaryl, -CO-NH-C(CH₃)₂-Hetaryl, -CO-N(Cyclopropyl)-Het und -CO-N(Cyclopropyl)-Hetaryl substituiert sein kann, welcher wiederum gegebenenfalls mit einem oder mehreren Resten ausgewählt aus der Gruppe OH, Oxo, F, Cl, Br, C₁₋₆-Alkyl, C₁₋₃-Fluoroalkyl und C₁₋₆-Alkanol substituiert sein kann, wobei R^{2.1} H, Methyl, Ethyl, Propyl oder Isopropyl sein kann,
sowie pharmakologisch verträgliche Salze davon.

16. Verbindungen der Formel 1 nach einem der Ansprüche 1 bis 13, wobei
a) R³, R⁴, R^{4'} und R⁵ H sind und
**R⁶ und R⁷** gemeinsam eine Brücke ausgewählt aus der Gruppe bestehend aus Methylen und Ethylen bilden,
oder wobei
b) **R³, R⁴, R^{4'}** und **R⁷** H sind und
**R⁵ und R⁶** gemeinsam eine Brücke ausgewählt aus der Gruppe bestehend aus Methylen und Ethylen bilden,
sowie pharmakologisch verträgliche Salze davon.

17. Verbindungen der Formel **1** nach einem der Ansprüche 1 bis 4, wobei die Verbindung ausgewählt ist aus der Gruppe bestehend aus 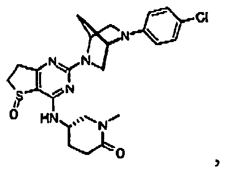 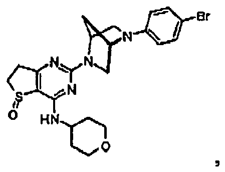 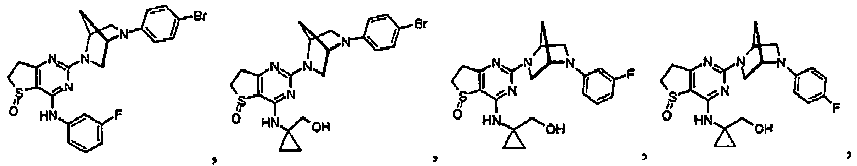 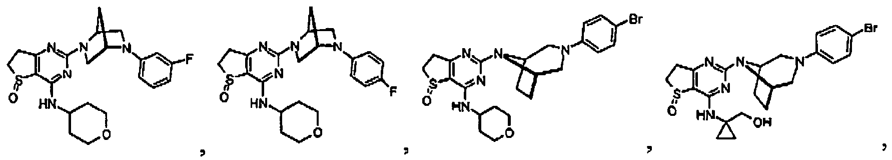 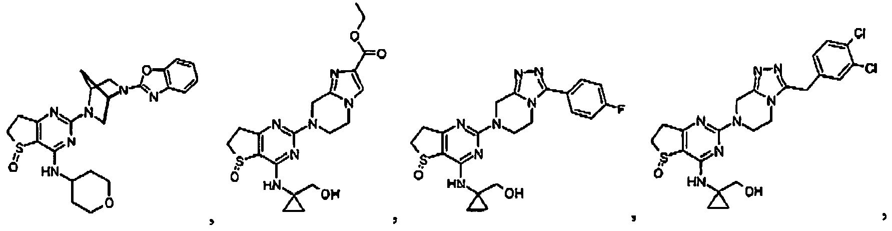 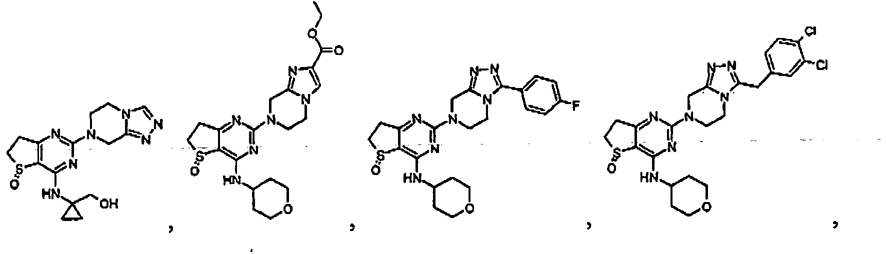 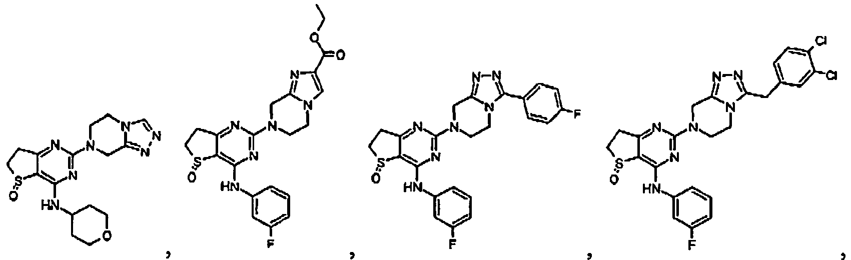 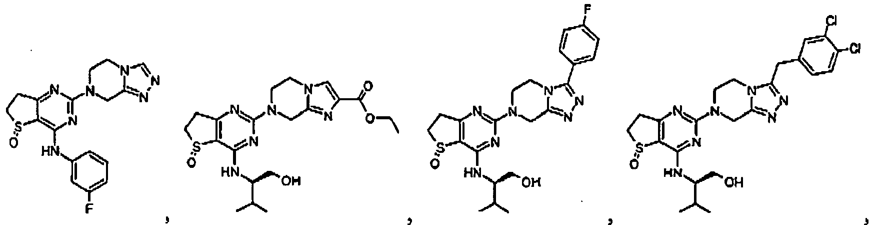 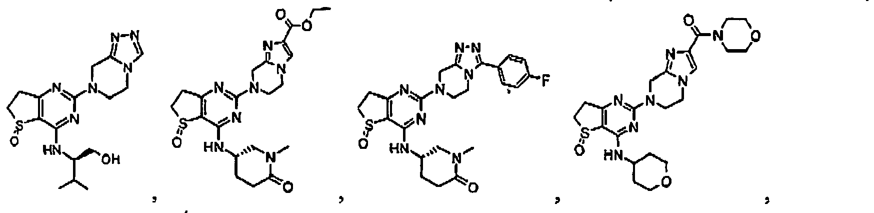 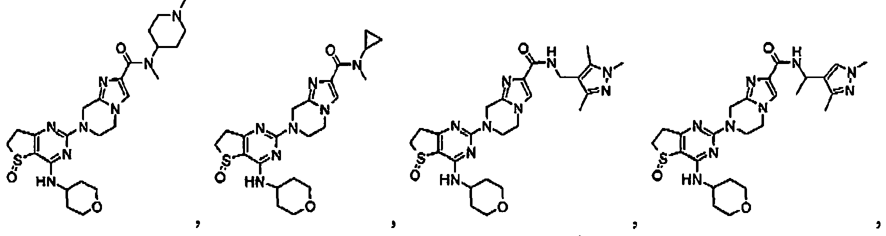 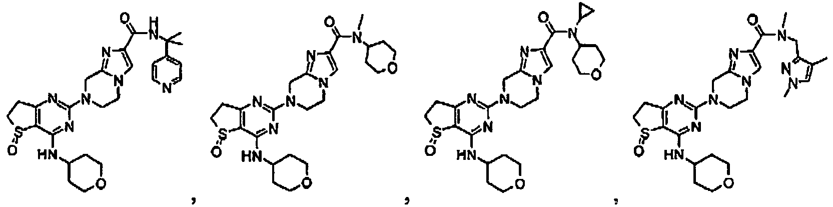 und 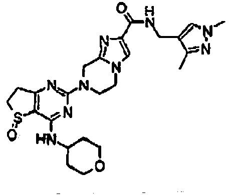 sowie pharmakologisch verträgliche Salze davon.

18. Verbindungen nach einem der Ansprüche 1 bis 17 als Arzneimittel.

19. Verwendung von Verbindungen nach einem der Ansprüche 1 bis 17 zur Herstellung eines Medikaments zur Behandlung von Atemwegs- oder gastrointestinalen Beschwerden oder Erkrankungen, wie auch entzündliche Erkrankungen der Gelenke, der Haut oder der Augen, Krebserkrankungen, sowie von Erkrankungen des periphären oder zentralen Nervensystems.

20. Verwendung von Verbindungen nach einem der Ansprüche 1 bis 17 zur Herstellung eines Medikaments zur Vorbeugung und Behandlung von Atemwegs- oder Lungenerkrankungen, die mit einer erhöhten Schleimproduktion, Entzündungen und / oder obstruktiven Erkrankungen der Atemwege einher gehen.

21. Verwendung von Verbindungen nach einem der Ansprüche 1 bis 17 zur Herstellung eines Medikament zur Behandlung von entzündlichen und obstruktiven Erkrankungen wie COPD, chronischer Sinusitis, Asthma, Morbus Crohn, Colitis ulcerosa.

22. Verwendung von Verbindungen nach einem der Ansprüche 1 bis 17 zur Herstellung eines Medikaments zur Vorbeugung und Behandlung von Erkrankungen des periphären oder zentralen Nervensystems wie Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Hirn-Trauma.

23. Pharmazeutische Formulierungen **gekennzeichnet durch** den Gehalt an einer oder mehreren Verbindungen der Formel **1** nach einem der Ansprüche 1 bis 17 in Kombination mit einem oder mehreren Wirkstoffen ausgewählt aus der Gruppe bestehend aus Betamimetika, Corticosteroiden, weiteren PDE4-Inhibitoren, EGFR-Hemmern und LTD4-Antagonisten, CCR3-Inhibitoren, iNOS-Inhibitoren und SYK-Inhibitoren.

## Claims

1. Compounds of formula **1** 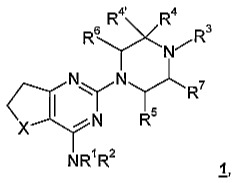 wherein
**X** is SO or SO₂,
**R¹** is H, C₁₋₆-alkyl,
**R²** is H or a group selected from among C₁₋₁₀-alkyl and C₂₋₆-alkenyl, which may optionally be substituted by one or more groups selected from halogen and C₁₋₃-fluoroalkyl or which may optionally be substituted by one or more groups selected from among OR^{2.1}, COOR^{2.1}, CONR^{2.2}R^{2.3}, SR^{2.1}, SO-R^{2.1}, SO₂-R^{2.1}, C₆₋₁₀-aryl, a het, a hetaryl, a mono- or bicyclic C₃₋₁₀-cycloalkyl, CH₂-NR²²R^{2.3} and NR^{2.2}R^{2.3}, which in turn may optionally be substituted by one or more groups selected from among OH, halogen, OR^{2.1}, oxo, CF₃, CHF₂, CH₂F, C₁₋₆-alkyl, C₁₋₆-alkanol, C₆₋₁₀-aryl, COOR^{2.1}, CH₂-NR^{2.2}R^{2.3} and NR^{2.2}R^{2.3},
wherein R^{2.1} is H or a group selected from among C₁₋₆-alkyl, C₁₋₆-alkanol, C₁₋₃-fluoroalkyl, mono- or bicyclic C₃₋₁₀ cycloalkyl, C₆₋₁₀-aryl-C₁₋₆-alkylene, hetaryl-C₁₋₆-alkylene, het-C₁₋₆-alkylene, C₃₋₁₀-cycloalkyl-C₁₋₆-alkylene, a mono- or bicyclic C₆₋₁₀-aryl, a hetaryl and a het, which may optionally be substituted by one or more groups selected from among OH, O-(C₁₋₃-alkyl), halogen, C₁₋₆-alkyl and C₆₋₁₀-aryl,
wherein **R^{2.2}** and **R^{2.3}** independently of one another denote H or a group selected from among C₁₋₆-alkyl, mono- or bicyclic C₃₋₁₀-cycloalkyl, C₆₋₁₀-aryl-C₁₋₆-alkylene, hetaryl-C₁₋₆-alkylene, mono- or bicyclic C₆₋₁₀-aryl, a het a hetaryl, CO-NH₂, CO-NH(CH₃). CO-N(CH₃)₂, SO₂-(C₁₋₂-alkyl), CO-R^{2.1} and COOR^{2.1},
which may optionally be substituted by one or more groups selected from among OH, halogen, C₁₋₆-alkyl, C₆₋₁₀-aryl and COOR^{2.1},
wherein
**het** is a three- to eleven-membered, mono- or bicyclic, saturated or partially saturated, optionally annelated or optionally bridged heterocyclic group, which contains 1, 2, 3 or 4 heteroatoms independently selected from among N, S or O,
and wherein
**hetaryl** is a five- to ten-membered, mono- or bicyclic, optionally annelated heteroaryl, which contains 1, 2, 3 or 4 heteroatoms independently selected from among N, S or O,
and wherein
**cycloalkyl** may be saturated or partially saturated,
or
**R²** denotes a mono- or polycyclic C₃₋₁₀ cycloalkyl, which may optionally be mono- or poly-bridged via C₁₋₃-alkyl groups and which may optionally be substituted by a group selected from among branched or unbranched C₁₋₆-alkanol, C₁₋₃-fluoroalkyl, C₁₋₃-alkylene-OR^{2.1}, OR^{2.1}, COOR^{2.1}, SO₂-NR^{2.2}R^{2.3}, het, C₆₋₁₀-aryl, C₁₋₆-alkyl, C₆₋₁₀-aryl-C₁₋₆-alkylene, hetaryl-C₁₋₆-alkylene, mono- or bicyclic C₃₋₁₀ cycloalkyl and NR^{2.2}R^{2.3}, which may optionally be substituted by one or more groups selected from among OH, OR^{2.1}, oxo, halogen, CF₃, CHF₂, CH₂F, C₁₋₆-alkyl, C₆₋₁₀-aryl and NR^{2.2}R^{2.3},
or
**R²** denotes a mono- or polycyclic C₆₋₁₀-aryl, which may optionally be substituted by OH, SH or halogen or by one or more groups selected from among OR^{2.1}, COOR^{2.1}, NR^{2.2}R^{2.3}, CH₂-NR^{2.2}R^{2.3}, C₃₋₁₀-cycloalkyl, het, C₁₋₆-alkyl, C₁₋₃-fluoroalkyl, C₆₋₁₀-aryl-C₁₋₆-alkylene, het-C₁₋₆-alkylene, hetaryl-C₁₋₆-alkylene, C₆₋₁₀-aryl, SO₂-CH₃, SO₂-CH₂CH₃ and SO₂-NR^{2.2}R^{2.3}, which in turn may optionally be substituted by one or more groups selected from among OH, OR^{2.1}, oxo, halogen, CF₃, CHF₂, CH₂F, C₁₋₆-alkyl, C₆₋₁₀-aryl and NR^{2.2}R^{2.3},
or
**R²** denotes a group selected from among a het and a hetaryl, which in turn may optionally be substituted by one or more groups selected from among halogen, OH, oxo, CF₃, CHF₂ and CH₂F or by one or more groups selected from among OR^{2.1}, C₁₋₃-alkylene-OR^{2.1}, SR^{2.1}, SO-R^{2.1} and SO₂-R^{2.1} COOR^{2.1}, COR^{2,1}, C₁₋₆-alkanol, C₃₋₁₀-cycloalkyl, C₆₋₁₀-aryl, C₁₋₆-alkyl, C₆₋₁₀-aryl-C₁₋₈-alkylene, hetaryl-C₁₋₆-alkylene, het, hetaryl, C₁₋₃-alkylene-OR^{2.1} and NR^{2.2}R^{2.3}, which in turn may optionally be substituted by one or more groups selected from among OH, OR^{2.1}, oxo, halogen, CF₃, CHF₂, CH₂F, C₁₋₆-alkyl, C₆₋₁₀-aryl and NR^{2.2}R^{2.3},
or wherein
**NR¹R²** together denotes a heterocyclic four- to seven-membered ring, which may optionally be bridged, which contains 1, 2 or 3 heteroatoms selected from among N, O and S and which may optionally be substituted by one or more groups selected from among OH, OR^{2.1}, C₁₋₃-alkylene-O^{R.1}, oxo, halogen, C₁₋₆-alkyl, C₆₋₁₀-aryl, COOR^{2.1}, CH₂-NR^{2.2}-COO-R^{2.1} CH₂-NR^{2.2}-CO-R^{2.1}, CH₂-NR^{2.2}-CO-CH₂-NR^{2.2}R^{2.3}, CH₂-NR^{2.2}-SO₂-C₁₋₃-alkyl, CH₂-NR^{2.2}-SO₂-NR^{2.2}R^{2.3}, CH₂-NR^{2.3}-CO-NR^{2.2}R^{2.3}, CO-NR^{2.2}R^{3.3}, CH₂-NR^{2.2}R^{3.3} and NR^{2.2}R^{2.1},
and wherein
**R³** is a group selected from among a mono- or bicyclic C₆₋₁₀-aryl, a het and a hetaryl, which may optionally be substituted by one or more groups selected from among halogen, -C₁₋₃-alkyl, cyclopropyl, -C₁₋₃-fluoroalkyl, -C₁₋₃-alkylene-OR^{2.1}, -OH and -O-C₁₋₃-alkyl,
and wherein
a) **R³, R⁴, R^{4'}** and **R⁵** denote H and
**R⁶** and **R⁷** together form a bridge selected from among methylene, ethylene and propylene which may optionally be substituted by a group selected from among -CH₃, -OH, -F, -CF₃, -CHF₂, -CH₂F, -NH₂, -NH(C₁₋₃-alkyl), -N(C₁₋₃-alkyl), and O-(C₁₋₃-alkyl),
or wherein
b) **R³, R**⁴ ,**R**^{4'} and **R**⁷ denote H and
**R⁵ and R⁶** together form a bridge selected from among methylene, ethylene and propylene, which may optionally be substituted by a group selected from among CH₂, -OH, -F, -CF₃, -CHF₂, -CH₂F, -NH₂, -NH(C₁₋₃-alkyl), -N(C₁₋₃-alkyl), and OCH₃,
or wherein
c) **R⁵, R⁵, R^{4'} and R⁷** denote H and
**R³ and R⁴** together with the C and the N atom of the piperazine form a saturated or partially unsaturated 5- or 6-membered heterocyclic group containing two or three, but at least two nitrogen atoms
or wherein
d) **R**⁵, **R**⁶ and **R**⁷ denote H and
**R**³, **R**⁴ **and R**^{**4**'} together with the C and the N atom of the piperazine form an unsaturated or partially unsaturated, 5- or 6-membered heterocyclic group containing two or three, but at least two nitrogen atoms,
wherein each of these saturated, unsaturated or partially unsaturated, 5- or 6-membered heterocycles may optionally be substituted by one, two or three groups selected from among oxo, OH, halogen, C₁₋₆-alkyl, cyclopropyl, C₁₋₃-fluoroalkyl, C₁₋₃-alkylene-OR^{2.1}, C₁₋₆-alkanol, COOR^{2.1}, C₆₋₁₀-aryl, C₁₋₃-alkylene-C₆₋₁₀-aryl, hetaryl, C₁₋₃-alkylene-hetaryl, het, CO-het, C₁₋₃-alkylene-het, -CO-N(C₁₋₃-alkyl)-het, -CO-NH-het, -CO-O-N(C₁₋₃-alkyl)-het, -CO-O-NH-het, -CO-N(C₁₋₉-alkyl)-C₃₋₇-cycloalkyl. -CO-NH-C₃₋₇-cycloalkyl, -CO-O-N(C₁₋₃-alkyl)-C₃₋₇-cycloalkyl, -CO-O-NH-C₃₋₇-cycloalkyl, -CO-NH-(C₁₋₂-alkylene)-hetaryl, -CO-N(C₁₋₃-alkyl)-(C₁₋₂-alkylene)-hetaryl, -CO-NH-CH(C₁₋₃-alkyl)-hetaryl, -CO-NH-C(C₁₋₃-alkyl)₂-hetaryl, -CO-N(cyclopropyl)-het and -CO-N(cyclopropyl)-hetaryl,
which in turn may optionally be substituted by one or more groups selected from among OH, oxo, halogen, C₁₋₆-alkyl, C₁₋₃-fluoroalkyl, C₁₋₆-alkanol,
**and** the pharmacologically acceptable salts thereof.

2. Compounds of formula **1** according to claim 1, wherein
**X** is SO or SO₂,
**R¹** is H
**R²** is H or C₁₋₁₀-alkyl, which may optionally be substituted by one or more groups selected from halogen and C₁₋₃-fluoroalkyl or which may optionally be substituted by one or more groups selected from among OR^{2.1}, COOR^{2.1},CONR^{2.2} R^{2.3}, SR^{2.1},SO-R^{2.1}, SO₂-R^{2.1}, C₆₋₁₀-aryl, a het, a hetaryl, a mono- or bicyclic C₃₋₁₀-cycloalkyl, CH₂-NR^{2.2}R^{2.3} and NR^{2.2}R^{2.3}, which in turn may optionally be substituted by one or more groups selected from among OH, halogen, OR^{2.1}, oxo, CF₃, CHF₂, CH₂F, C₁₋₆-alkyl, C₁₋₆-alkanol, C₆₋₁₀-aryl, COOR^{2.1}, CH₂-NR^{2.2}R^{2.3} and NR^{2.2}R^{2.3},
wherein
**het** is a three- to seven-membered, monocyclic, saturated or partially saturated heterocyclic group or a seven- to eleven-membered, bicyclic, saturated or partially saturated heterocyclic group, which contains 1, 2, 3 or 4 heteroatoms selected independently of one another from among N, S or O, and wherein
**hetaryl** is a five- to six-membered, monocyclic, aromatic heteroaryl or a seven- to eleven-membered, bicyclic, aromatic heteroaryl, which contains in each case 1, 2, 3 or 4 heteroatoms selected independently of one another from among N, S or O,
and wherein
**cycloalkyl** may be saturated or partially saturated,
wherein **R^{2.1}, R^{2.2}** and **R^{2.3}** are defined as in claim 1
or
**R²** denotes a mono- or polycyclic C₃₋₁₀ cycloalkyl, which may optionally be mono- or poly-bridged via C₁₋₃-alkyl groups and which may optionally be substituted by a group selected from among branched or unbranched C₁₋₆-alkanol, C₁₋₃-alkylene. OR^{2.1}, OR^{2.1}, C₁₋₃-fluoroalkyl, COOR^{2.1}, SO₂-NR^{2.2}R^{2.3}, het, C₆₋₁₀-aryl, C₁₋₆-alkyl, C₆₋₁₀-aryl-C₁₋₆-alkylene, hetaryl-C₁-₆-alkylene, mono-or bicyclic C₃₋₁₀ cycloalkyl and NR^{2.2}R^{2.3},
which may optionally be substituted by one or more groups selected from among OH, OR^{2.1}, oxo, halogen, CF₃, CHF₂, CH₂F, C₁₋₆-alkyl, C₆₋₁₀-aryl and NR^{2.2}R^{2.3},
or
**R²** denotes a mono- or polycyclic C₁₋₁₀-aryl, which may optionally be substituted by OH, SH or halogen or by one or more groups selected from among OR^{2.1}, COOR^{2.1}, NR^{2.2}R^{2.3}, CH₂-NR^{2.2}R^{2.3},C₃₋₁₀-cycloalkyl, het, C₁₋₆-alkyl, C₁₋₃-fluoroalkyl, C₆₋₁₀-aryl-C₁₋₆-alkylene, het-C₁₋₆-alkylene, hetaryl-C₁₋₆-alkylene, C₆₋₁₀-aryl, SO₂-CH₃, SO₂-CH₂CH₃ and SO₂-NR^{2.2}R^{2.1}_{.}
which in turn may optionally be substituted by one or more groups selected from among OH, OR^{2.1}, oxo, halogen, CF₃, CHF₂, CH₂F, C₁₋₆-alkyl, C₆₋₁₀-aryl and NR^{2.2}R^{2.3}, or
**R²** denotes a group selected from among a het and a hetaryl, which in turn may optionally be substituted by one or more groups selected from among halogen, CF₃, CHF₂, CH₂F, OH and oxo or by one or more groups selected from among OR^{2.1}, C₁₋₃-alkylene-OR^{2.1}, SR^{2.1}SO-R^{2.1}, SO₂-R^{2.1}, COOR^{2.1}, COR^{2.1}, C₁₋₆-alkanol, C₁₋₃-alkylene-OR^{2.1}, C₃₋₁₀-cycloalkyl, C₆₋₁₀-aryl, C₁₋₆-alkyl, C₆₋₁₀-aryl-C₁₋₆-alkylene, hetaryl-C₁₋₆-alkylene, het, hetaryl and NR^{2.2}R^{2.3}, which in turn may optionally be substituted by one or more groups selected from among OH, OR^{2.1}, oxo, halogen, CF₃, CHF₂, CH₂F, C₁₋₆-alkyl, C₆₋₁₀-aryl and NR^{2.2}R^{2.1},
and wherein
**R³** denotes a group selected from among a mono- or bicyclic C₆₋₁₀-aryl, a het and a hetaryl, which may optionally be substituted by one or more groups selected from among halogen, -C₁₋₃-alkyl, cyclopropyl, -C₁₋₃-fluoroalkyl, -C₁₋₃-alkylene-OR^{2.1} -OH and -O-C₁₋₃-alkyl
and wherein
a) **R³, R⁴, R^{4'} and R⁵** denote H and
**R⁶ and R⁷** together form a bridge selected from among methylene, ethylene and propylene,
or wherein
b) **R³, R⁴, R^{4'} and R⁷** denote H and
**R⁵ and R⁶** together form a bridge selected from among methylene, ethylene and propylene,
or wherein
d) **R⁵, R⁶ and R⁷** denote H and
**R³, R⁴ and R^{4'}** together with the C and the N atom of the piperazine form an unsaturated or partially unsaturated, 5- or 6-membered heterocyclic group containing two or three, but at least two nitrogen atoms,
wherein this heterocyclic group may optionally be substituted by one, two or three groups selected from among oxo, OH, halogen, C₁₋₆-alkyl, cyclopropyl, C₁₋₆-fluoroalkyl, C₁₋₃-alkylene-OR^{2.1}, C₁₋₆-alkanol, COOR^{2.1}, C₆₋₁₀-aryl, C₁₋₃-alkylene-C₆₋₁₀-aryl, hetaryl, C₁₋₃-alkylene-hetaryl, het, CO-het, C₁₋₃-alkylene-het, -CO-N(CH₃)-het, -CO-NH-het, -CO-N(CH₃)-C₃₋₇-cycloalkyl, -CO-NH-C₃₋₇-cydaalkyl, -CO-NH-C₁₋₂-alkylene-hetaryl, -CO-N(CH₃)-C₁₋₂-alkylene-hetaryl, -CO-NH-CH(CH₃)-hetaryl, -CO-NH-C(CH₃)₂-hetaryl, -CO-N(cyclopropyl)-het and -CO-N(cyclopropyl)-hetaryl, which in turn may optionally be substituted by one or more groups selected from among OH, oxo, halogen, C₁₋₆-alkyl, C₁₋₃-fluoroalkyl, C₁₋₆-alkanol,
and the pharmacologically acceptable salts thereof.

3. Compounds of formula 1 according to one of claims 1 or 2, wherein
**X** denotes SO,
**R¹** denotes H
**R²** denotes H or C₁₋₁₀-alkyl, which may optionally be substituted by one or more groups selected from halogen and C₁₋₃-fluoroalkyl or which may optionally be substituted by one or more groups selected from among OR^{2.1}, COOR^{2.1}, CONR^{2.2}R^{2.3}, SR^{2.1}, SO-R^{2.1}, SO₂-R^{2.1}, phenyl, a het, a hetaryl, a monocyclic C₃₋₇-cycloalkyl, CH₂-NR^{2.2}R^{2.3}, and NR^{2.2}R^{2.3}, which in turn may optionally be substituted by one or more groups selected from among OH, halogen, OR^{2.1}, oxo, CF₃, CHF₂, CH₂F, C₁₋₆-alkyl, C₁₋₆-alkanol, phenyl, COOR^{2.1}, CH₂-NR^{2.2}R^{2.3} and NR^{2.2}R^{2.3},
wherein R^{2.1} is H or a group selected from among C₁₋₅-alkyl, C₁₋₆-alkanol, C₁₋₃-haloalkyl, monocyclic C₃₋₇ cycloalkyl, phenyl-C₁₋₆-alkylene, hetaryl-C₁₋₆-alkylene, het-C₁₋₆-alkylene, C₁₋₇-cycloalkyl-C₁₋₆-alkylene, phenyl, a hetaryl and a het,
which may optionally be substituted by one or more groups selected from among OH, halogen, C₁₋₆-alkyl, O-(C₁₋₃-alkyl) and phenyl,
wherein **R^{2.2}** and **R^{2.3}** independently of one another denote H or a group selected from among C₁₋₆-alkyl, monocyclic C₃₋₇ cycloalkyl, phenyl-C₁₋₃-alkylene, hetaryl-C₁₋₃-alkylene, phenyl, monocyclic C₃₋₇ heterocycle, hetaryl, CO-NH₂, CO-NHCH₃, CON(CH₃)₂, SO₂-(C₁-C₂-alkyl), CO-R^{2.1}, and COOR^{2.1}, which may optionally be substituted by one or more groups selected from among OH, halogen, C₁₋₆-alkyl, phenyl and COOR^{2.1}.
wherein
**het** is a three-to seven-membered, monocyclic, saturated or partially saturated heterocyclic group, which contains 1, 2 or 3 heteroatoms selected independently of one another from among N, S or O,
and wherein
**hetaryl** is a five- to six-membered, monocyclic, aromatic heteroaryl, which contains 1, 2 or 3 heteroatoms selected independently of one another from among N, S or O,
and wherein
**cycloalkyl** may be saturated or partially saturated,
or
**R**² denotes a monocyclic C₃₋₇ cycloalkyl, which may optionally be substituted by a group selected from among branched or unbranched C₁₋₆-alkanol, C₁₋₃-fluoroalkyl, C₁₋₃-alkylene-OR^{2.1}, OR^{2.1}, COOR^{2.1}, SO₂-NR^{2.2}R^{2.3}, het, phenyl, C₁₋₆-alkyl, phenyl-C₁₋₆-alkylone, hetaryl-C₁₋₆-alkylene, monocyclic C₃₋₇ cycloalkyl and NR^{2.2}R^{2.3}, which may optionally be substituted by one or more groups selected from among OH, OR^{2.1}, oxo, halogen, CF₃, CHF₂, CH₂F, C₁₋₆-alkyl, phenyl and NR^{2.2}R^{2.3},
or
**R²** denotes a phenyl, which may optionally be substituted by OH, SH or halogen or by one or more groups selected from among OR^{2.1}, COOR^{2.1}, NR^{2.2}R^{2.3}, CH₂-NR^{2.2}R^{2.3}, C₃₋₇-cycloalkyl, het, C₁₋₆-alkyl, CF₃, CHF₂, CH₂F, phenyl-C₁₋₆-alkylene, het-C₁₋₆-alkylene, hetaryl-C₁₋₆,alkylene, phenyl, SO₂-CH₃, SO₂-CH₂CH₃ and SO₂-NR^{2.2}R^{2.3}, which in turn may optionally be substituted by one or more groups selected from among OH, OR^{2.1}, oxo, halogen, CF₃, CHF₂, CH₂F, C₁₋₆-alkyl, phenyl and NR^{2.2}R^{2.3},
or
**R²** denotes a group selected from among a het and a hetaryl, which in turn may optionally be substituted by one or more groups selected from among halogen, OH, oxo, CF₃, CHF₂ and CH₂F or by one or more groups selected from among OR^{2.1}, C₁₋₃-alkylene-OR^{2.1}, SR^{2.1},SO-R^{2.1}, SO₂-R^{2.1}, COOR^{2.1}, COR^{2.1}, C₁₋₆-alkanol, C₃₋₁₀-cycloalkyl, phenyl, C₁₋₆-alkyl, phenyl-C₁₋₆-alkylene, hetaryl-C₁₋₆-alkylene, het, hetaryl, C₁₋₆-alkanol, C₁₋₃-alkylone-OR^{2.1} and NR^{2.2}R^{2.3}, which in turn may optionally be substituted by one or more groups selected from among OH, OR^{2.1}, oxo, halogen, CF₃, CHF₂, CH₂F, C₁₋₆-alkyl, phenyl and NR^{2.2}R^{2.3},
and wherein
**R³** denotes a group selected from among a mono- or bicyclic C₁₋₁₀-aryl, a het and a hetaryl, which may optionally be substituted by one or more groups selected from among halogen, C₁₋₃-alkyl, cyclopropyl, C₁₋₃-fluoroalkyl, C₁₋₃-alkanol, C₁₋₃-alkylene-OR^{2.1}, OH and O-C₁₋₃-alkyl
and wherein
a) **R³**, **R⁴**, **R**^{**4**'} and **R⁵** denote H and **R⁶ and R⁷** together form a bridge selected from among methylene, ethylene and propylene,
or wherein
b) **R³, R⁴, R^{4'} and R⁷** denote H and
**R⁵ and R⁶** together form a bridge selected from among methylene, ethylene and propylene,
or wherein
d) **R⁵, R⁶ and R⁷** denote H and
**R³**, **R⁴ and R**^{**4**'} together with the C- and the N atom of the piperazine form an unsaturated or partially unsaturated, 5- or 6-membered heterocyclic group containing two or three, but at least two nitrogen atoms,
wherein this heterocyclic group may optionally be substituted by one, two or three groups selected from among oxo, OH, halogen, C₁₋₆-alkyl, cyclopropyl, C₁₋₃-fluoroalkyl, C₁₋₃-alkylene-OR^{2.1}, C₁₋₆-alkanol, COOR^{2.1}, C₆₋₁₀-aryl, C₁₋₃-alkylene-C₆₋₁₀-aryl, hetaryl, C₁₋₃-alkylene-hetaryl, het, CO-het, C₁₋₃-alkylene-het -CO-N(CH₃)-het, -CO-NH-het, -CO-O-N(CH₃)-het, -CO-O-NH-het, -CO-N(CH₃)-C₃₋₇-cycloalkyl, -CO-NH-C₃₋₇-cycloalkyl, -CO-O-N(CH₃)-C₃₋₇-cycloalkyl, -CO-O-NH-C₃₋₇-cycloalkyl, -CO-NH-C₁₋₂-alkylene-hetaryl, -CO-N(CH₃)-C₁₋₂-alkylene-hetaryl, -CO-NH-CH(CH₃)-hetaryl, -CO-NH-C(CH₃)₂-hetaryl, -CO-N(cyclopropyl)-het and -CO-N(cyclopropyl)-hetaryl,
which in turn may optionally be substituted by one or more groups selected from among OH, oxo, halogen, C₁₋₆-alkyl, C₁₋₃-fluoroalkyl and C₁₋₆-alkanol,
and the pharmacologically acceptable salts thereof.

4. Compounds of formula **1** according to one of claims 1 to 3, wherein
**X** is SO,
**R²** is H or C₁₋₆-alkyl, which may optionally be substituted by F, CF₃, CHF₂ or CH₂F or which in turn may optionally be substituted by one or more groups selected from among OR^{2.1}, COOR^{2.1},CONR^{2.2}R^{2.3}, SR^{2.1}, SO-R^{2.1}, SO₂-R^{2.1}, phenyl, a het, a hetaryl, a monocyclic C₃₋₇-cycloalkyl, CH₂-NR^{2.2}R^{2.3} and NR^{2.2}R^{2.3}, which in turn may optionally be substituted by one or more groups selected from among OH, halogen, CF₃, CHF₂, CH₂F, OR^{2.1}, oxo, C₁₋₆-alkyl, C₁₋₆-alkanol, phenyl, COOR^{2.1}, CH₂-NR^{2.2}R^{2.3} and NR^{2.2}R^{2.3},
wherein **R^{2.1}**, is H or a group selected from among C₁₋₆-alkyl, C₁₋₆-alkanol, monocyclic C₃₋₇ cycloalkyl, phenyl-CH₂, hetaryl-methylene, het-methylene, C₃₋₇-cycloalkyl-methylene, phenyl, a hetaryl and a het,
which may optionally be substituted by one or more groups selected from among OH, F, Cl, Br, I, methyl, ethyl, propyl, isopropyl, O-methyl, O-ethyl, O-propyl, O-isopropyl and phenyl,
wherein **R^{2.2} and R^{2.3}** independently of one another denote H or a group selected from among methyl, ethyl, propyl, isopropyl, monocyclic C₃₋₇ cycloalkyl, phenylmethylene, hetaryl-methylene, phenyl, het, hetaryl, CO-NH₂, CO-NHCH₃, CON(CH₃)₂, SO₂-(C₁-C₂-alkyl), CO-R^{2.1} and COOR^{2.1}, which may optionally be substituted by one or more groups selected from among OH, F, Cl, Br, methyl, ethyl, phenyl and COOR^{2.1},
and wherein **R¹, R³, R⁴, R^{4'}, R⁵, R⁶ and R⁷** are defined as in one of claims 1 to 3,
and the pharmacologically acceptable salts thereof.

5. Compounds of formula **1** according to one of claims 1 to 3, wherein
**R²** is a group according to formula **2** 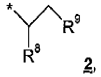
wherein **R⁹** is OH or NH₂ and
wherein **R⁸** is a group selected from among C₁₋₆-alkyl, hetaryl and phenyl, which may optionally be substituted by one or more groups selected from among OH, F, Br, OR^{2.1}, oxo, methyl, ethyl, C₁₋₂-alkanol, phenyl, COOR^{2.1}, CH₂-NR^{2.2}R^{2.3} and NR^{2.2}R^{2.3},
and the pharmacologically acceptable salts thereof.

6. Compounds of formula **1** according to claim 5, wherein
**R²** is a group according to formula **2** 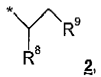
wherein **R⁹** is OH or NH₂ and
wherein **R⁸** is methyl, ethyl, propyl, isopropyl
and the pharmacologically acceptable salts thereof.

7. Compounds of formula **1** according to one of claims 1 to 3, wherein
**R²** is a monocyclic three-, four-, five-, six- or seven-membered cycloalkyl ring which may optionally be substituted in the spiro position by a group selected from among -CH₂-OR^{2.1}, branched or unbranched C₂₋₆-alkylene-OR^{2.1}, methyl, ethyl, propyl, isopropyl, butyl, isobutyl, cyclopropyl, and -CF₃, CHF₂, CH₂F and C₂₋₄-fluoroalkyl, wherein
**R^{2.1}** is selected from among methyl, ethyl, propyl, isopropyl, butyl, isobutyl,
and the pharmacologically acceptable salts thereof.

8. Compounds of formula **1** according to one of claims 1 to 3, wherein
**R²** is a phenyl, which is optionally substituted in one or both meta positions by one or more groups selected from among methyl, ethyl, propyl, isopropyl, cyclopropyl, F, Cl, Br, OH, OR^{2.1}, COOR^{2.1}, CF₃, CHF₂, CH₂F, NH₂, NH(CH₃) and N(CH₃)₂, wherein R^{2.1} may be H, methyl or ethyl,
and the pharmacologically acceptable salts thereof.

9. Compounds of formula **1** according to one of claims 1 to 3, wherein
**R²** is a group selected from among monocyclic, saturated three-, four-, five-, six- or seven-membered heterocyclic groups with 1, 2 or 3 heteroatoms in each case selected from among N, O and S, which may optionally be substituted by one or more groups selected from among fluorine, OH, CF₃, CHF₂, CH₂F and oxo or by one or more groups selected from among OR^{2.1}, C₁₋₃-alkylene-OR^{2.1}, SR^{2.1},SO-R^{2.1}, SO₂-R^{2.1}, COOR^{2.1}, COR^{2.1}, C₁₋₆-alkanol, C₃₋₁₀-cycloalkyl, phenyl, C₁₋₆-alkyl, phenyl-C₁₋₆-alkylene, hetaryl-C₁₋₆-alkylene, het, hetaryl and **NR^{2.2}R^{2.2}**, which in turn may optionally be substituted by one or more groups selected from among OH, OR^{2.1}, oxo, F, Cl, CF₃, CHF₂, CH₂F, C₁₋₆-alkyl, phenyl and **NR^{2.2}R^{2.3}**,
wherein **R^{2.1}**, **R^{2.2}** and **R^{2.3}** are defined as in claims 1 to 3,
and the pharmacologically acceptable salts thereof.

10. Compounds of formula **1** according to claim 9, wherein
**R²** is a group selected from among a monocyclic, saturated six-membered heterocyclic group with at least one heteroatom selected in each case from among N, O and S, which may optionally be substituted by one or more groups selected from among F, Cl, Br, CF₃, CHF₂, CH₂F, OH, oxo, NH₂, NHCH₃, N(CH₃)₂, methyl, ethyl, propyl, isopropyl, cyclopropyl, methoxy and ethoxy,
and the pharmacologically acceptable salts thereof.

11. Compounds of formula **1** according to claim 10, wherein
**R²** is a group selected from among piperidine or tetrahydropyran, which may optionally be substituted by one or more groups selected from among F, Cl, Br, OH, CF₃, CHF₂, CH₂F, NH₂, NHCH₃, N(CH₃)₂. oxo, methyl and methoxy,
and the pharmacologically acceptable salts thereof.

12. Compounds of formula **1** according to one of claims 1 to 3, wherein
**R³** is a group selected from the group comprising a phenyl, which may optionally be substituted by one or more groups selected from among F, Cl, Br, methyl, ethyl, propyl, isopropyl, cyclopropyl, C₁₋₃-fluoroalkyl, C₁₋₃-alkanol, OH and O-C₁₋₃-alkyl,
and the pharmacologically acceptable salts thereof.

13. Compounds of formula **1** according to one of claims 1 to 3, wherein
**R³** is a group selected from among a mono- or bicyclic, saturated, unsaturated or partially saturated three-to ten-membered heterocyclic group with 1 to 3 heteroatoms selected from N, O and S, which may optionally be substituted by one or more groups selected from among F, Cl, Br, methyl, ethyl, propyl, isopropyl, cyclopropyl, C₁₋₃-fluoroalkyl, C₁₋₃-alkanol, OH and O-C₁₋₃-alkyl,
and the pharmacologically acceptable salts thereof.

14. Compounds of formula 1 according to one of claims 1 to 3, wherein
**R³ and R⁴** together with the C and the N atom of the piperazine form a saturated or partially unsaturated 5- or 6-membered heterocyclic group containing two or three, but at least two nitrogen atoms,
or wherein
**R^{3,} R⁴ and R^{4'}** together with the C- and the N atom of the piperazine form an unsaturated or partially unsaturated, 5- or 6-membered heterocyclic group containing two or three, but at least two nitrogen atoms,
wherein this heterocyclic group may optionally be substituted by one, two or three groups selected from among oxo, OH, F, Cl, Br, C₁₋₆-alkyl, cyclopropyl, C₁₋₃-fluoroalkyl, C₁₋₆₋alkanol, COOR^{2.1}, phenyl, C₁₋₃-alkylene-phenyl, hetaryl, C₁₋₃-alkylene-hetaryl, het, CO-het, C₁₋₃-alkylene-het, -CO-N(CH₃)-het, -CO-NH-het, -COO-N(CH₃)-het, -CO-O-NH-het, -CO-N(CH₃)-C₃₋₇-cycloalkyl, -CO-NH-C₃₋₇-cycloalkyl, -CO-O-N(CH₃)-C₃₋₇-cycloalkyl, -CO-O-NH-C₃₋₇-cycloalkyl, -CO-N H-C₁₋₂-alkylene-hetaryl, -CO-N(CH₃)-C₁₋₂-alkylene-hetaryl, -CO-NH-CH(CH₃)-hetaryl, -CO-NH-C(CH₃)₂-hetaryl, -CO-N(cyclopropyl)-het and -CO-N(cyclopropyl)-hetaryl,
which in turn may optionally be substituted by one or more groups selected from among OH, oxo, F, Cl, Br, C₁₋₆-alkyl, C₁₋₆-fluoroalkyl, C₁₋₆-alkanol,
and the pharmacologically acceptable salts thereof.

15. Compounds of formula **1** according to claim 14, wherein
**R³, R⁴ and R^{4'}** together with the C and the N atom of the piperazine form a five-membered heterocyclic group selected from among imidazole, pyrazole and triazole, which may optionally be substituted by one, two or three groups selected from among oxo, OH, F, Cl, Br, C₁₋₃-fluoroalkyl, methyl, ethyl, propyl, isopropyl, cyclopropyl, COOR^{2.1}, phenyl, C₁₋₃-alkylene-phenyl, hetaryl, C₁₋₃-alkylene-hetaryl, het, CO-het, C₁₋₃-alkylene-het, -CO-N(CH₃)-het, -CO-NH-het, -CO-O-N(CH₃)-het, -CO-O-NH-het, -CO-N(CH₅)-C₃₋₇-cycloalkyl, -CO-NH-C₂₋₇-cycloalkyl, -CO-O-N(CH₃)-C₃₋₇-cycloalkyl, -CO-O-NH-C₃₋₇-cycloalkyl, -CO-NH-C₁₋₂-alkylene-hetaryl, -CON(CH₃)-C₁₋₂-alkylene-hetaryl, -CO-NH-CH(CH₃)-hetaryl, -CO-NH-C(CH₃)₂-hetaryl, - CO-N(cyclopropyl)-het and -CO-N(cyclopropyl)-hetaryl, which in turn may optionally be substituted by one or more groups selected from among OH, oxo, F, Cl, Br, C₁₋₆-alkyl, C₁₋₃-fluoroalkyl and C₁₋₆-alkanol,
wherein **R^{2.1}** may be H, methyl, ethyl, propyl or isopropyl,
and the pharmacologically acceptable salts thereof.

16. Compounds of formula **1** according to one of claims 1 to 13,
wherein
a) **R³**, **R⁴**, **R^{4'} and R⁵** denote H and
**R⁶ and R⁷** together form a bridge selected from among methylene and ethylene, or wherein
b) **R³, R⁴, R^{4'} and R⁷** denote H and
**R⁵ and R⁶** together form a bridge selected from among methylene and ethylene,
and the pharmacologically acceptable salts thereof.

17. Compounds of formula **1** according to one of claims 1 to 4, wherein the compound is selected from among 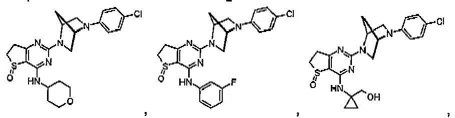 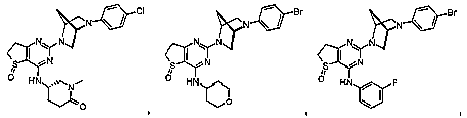 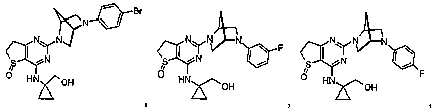 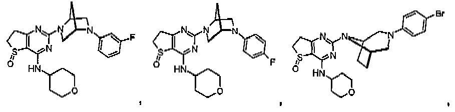 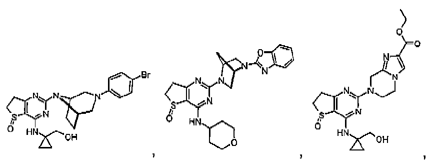 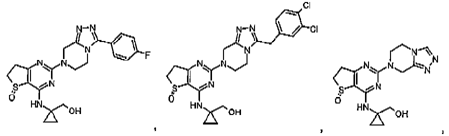 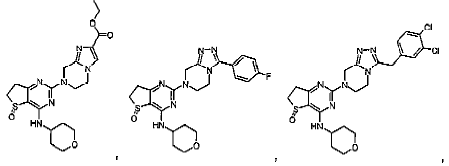 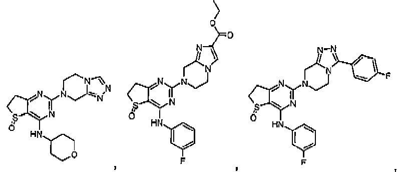 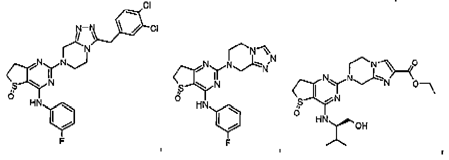 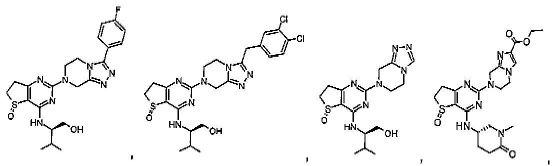 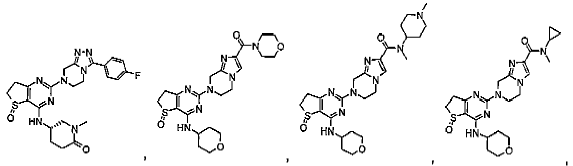 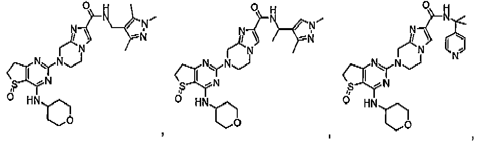 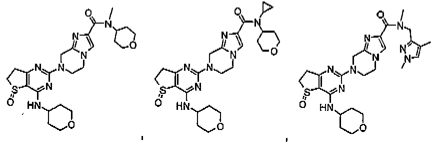 and 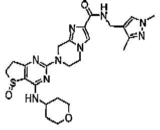 and the pharmacologically acceptable salts thereof.

18. Compounds according to one of claims 1 to 17 as medicaments.

19. Use of compounds according to one of claims 1 to 17, for preparing a medicament for the treatment of respiratory or gastrointestinal complaints or diseases, such as inflammatory diseases of the joints, skin or eyes, cancers, and diseases of the peripheral or central nervous system.

20. Use of compounds according to one of claims 1 to 17 for preparing a medicament for the prevention and treatment of respiratory or pulmonary diseases which are associated with increased mucus production, inflammation and/or obstructive diseases of the respiratory tract.

21. Use of compounds according to one of claims 1 to 17 for preparing a medicament for the treatment of inflammatory and obstructive diseases such as COPD, chronic sinusitis, asthma, Crohn's disease, ulcerative colitis.

22. Use of compounds according to one of claims 1 to 17 for preparing a medicament for the prevention and treatment of diseases of the peripheral or central nervous system such as depression, bipolar or manic depression, acute and chronic anxiety states, schizophrenia, Alzheimer's disease, Parkinson's disease, acute and chronic multiple sclerosis or acute and chronic pain as well as injury to the brain caused by stroke, hypoxia or cranio-cerebral trauma.

23. Pharmaceutical formulations **characterised in that** they contain one or more compounds of formula 1 according to one of claims 1 to 17 combined with one or more active substances selected from among betamimetics, corticosteroids, other PDE4-inhibitors, EGFR- inhibitors and LTD4-antagonists, CCR3-inhibitors, iNOS-inhibitors and SYK-inhibitors.

## Revendications

1. Composés de formule 1 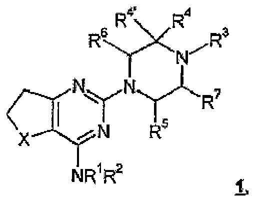 dans laquelle
X est SO ou SO₂
R¹ est H, un groupe alkyle en C₁ à C₈,
R² est H ou un radical choisi dans le groupe comprenant un groupe alkyle en C₁ à C₁₀ et alcényle en C₃ à C₆ qui peut être substitué éventuellement par un ou plusieurs radicaux choisis parmi un atome d'halogène et un groupe fluoroalkyle en C₁ à C₃ ou qui peut être substitué éventuellement par un ou plusieurs radicaux choisis dans le groupe comprenant OR^{2,1} COOR^{2,1}, CONR^{2,2}R^{2,3}, SR^{2,1}, SO-R^{2,1}, SO₂R^{2,1}, un groupe aryle en C₆ à C₁₀, un groupe het, un groupe hétaryle, un groupe cycloalkyle en C₃ à C₁₀ mono- ou bicyclique, CH₂-NR^{2,2}R_{2,3} et NR^{2,2}R^{2,5}, qui peut lui-même être substitué éventuellement par un ou plusieurs radicaux choisis dans le groupe comprenant OH, un atome d'halogène, OR^{2,1}, un groupe oxo, CF₃, CHF₂, CH₂F, alkyle en C₁ à C₆, alcanol en C₁ à C₆, aryle en C₆ à C₁₀, COOR^{2,1}, CH₂-NR^{2,2}R^{2,3} et NR^{2,2}R^{2,3},
dans laquelle R^{2,1} est H ou un radical choisi dans le groupe comprenant un groupe alkyle en C₁ à C₆, alcanol en C₁ à C₆, fluoroalkyle en C₁ à C₃, cycloalkyle en C₈ à C₁₀ mono- ou bicyclique, aryle en C₆ à C₁₀-alkylène en C₁ à C₆, hétaryl-alkylène en C₁ à C₆,
Het-alkylène en C₁ à C₆, cycloalkyle en C₃ à C₁₀-alkylène en C₁ à C₆, un groupe aryle en C₆ à C₁₀, un groupe hétaryle et un groupe Het qui peut être substitué éventuellement par un ou plusieurs radicaux choisis dans le groupe comprenant OH, O-(alkyle en C₁ à C₃), un atome d'halogène, un groupe alkyle en C₁ à C₆ et aryle en C₈ à C₁₀,
dans laquelle R^{2,2} et R^{2,3} sont indépendamment l'un de l'autre, H ou un radical choisi dans le groupe comprenant un groupe alkyle en C₁ à C₆, cycloalkyle en C₃ à C₁₀ mono- ou bicyclique, aryle en C₈ à C₁₀-alkylène en C₁ à C₈, hétaryl-alkylène en C₁ à C₆, aryle en C₆ à C₁₀ mono- ou bicyclique, un groupe het, un groupe hétaryle, CO-NH₂, CO-NH(CH₃), CO-N(CH3)₂, SO₂-(alkyle en C₁ à C₂), CO-R^{2,1} et COOR^{2,1},
qui peut être substitué éventuellement par un ou plusieurs radicaux choisis dans le groupe comprenant OH, un atome d'halogène, un groupe alkyle en C₁ à C₈, aryle en C₆ à C₁₀ et COOR^{2,1},
dans laquelle
le groupe het est un hydrocycle de trois à onze chaînons, mono- ou bicyclique, saturé ou partiellement saturé, éventuellement annelé ou éventuellement ponté qui contient 1, 2, 3 ou 4 hétéroatomes choisis indépendamment dans le groupe comprenant N, S ou O,
et dans laquelle
le groupe hétaryle est un groupe hétéroaryle de cinq à dix chaînons, mono- ou bicyclique, éventuellement annelé qui contient 1, 2, 3 ou 4 hétéroatomes choisis indépendamment les uns des autres dans le groupe comprenant N, S ou O,
et dans laquelle
le groupe cycloalkyle peut être saturé ou partiellement saturé,
ou
R² est un groupe cycloalkyle en C₃ à C₁₀ mono- ou polycyclique qui peut éventuellement être ponté de façon simple ou multiple par des groupes alkyle en C₁ à C₃ et qui peut éventuellement être substitué par un radical choisi dans le groupe comprenant un groupe alcanol en C₁ à C₆, fluoroarkyle en C₁ à C₃, alkylène en C₁ à C₃-OR^{2,1}, OR^{2,1}, COOR^{2,1}, SO₂-NR^{2,2}R^{2,3}, Het, aryle en C₆ à C₁₀, alkyle en C₁ à C₆, aryle en C₆ à C₁₀-alkylène en C₁ à C₆, hétaryl-alkylène en C₁ à C₆, cycloalkyle en C₃ à C₁₀ mono- ou bicyclique et NR^{2,2}R^{2,3} qui peut être éventuellement substitué par un ou plusieurs radicaux choisis dans le groupe comprenant OH, OR^{2,1}, oxo, un atome d'halogène, CF₃, CHF₂, CH₂F, alkyle en C₁ à C₆, aryle en C₆ à C₁₀ et NR²²R²³,
ou
R² est un groupe aryle en C₆ à C₁₀ mono- ou polycyclique qui peut être substitué éventuellement par OH, SH ou un atome d'halogène ou par un ou plusieurs radicaux choisis dans le groupe comprenant OR^{2,1}, COOR^{2,1}, NR^{2,2}R^{2,3}, CH₂-NR^{2,2}R^{2,3}, cycloalkyle en C₃ à C₁₀, Het, alkyle en C₁ à C₆, fluoroalkyle en C₁ à C₃, aryle en C₆ à C₁₀-alkylène en C₁ à C₆, Het-alkylène en C₁ à C₆, hétaryle-alkylène en C₁ à C₆, aryle en C₆ à C₁₀, SO₂-CH₃, SO₂-CH₂CH₃ et SO₂-NR^{2,2}R^{2,3}, qui peut lui-même être substitué éventuellement par un ou plusieurs radicaux choisis dans le groupe comprenant OH, OR^{2,1}, oxo, un atome d'halogène, CF₃, CHF₂, CH₂F, alkyle en C₁ à C₆, aryle en C₆ à C₁₀ et NR²²R²³,
ou
R² est un radical choisi dans un groupe constitué d'un groupe het et un groupe hétaryle qui peut être substitué éventuellement par un ou plusieurs radicaux choisis dans le groupe comprenant un atome d'halogène, OH, un groupe oxo, CF₃, CHF₂ et CH₂F ou par un ou plusieurs radicaux choisis dans le groupe comprenant OR²¹, un groupe alkylène en C₁ à C₃-OR^{2,1} SR^{2,1}, SO-R^{2,1} et SO₂-R^{2,1}, COOR^{2,1}, COR^{2,1}, alcanol en C₁ à C₆, cycloalkyle en C₃ à C₁₀, aryle en C₆ à C₁₀, alkyle en C₁ à C₆, aryle en C₆ à C₁₀-alkylène en C₁ à C₆, hétaryl-alkylène en C₁ à C₆, het, hétaryle, alkylène en C₁ à C₃-OR^{2,1} et NR^{2,2}R^{2,3},
qui peut éventuellement être substitué par un ou plusieurs radicaux choisis dans le groupe comprenant OH, OR²¹, oxo, un atome d'halogène, CF₃, CHF₂, CH₂F, alkyle en C₁ à C₆, aryle en C₆ à C₁₀ et NR²²R²³,
et dans laquelle
NR¹R² désigne conjointement un cycle de quatre à sept chaînons hétérocycliques qui peut être éventuellement ponté, qui contient 1, 2 ou 3 hétéroatomes choisis dans le groupe comprenant N, 0 et S et qui peut éventuellement être substitué par un ou plusieurs radicaux choisis dans le groupe comprenant OH, OR^{2,1}, alkylène en C₁ à C₃-OR^{2,1}, oxo, un atome d'halogène, un groupe alkyle en C₁ à C₆, aryle en C₆ à C₁₀, COOR^{2,1}, CH₂-NR^{2,2}-COO-R^{2,1}, CH₂-NR^{2,2}-CO-R^{2,1}, CH₂-NR^{2,2}-CO-CH₂-NR^{2,2}R^{2,3}, CH₂-NR^{2,2}-SO₂-alkyle en C₁ à C₃, CH₂-NR^{2,2}-SO₂-NR^{2,2}R^{2,3}, CH₂-NR^{2,2}-CO-NR^{2,2}R^{2,3}, CO-NR^{2,2}R^{2,5}, CH₂-NR^{2,2}R^{2,3} et NR^{2,2}R^{2,5},
et dans laquelle
R³ est un radical choisi dans le groupe comprenant un groupe aryle en C₆ à C₁₀, un groupe het et un groupe hétaryle qui peut être substitué éventuellement par un ou plusieurs radicaux choisis dans le groupe comprenant un atome d'halogène, un groupe alkyle en C₁ à C₃, cyclopropyle, fluoroalkyle en C₁ à C₃, alkylène en C₁ à C₃-OR²¹, -OH et O-alkyle en C₁ à C₃,
et dans laquelle
a) R⁵, R⁴, R^{4'} et R⁵ sont H et
R⁶ et R⁷ forment conjointement un pont choisi dans le groupe comprenant un groupe méthylène, éthylène et propylène qui peut être substitué éventuellement par un radical choisi dans le groupe constitué de -CH₃, -OH, - F, -CF₃, -CHF₂, -CH₂F, -NH₂, -NH(alkyle en C₁ à C₃), - N(alkyle en C₁ à C₃)₂ et O-(alkyle en C₁ à C₃),
ou dans laquelle
b) R³, R⁴, R^{4'} et R⁷ sont H et
R⁵ et R⁶ forment conjointement un pont choisi dans le groupe comprenant un groupe méthylène, éthylène et propylène qui peut être substitué éventuellement par un radical choisi dans le groupe comprenant CH₃, -OH, -F, -CF₃, -CHF₂, -CH₂F, -NH₂, -NH(alkyle en C₁ à C₃), - N(alkyle en C₁ à C₃)₂ et OCH₃,
et dans laquelle
c) R⁵, R⁶, R^{4'} et R⁷ sont H et
R³ et R⁴ conjointement avec l'atome de C et l'atome de N du groupe pipérazine forment un hétérocycle saturé ou partiellement insaturé de 5 ou 6 chaînons contenant deux ou trois, mais au moins deux atomes d'azote
ou dans laquelle
d) R⁵, R⁶ et R⁷ sont H,
R³, R⁴ et R^{4'} conjointement avec l'atome de C ou l'atome de N du groupe pipérazine forment un hétérocycle insaturé ou partiellement insaturé de 5 ou 6 chaînons contenant deux ou trois, mais au moins deux atomes d'azote,
chacun de ces hétérocycles saturé, insaturé ou partiellement insaturé de 5 ou 6 chaînons pouvant être substitué respectivement par un, deux ou trois radicaux du groupe comprenant le groupe oxo, OH, un atome d'halogène, un groupe alkyle en C₁ à C₆, cyclopropyle, fluoroalkyle en C₁ à C₃, alkylène en C₁ à C₃-OR^{2,1}, alcanol en C₁ à C₆, COOR^{2,1}, aryle en C₆ à C₁₀, alkylène en C₁ à C₃-aryle en C₆ à C₁₀, hétaryle, alkylène en C₁ à C₃-hétaryle, het, Co-Het, alkylène en C₁ à C₃-het, -CON(alkyle en C₁ à C₃)-het, -CO-NH-Het, -CO-O-N(alkyle en C₁ à C₃)-Het, -CO-O-NH-het, -CO-N(alkyle en C₁ à C₃)-cycloalkyle en C₃ à C₇, -CO-NH-cycloalkyle en C₃ à C₇, - CO-O-N(alkyle en C₁ à C₃)-cycloalkyle, -CO-O-NH-cycloalkyle en C₃ à C₇, -CO-NH-(alkylène en C₁ à C₂) - hétaryle, -CO-N(alkyle en C₁ à C₃)-alkylène en C₁ à C₂)-hétaryle, -CO-NH-CH(alkyle en C₁ à C₃)-hétaryle, -CO-NH-C(alkyle en C₁ à C₃)₂-hétaryle, -CO-N(cyclopropyl)-het et -CO-N(cyclopropyl)-hétaryle,
qui peut lui-même être substitué éventuellement par un ou plusieurs radicaux choisis dans le groupe OH, oxo, un atome d'halogène, un groupe alkyle en C₁ à C₆, fluoroalkyle en C₁ à C₃, alcanol en C₁ à C₆,
et leurs sels pharmacologiquement acceptables.

2. Composés de formule 1 selon la revendication 1,
dans laquelle
X est SO ou SO₂
R¹ est H,
R² est H ou un groupe alkyle en C₁ à C₁₀ qui peut être substitué éventuellement par un ou plusieurs radicaux choisis parmi un atome d'halogène et un groupe fluoroalkyle en C₁ à C₃ ou qui peut être substitué éventuellement par un ou plusieurs radicaux choisis dans le groupe comprenant OR^{2,1}, COOR^{2,1}, CONR^{2,2}R^{2,3}, SR^{2,1}, SO-R^{2,1}, SO₂R^{2,1}, un groupe aryle en C₆ à C₁₀, un groupe het, un groupe hétaryle, un groupe cycloalkyle en C₃ à C₁₀ mono- ou bicyclique, CH₂-NR^{2,2}R^{2,3} et NR^{2,2}R^{2,3}, qui peut lui-même être substitué éventuellement par un ou plusieurs radicaux choisis dans le groupe comprenant OH, un atome d'halogène, OR^{2,1}, un groupe oxo, CF₃, CHF₂, CH₂F, alkyle en C₁ à C6, alcanol en C₁ à C₆, aryle en C₆ à C₁₀, COOR^{2,1}, CH₂-NR^{2,2}R^{2,3} et NR^{2,2}R^{2,3},
dans laquelle
le groupe het est un hétérocycle de trois à sept chaînons, monocyclique, saturé ou partiellement saturé ou un hétérocycle de sept à onze chaînons bicyclique, saturé ou partiellement saturé qui contient 1, 2, 3 ou 4 hétéroatomes choisis indépendamment les uns des autres dans le groupe comprenant N, S ou O,
et dans laquelle
le groupe hétaryle est un groupe hétéroaryle de cinq à six chaînons, monocyclique, aromatique ou un groupe hétéroaryle de sept à onze chaînons bicyclique, aromatique qui contient 1, 2, 3 ou 4 hétéroatomes choisis indépendamment les uns des autres dans le groupe comprenant N, S ou O,
et dans laquelle
le groupe cycloalkyle peut être saturé ou partiellement saturé,
ou
R^{2,1}, R^{2,2} et R^{2,3} sont tels que définis dans la revendication 1,
ou
R² est un groupe cycloalkyle en C₃ à C₁₀ mono- ou polycyclique qui peut être ponté éventuellement de façon simple ou multiple par des groupes alkyle en C₁ à C₃ et qui peut être substitué éventuellement par un radical choisi dans le groupe comprenant un groupe alcanol ramifié ou non ramifié en C₁ à C₆, alkylène en C₁ à C₃-OR^{2,1}, OR^{2,1}, fluoroalkyle en C₁ à C₃, COOR^{2,1}, SO₂-NR^{2,2}R^{2,3}, het, aryle en C₈ à C₁₀, alkyle en C₁ à C6, aryle en C₆ à C₁₀-alkylène en C₁ à C₆, hétaryl-alkylène en C₁ à C₆, cycloalkyle mono- ou en C₃ à C₁₀ mono- ou bicyclique et NR^{2,2}R^{2,3}, qui peut éventuellement être substitué par un ou plusieurs radicaux choisis dans le groupe comprenant OH, OR^{2,1}, un groupe oxo, un atome d'halogène, CF₃, CHF₂, CH₂F, un groupe alkyle en C₁ à C₆, aryle en C₆ à C₁₀ et NR^{2,2}R2,3,
ou
R² est un groupe aryle en C₆ à C₁₀ mono- ou polycyclique qui peut être substitué éventuellement par OH, SH ou un atome d'halogène ou par un ou plusieurs radicaux choisis dans le groupe comprenant OR^{2,1}, COOR^{2,1}, NR^{2,2}R^{2,3}, CH2-NR^{2,2}R^{2,3}, cycloalkyle en C₃ à C₁₀, Het, alkyle en C₁ à C₆, fluoroalkyle en C₁ à C₃, aryle en C₆ à C₁₀-alkylène en C₁ à C₆, Het-alkylène en C₁ à C₆, hétaryle-alkylène en C₁ à C₆, aryle en C₆ à C₁₀, SO₂-CH₃, SO₂-CH₂CH₃ et SO₂-NR², 2R^{2,3}, qui peut lui-même être substitué éventuellement par un ou plusieurs radicaux choisis dans le groupe comprenant OH, OR^{2,1}, oxo, un atome d'halogène, CF₃, CHF₂, CH₂F, une groupe alkyle en C₁ à C₆, aryle en C₆ à C₁₀ et NR^{2,2}R^{2,3}
ou
R² est un radical choisi dans un groupe constitué d'un groupe het et un groupe hétaryle qui peut être substitué éventuellement par un ou plusieurs radicaux choisis dans le groupe comprenant un atome d'halogène, CF₃, CHF₂, CH₂F, OH et oxo ou par un ou plusieurs radicaux choisis dans le groupe comprenant OR^{2,1}, un groupe alkylène en C₁ à C₃-OR^{2,1}, SR^{2,1}, SO-R^{2,1}, SO2-R^{2,1}, COOR^{2,1}, COR^{2,1}, alcanol en C₁ à C₆, alkylène en C₁ à C₃-OR^{2,1}, cycloalkyle en C₃ à C₁₀, aryle en C₆ à C₁₀, alkyle en C₁ à C₆, aryle en C₆ à C₁₀-alkylène en C₁ à C₆, hétaryl-alkylène en C₁ à C₆, het, hétaryle et NR^{2,2}R^{2,3},
qui peut éventuellement être substitué à nouveau par un ou plusieurs radicaux choisis dans le groupe comprenant OH, OR^{2,1}, oxo, un atome d'halogène, CF₃, CHF₂, CH₂F, alkyle en C₁ à C₆, aryle en C₆ à C₁₀ et NR^{2,2}R^{2,3},
et dans laquelle
R³ est un radical choisi dans le groupe comprenant un groupe aryle en C₆ à C₁₀ mono- ou bicyclique, un groupe het et un groupe hétaryle qui peut être substitué éventuellement par un ou plusieurs radicaux choisis dans le groupe comprenant un atome d'halogène, un groupe alkyle en C₁ à C₃, cyclopropyle, fluoroalkyle en C₁ à C₃, alkylène en C₁ à C₃-OR^{2,1}, -OH et O-alkyle en C₁ à C₃,
et dans laquelle
a) R⁵, R⁴, R^{4'} et R⁵ sont H et
R⁶ et R⁷ forment conjointement un pont choisi dans le groupe comprenant un groupe méthylène, éthylène et propylène,
ou dans laquelle
b) R³, R⁴, R^{4'} et R⁷ sont H et
R⁵ et R⁶ forment conjointement un pont choisi dans le groupe comprenant un groupe méthylène, éthylène et propylène,
et dans laquelle
d) R⁵, R⁶ et R⁷ sont H et
R³ et R⁴ et R^{4'} conjointement avec l'atome de C et l'atome de N du groupe pipérazine forment un hétérocycle insaturé ou partiellement insaturé de 5 ou 6 chaînons contenant deux ou trois, mais au moins deux atomes d'azote
cet hétérocycle pouvant être substitué éventuellement par un, deux ou trois radicaux du groupe comprenant le groupe oxo, OH, un atome d'halogène, un groupe alkyle en C₁ à C₆, cyclopropyle, fluoroalkyle en C₁ à C₃, alkylène en C₁ à C₃-OR^{2,1}, alcanol en C₁ à C₆, COOR^{2,1}, aryle en C₆ à C₁₀, alkylène en C₁ à C₃-aryle en C₆ à C₁₀, hétaryle, alkylène en C₁ à C₃-hétaryle, het, Co-Het, alkylène en C₁ à C₃-het, -CO-N(alkyle en C₁ à C₃)-het, -CO-NH-Het, -CO-O-N(alkyle en C₁ à C₃)-Het, - CO-O-NH-het, -CO-N(alkyle en C₁ à C₃)-cycloalkyle en C₃ à C₇, -CO-NH-cycloalkyle en C₃ à C₇, -CO-O-N(alkyle en C₁ à C₃-cycloalkyle, -CO-O-NH-cycloalkyle en C₃ à C₇, - CO-NH-(alkylène en C₁ à C₂)-hétaryle, -CO-N(alkyle en C₁ à C₃)-alkylène en C₁ à C₂)-hétaryle, -CO-NH-CH(alkyle en C₁ à C₃)-hétaryle, -CO-NH-C(alkyle en C₁ à C₃)₂-hétaryle, -CO-N(cyclopropyl)-het et -CO-N(cyclopropyl)-hétaryle,
qui peut lui-même être substitué éventuellement par un ou plusieurs radicaux choisis dans le groupe OH, oxo, un atome d'halogène, un groupe alkyle en C₁ à C₆, fluoroalkyle en C₁ à C₃, alcanol en C₁ à C₆,
et leurs sels pharmacologiquement acceptables.

3. Composés de formule 1 selon l'une des revendications 1 ou 2, dans laquelle
X est SO
R¹ est h
R² est H ou un groupe alkyle en C₁ à C₁₀ qui peut être substitué éventuellement par un ou plusieurs radicaux choisis parmi un atome d'halogène et un groupe fluoroalkyle en C₁ à C₃ ou qui peut être substitué éventuellement par un ou plusieurs radicaux choisis dans le groupe comprenant OR^{2,1}, COOR^{2,1}, CONR^{2,2}R^{2,3}, SR^{2,1}, SO-R^{2,1}, SO2R^{2,1}, un groupe phényle, un groupe het, un groupe hétaryle, un groupe cycloalkyle en C₃ à C₇ monocyclique, CH₂-NR^{2,2}R^{2,3} et NR^{2,2}R^{2,3}, qui peut lui-même être substitué éventuellement par un ou plusieurs radicaux choisis dans le groupe comprenant OH, un atome d'halogène, OR^{2,1}, un groupe oxo, CF₃, CHF₂, CH₂F, alkyle en C₁ à C₆, alcanol en C₁ à C₆, phényle, COOR^{2,1}, CH2-NR^{2,2}R^{2,3} et NR^{2,2}R^{2,3}
dans laquelle R^{2,1} est H ou un radical choisi dans le groupe comprenant un groupe alkyle en C₁ à C₆, alcanol en C₁ à C₆, halogénoalkyle en C₁ à C₃, cycloalkyle en C₃ à C₇, phényle-alkylène en C₁ à C₆, hétaryl-alkylène en C₁ à C₆, het-alkylène en C₁ à C₆, cycloalkyle en C₃ à C₇-alkylène en C₁ à C₆, phényle, un groupe hétaryle et un groupe het qui peut éventuellement être substitué par un ou plusieurs radicaux choisis dans le groupe comprenant OH, un atome d'halogène, un groupe alkyle en C₁ à C₃, O-(alkyle en C₁ à C₃) et phényle,
R^{2,2} et R^{2,3} indépendamment l'un de l'autre étant H ou un radical choisi dans le groupe comprenant un groupe alkyle en C₁ à C₈, cycloalkyle en C₃ à C₇ monocyclique, phényl-alkylène en C₁ à C₃, hétaryl-alkylène en C₁ à C₃, phényle, un hétérocycle en C₃ à C₇ monocyclique, un groupe hétéroaryle, CO-NH₂, CO-NHCH₃, CON (CH₃)₂, SO₂- (alkyle en C₁ à C₂), CO-R^{2,1} et COOR^{2,1} qui peut éventuellement être substitué par un ou plusieurs radicaux choisis dans le groupe comprenant OH, un atome d'halogène, un groupe alkyle en C₁ à C₆, phényle et COOR^{2,1},
dans laquelle
le groupe het est un hétérocycle de trois à sept chaînons, monocyclique, saturé ou partiellement saturé qui contient 1, 2 ou 3 hétéroatomes choisis indépendamment les uns des autres dans le groupe comprenant N, S ou O,
et dans laquelle
le groupe hétaryle est un groupe hétéroaryle de cinq à six chaînons, monocyclique, aromatique qui contient 1, 2 ou 3 hétéroatomes choisis indépendamment les uns des autres dans le groupe comprenant N, S ou 0,
et dans laquelle
le groupe cycloalkyle peut être saturé ou partiellement saturé,
ou
R² est un groupe cycloalkyle en C₃ à C₇ monocyclique qui peut être substitué éventuellement par un radical choisi dans le groupe comprenant un groupe alcanol ramifié ou non ramifié en C₁ à C₆, fluoroalkyle en C₁ à C₃, alkylène en C₁ à C₃-OR^{2,1}, OR^{2,1}, COOR^{2,1}, SO₂-NR^{2,2}R^{2,3}, het, phényle, alkyle en C₁ à C₆, phényl-alkylène en C₁ à C₆, hétaryl-alkylène en C₁ à C₆, cycloalkyle monocyclique en C₃ à C₇ et NR^{2,2}, R^{2,3}, qui peut éventuellement être substitué par un ou plusieurs radicaux choisis dans le groupe comprenant OH, OR^{2,1}, un groupe oxo, un atome d'halogène, CF₃, CHF₂, CH₂F, un groupe alkyle en C₁ à C₆, phényle et NR^{2,2}R^{2,3},
ou
R² est un groupe phényle qui peut être substitué éventuellement par OH, SH ou un atome d'halogène ou par un ou plusieurs radicaux choisis dans le groupe comprenant OR^{2,1}, COOR^{2,1}, NR^{2,2}R^{2,3}, CH₂-NR^{2,2}R^{2,3}, cycloalkyle en C₃ à C₇, Het, alkyle en C₁ à C₆, CF₃, CHF₂, CH₂F, un groupe phényl-alkylène en C₁ à C₆, het-alkylène en C₁ à C₆, hétaryl-alkylène en C₁ à C₆, phényle, SO₂-CH₃-SO₂-CH₂CH₃ et SO₂NR^{2,2}R^{2,3} qui peut lui-même être substitué éventuellement par un ou plusieurs radicaux choisis dans le groupe comprenant OH, OR^{2,1}, un groupe oxo, un atome d'halogène, CF₃, CHF₂, CH₂F, un groupe allyle en C₁ à C₆, phényle et NR^{2,2}R^{2,3}
ou
R² est un radical choisi dans un groupe comprenant un groupe het et un groupe hétaryle qui peut être substitué éventuellement par un ou plusieurs radicaux choisis dans le groupe comprenant un atome d'halogène, OH, un groupe oxo, CHF₂ et CH₂F ou par un ou plusieurs radicaux choisis dans le groupe comprenant OR^{2,1}, un groupe alkylène en C₁ à C₃-OR^{2,1}, SR^{2,1}, SO-R^{2,1}, SO₂-R^{2,1}, COOR^{2,1}, COR^{2,1}, alcanol en C₁ à C₆, cycloalkyle en C₃ à C₁₀, phényle, alkyle en C₁ à C₆, phényl-alkylène en C₁ à C₆, hétaryl-alkylène en C₁ à C₆, het, hétaryle, alcanol en C₁ à C₆, alkylène en C₁ à C₃-OR^{2,1} et NR^{2,2}R^{2,3}, qui peut éventuellement être substitué à nouveau par un ou plusieurs radicaux choisis dans le groupe comprenant OH, OR^{2,1}, oxo, un atome d'halogène, CF₃, CHF₂, CH₂F, alkyle en C₁ à C₆, phényle et NR^{2,2}R^{2,3},
et dans laquelle
R³ est un radical choisi dans le groupe comprenant un groupe aryle en C₈ à C₁₀ mono- ou bicyclique, un groupe het et un groupe hétaryle qui peut être substitué éventuellement par un ou plusieurs radicaux choisis dans le groupe comprenant un atome d'halogène, un groupe alkyle en C₁ à C₃, cyclopropyle, fluoroalkyle en C₁ à C₃, alcanol en C₁ à C₃, alkylène en C₁ à C₃-OR^{2,1}, OH et O-alkyle en C₁ à C₃,
et dans laquelle
a) R³, R⁴, R^{4'} et R⁵ sont H et
R⁶ et R⁷ forment conjointement un pont choisi dans le groupe comprenant un groupe méthylène, éthylène et propylène,
ou dans laquelle
b) R³, R⁴, R^{4'} et R⁷ sont H et
R⁵ et R⁶ forment conjointement un pont choisi dans le groupe comprenant un groupe méthylène, éthylène et propylène,
ou dans laquelle
d) R⁵, R⁶ et R⁷ sont H et
R³, R⁴ et R⁴' conjointement avec l'atome de C et l'atome de N du groupe pipérazine forment un hétérocycle insaturé ou partiellement insaturé de 5 ou 6 chaînons contenant deux ou trois, mais au moins deux atomes d'azote
cet hétérocycle pouvant être substitué éventuellement par un, deux ou trois radicaux du groupe comprenant le groupe oxo, OH, un atome d'halogène, un groupe alkyle en C₁ à C₆, cyclopropyle, fluoroalkyle en C₁ à C₃, alkylène en C₁ à C₃-OR^{2,1}, alcanol en C₁ à C₆, COOR^{2,1}, aryle en C₆ à C₁₀, alkylène en C₁ à C₃-aryle en C₆ à C₁₀, hétaryle, alkylène en C₁ à C₃-hétaryle, het, CO-Het, alkylène en C₁ à C₃-het, -CO-N(CH₃)-het, -CO-NH-Het, -CO-O-N(CH₃)-Het, -CO-O-NH-het, -CO-N(CH₃)-cycloalkyle en C₃ à C₇, -CO-NH-cycloalkyle en C₃ à C₇, - CO-O-N(CH₃)-cycloalkyle en C₃ à C₇, -CO-O-NH-cycloalkyle en C₃ à C₇, -CO-NH-alkylène en C₁ à C₂-hétaryle, -CON(CH₃)-alkylène en C₁ à C₂-hétaryle, -CO-NH-CH(CH₃)-hétaryle, -CO-NH-C(CH₃)₂-hétaryle, -CO-N(cyclopropyl)-het et -CO-N(cyclopropyl)-hétaryle,
qui peut lui-même être substitué éventuellement par un ou plusieurs radicaux choisis dans le groupe OH, oxo, un atome d'halogène, un groupe alkyle en C₁ à C₆, fluoroalkyle en C₁ à C₃, alcanol en C₁ à C₆,
et leurs sels pharmacologiquement acceptables.

4. Composés de formule 1 selon les revendications 1 à 3, dans laquelle
X est SO
R² est H ou un groupe alkyle en C₁ à C₆ qui peut être substitué éventuellement par F, CF₃, CHF₂ ou CH₂F ou qui peut être substitué éventuellement par un ou plusieurs radicaux choisis dans le groupe comprenant OR^{2,1}, COOR^{2,1}, CONR^{2,2}R2,3, SR^{2,1}, SO-R^{2,1}, SO2R^{2,1}, un groupe phényle, un groupe het, un groupe hétaryle, un groupe cycloalkyle en C₃ à C₇ monocyclique, CH₂-NR^{2,2}R^{2,3} et NR^{2,2}R^{2,3}, qui peut lui-même être substitué éventuellement par un ou plusieurs radicaux choisis dans le groupe comprenant OH, un atome d'halogène, CF₃, CHF₂, CH_{2F}, OR^{2,1}, un groupe oxo, alkyle en C₁ à C₆, alcanol en C₁ à C₆, phényle, COOR^{2,1}, CH2-NR^{2,2}R^{2,3} et NR^{2,2}R^{2,3},
dans laquelle R^{2,1} est H ou un radical choisi dans le groupe comprenant un groupe alkyle en C₁ à C₆, alcanol en C₁ à C_{6,} cycloalkyle en C₃ à C₇ monocyclique, phényle-CH₂, hétaryl-méthylène, het-méthylène, cycloalkyle en C₃ à C₇-méthylène, phényle, un groupe hétaryle et un groupe het qui peut éventuellement être substitué par un ou plusieurs radicaux choisis dans le groupe comprenant OH, F, Cl, Br, I, un groupe méthyle, éthyle, propyle, isopxopyle, O-méthyle, 0-ëthyle, O-propyle, O-isopropyle et phényle,
R^{2,2} et R^{2,3} indépendamment l'un de l'autre étant H ou un radical choisi dans le groupe comprenant un groupe méthyle, éthyle, propyle, isopropyle, cycloalkyle en C₃ à C₇ monocyclique, phényl-méthylène, hétaryl-méthyléne, phényle, het, hétaryle, CO-NH₂, CO-NHCH₃, CON(CH₃)₂, SO₂-(alkyle en C₁ à C₂), CO-R^{2,1} et COOR^{2,1} qui peut éventuellement être substitué par un ou plusieurs radicaux choisis dans le groupe comprenant OH, F, Cl, Br, un groupe méthyle, éthyle, phényle et COOR^{2,1}
et dans laquelle R¹, R³, R⁴, R^{4'}, R⁵, R⁶ et R⁷ sont tels que définis dans les revendications 1 à 3,
et leurs sels pharmacologiquement acceptables

5. Composés de formule 1 selon l'une des revendications 1 à 3, dans laquelle
R² est un radical selon la formule 2 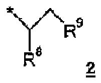 dans laquelle R⁹ est OH ou NH2 et
R⁸ est un radical choisi dans le groupe comprenant un groupe alkyle en C₁ à C₆, hétaryle et phényle qui peut être substitué éventuellement par un ou plusieurs radicaux choisis dans le groupe comprenant OH, F, Br, OR^{2,1}, oxo, un groupe méthyle, éthyle, alcanol en C₁ à C₂, phényle, COOR^{2,1}, CH2-NR^{2,2}R^{2,3} et NR^{2,2}R^{2,3},
et leurs sels pharmacologiqaement acceptables.

6. Composés de formule 1 selon la revendication 5, dans laquelle
R² est un radical selon la formule 2 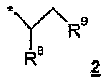 dans laquelle
dans laquelle R⁹ est OH ou NH₂ et
R⁸ est un groupe méthyle, éthyle, propyle, isopropyle
et leurs sels pharmacologiquement acceptables.

7. Composés de formule 1 selon l'une des revendications 1 à 3, dans laquelle
R² est un cycle cycloalkyle monocyclique de trois, quatre, cinq, six ou sept chaînons qui peut être substitué éventuellement en position spiro par un radical choisi dans le groupe comprenant -CH₂-OR^{2,1}, un groupe alkylène en C₂ à C₅-OR^{2,1} ramifié ou non ramifié, un groupe méthyle, éthyle, propyle, isopropyle, butyle, isobutyle, cyclopropyle et -CF₃, CHF₂, CH₂F et un groupe fluoroalkyle en C₂ à C₄, dans laquelle
R^{2,1} est choisi dans le groupe comprenant un groupe méthyle, éthyle, propyle, isopropyle, butyle, isobutyle
et leurs sels pharmacologiquement acceptables.

8. Composés de formule 1 selon l'une des revendications 1 à 3, dans laquelle
R² est un groupe phényle qui peut être substitué éventuellement dans une ou les deux positions méta, par une ou plusieurs radicaux choisis dans le groupe comprenant un groupe méthyle, éthyle, propyle, isopropyle, cyclopropyle, F, Cl, Br, OH, OR^{2,1}, COOR^{2,1}, CF₃ , CHF₂, CH₂F , NH₂, NH(CH₃) et N(CH₃)₂, R^{2,1} pouvant être H, un groupe méthyle ou éthyle,
et leurs sels pharmacologiquement acceptables.

9. Composés de formule 1 selon l'une des revendications 1 à 3, dans laquelle
R² est un radical choisi dans le groupe comprenant un hétérocycle monocyclique, saturé, de trois, quatre, cinq, six ou sept chaînons comportant 1, 2 ou 3 hétéroatomes choisis respectivement dans le groupe comprenant N, O et S qui peut éventuellement être substitué par un ou plusieurs radicaux choisis dans le groupe comprenant un atome de fluor, OH, CF₃, CHF₂, CH₂F et oxo ou par un ou plusieurs radicaux choisis dans le groupe comprenant OR^{2,1}, un groupe alkylène en C₁ à C₃-OR^{2,1}, SR^{2,1}, SO-R^{2,1}, SO2-R^{2,1}, COOR^{2,1}, alcanol en C₁ à C₆, cycloalkyle en C₃ à C₁₀, phényle, alkyle en C₁ à C₆, phényl-alkylène en C₁ à C₆, hétaryl-alkylène en C₁ à C₆, kiet, hétaryle et NR^{2,2}R^{2,3} qui peut lui-même être substitué éventuellement par un ou plusieurs radicaux choisis dans le groupe comprenant OH, OR^{2,1}, oxo, F, Cl, CF₃, CHF₂, CH₂F, un groupe alkyle en C₁ à C₆, phényle et NR^{2,2}R^{2,3}
R^{2,1}, R^{2,2} et R^{2,3} étant tels que définis dans les revendications 1 à 3,
et leurs sels pharmacologiquement acceptables.

10. Composés de formule 1 selon la revendication 9, dans laquelle
R² est un radical choisi dans le groupe comprenant un hétérocycle monocyclique, saturé, de six chaînons comportant au moins un hétéroatome choisi respectivement dans le groupe comprenant N, O et S qui peut éventuellement être substitué par un ou plusieurs radicaux choisis dans le groupe comprenant F, Cl, Br, CF₃, CHF₂, CH_{2F}, OH, oxo, NH₂, NHCH₃, N(CH₃)₂, un groupe méthyle, éthyle, isopropyle, cyclopropyle, méthoxy et éthoxy,
et leurs sels pharmacologiquement acceptables.

11. Composés de formule 1 selon la revendication 10, dans laquelle
R² est un radical choisi dans un groupe comprenant la pipéridine ou le tétrahydropyrane, qui peut éventuellement être substitué par un ou plusieurs radicaux choisis dans le groupe F, Cl, Br, OH, CF₃, CHF₂, CH₂F, NH₂, NHCH₃, N(CH₃)₂, un groupe oxi, méthyle et méthoxy,
et leurs sels pharmacologiquement acceptables.

12. Composés de formule 1 selon l'une des revendications 1 à 3, dans laquelle
R² est un radical choisi dans un groupe comprenant un groupe phényle qui peut éventuellement être substitué par un ou plusieurs radicaux choisis dans le groupe F, Cl, Br, méthyle, éthyle, propyle, isopropyle, cyclopropyle, fluoroalkyle en C₁ à C₃, alcanol en C₁ à C₃, OH et O-alkyle en C₁ à C₃,
et leurs sels pharmacologiquement acceptables.

13. Composés de formule 1 selon l'une des revendications 1 à 3, dans laquelle
R² est un radical choisi dans le groupe comprenant un hétérocycle mono- ou bicyclique, saturé, insaturé ou partiellement saturé de trois à dix chaînons comportant 1 à 3 hétéroatomes choisis parmi N, O et S qui peut être substitué éventuellement par un ou plusieurs radicaux choisis dans le groupe F, Cl, Br, un groupe méthyle, éthyle, propyle, isopropyle, cyclopropyle, fluoroalkyle en C₁ à C₃, alcanol en C₁ à C₃, OH et O-alkyle en C₁ à C₃,
et leurs sels pharmacologiquement acceptables.

14. Composés de formule 1 selon l'une des revendications 1 à 3, dans laquelle
R³ et R⁴ forment conjointement avec l'atome de C et de N de la pipérazine, un hétérocycle saturé ou partiellement insaturé de 5 ou 6 chaînons contenant deux ou trois, mais au moins deux atomes de carbone
ou dans laquelle
R³, R⁴ et R^{4'} forment, conjointement avec l'atome de C et de N de la pipérazine, un hétérocycle insaturé ou partiellement insaturé de 5 à 6 chaînons contenant deux ou trois mais au moins deux atomes d'azote,
cet hétérocycle pouvant être substitué éventuellement par un, deux ou trois radicaux choisis dans le groupe comprenant un groupe oxo, O_{H}, F, Cl, Br, alkyle en C₁ à C₆, cyclopropyle, fluoroalkyle en C₁ à C₃, alcanol en C₁ à C₆, COOR^{2,1}, phényle, alkylène en C₁ à C₃-phényle, hétaryle, alkylène en C₁ à C₅-hétaryle, het, CO-het, alkylène en C₁ à C₃-het, -CO-N(CH₃)het, -CO-NH-het, -CO-O-N(CH₃)-het, -CO-O-NH-het, -CO-N(CH3)-cycloalkyle en C₃ à C₇, -CO-NH-cycloalkyle en C₃ à C₇, - CO-O-N(CH₃)-cycloalkyle en C₃ à C₇, -CO-O-NH-cycloalkyle en C₃ à C₇, -CO-NH-alkylène en C₁ à C₂-hétaryle, -CON(CH₃)-alkylène en C₁ à C₂-hétaryle, -CO-NH-CH(CH₃)-hétaryle, -CO-NH-C(CH₃)₂-hétaryle, -CO-N(cyclopropyle)-het et -CO-N(cyclopropyl)-hétaryle,
qui peut lui-même être substitué éventuellement par un ou plusieurs radicaux choisis dans le groupe OH, oxo, F, Cl, Br, alkyle en C₁ à C₆, fluoroalkyle en C₁ à C₃, alcanol en C₁ à C₆,
et leurs sels pharmacologiquement acceptables.

15. Composés de formule 1 selon la revendication 14, dans laquelle
R³, R⁴ et R^{4'} forment, conjointement avec l'atome de C et de N de la pipérazine, un hétérocycle à cinq chaînons choisi dans le groupe comprenant l'imidazol, le pyrazol et le triazol, pouvant être substitué éventuellement par un, deux ou trois radicaux choisis dans le groupe comprenant un groupe oxo, OH, F, Cl, Br, fluoroalkyle en C₁ à C₃, méthyle, éthyle, propyle, isopropyle, cyclopropyle, COOR^{2,1}, phényle, alkylène en C₁ à C₃-phényle, hétaryle, alkylène en C₁ à C₃-hétaryle, het, CO-Yxet, alkylène en C₁ à C₃-het, -CO-N(CH₃)het, - CO-NH-het, -CO-O-N(CH₃)-het, -CO-O-NH-het, -CO-N(CH₃)-cycloalkyle en C₃ à C₇, -CO-NH-cycloalkyle en C₃ à C₇, - CO-O-N(CH₃)-cycloalkyle en C₃ à C₇, -CO-O-NH-cycloalkyle en C₃ à C₇, -CO-NH-alkylène en C₁ à C₂-hétaryle, -CON(CH₃)-alkylène en C₁ à C₂-hétaryle, -CO-NH-CH(CH₃)-hétaryle, -CO-NH-C(CH₃)₂-hétaryle, -CO-N(cyclopropyle)-het et -CO-N(cyclopropyl)-hétaryle,
qui peut lui-même être substitué éventuellement par un ou plusieurs radicaux choisis dans le groupe OH, oxo, F, Cl, Br, alkyle en C₁ à ₆, fluoroalkyle en C₁ à C₃, alcanol en C₁ à C₆,
R^{2,1} pouvant être du H, du méthyle, de l'éthyle, du propyle ou de l'isopropyle,
et leurs sels pharmacologiquement acceptables.

16. Composés de formule 1 selon l'une des revendications 1 à 13, dans laquelle
a) R³, R⁴, R^{4'} et R⁵ sont H et
R⁶ et R⁷ forment conjointement un pont choisi dans le groupe comprenant un groupe méthylène et éthylène
ou dans laquelle
b) R³, R⁴, R^{4'} et R⁷ sont H et
R⁵ et R⁶ forment conjointement un pont choisi dans le groupe comprenant les groupes méthylène et éthylène
et leurs sels pharmacologiquement acceptables.

17. Composés de formule 1 selon l'une des revendications 1 à 4, dans laquelle le composé est choisi dans le groupe comprenant : 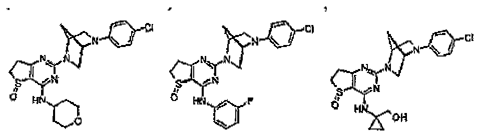 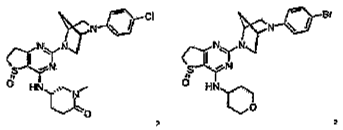 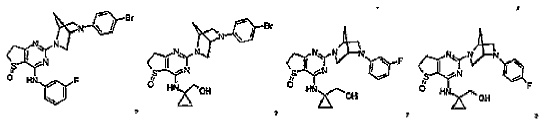 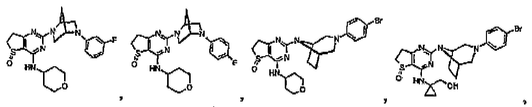 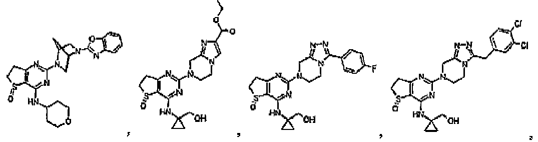 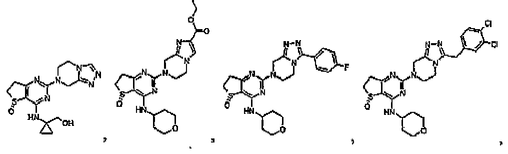 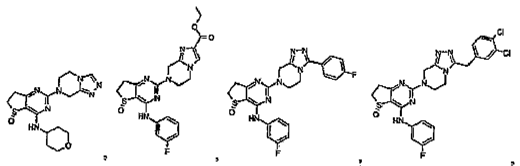 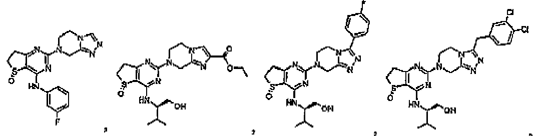 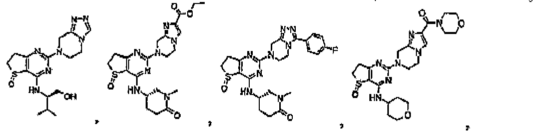 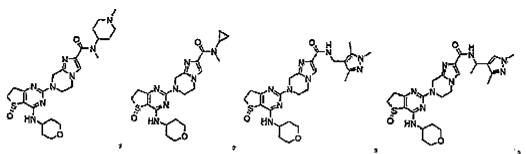 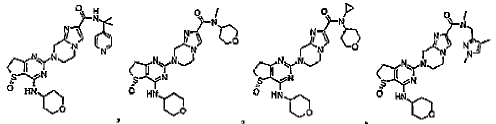 et 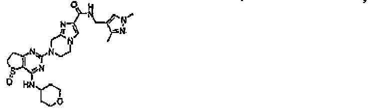 et leurs sels pharmacologiquement acceptables.

18. Composés selon l'une des revendications 1 à 17 comme médicaments.

19. Utilisation de composés selon l'une des revendications 1 à 17 pour la production d'un médicament destiné au traitement des troubles ou maladies des voies respiratoires ou gastro-intestinaux ainsi que les maladies des articulations, de la peau ou des yeux, les cancers et les maladies du système nerveux périphérique ou central.

20. Utilisation des composés selon l'une des revendications 1 à 17 pour la production d'un médicament destiné à prévenir et à traiter les maladies des voies respiratoires ou pulmonaires qui s'accompagnent d'une production accrue de mucus, d'inflammations et/ou les maladies obstructives des voies respiratoires.

21. Utilisation des composés selon l'une des revendications 1 à 17, pour la production d'un médicament destiné à traiter les maladies inflammatoires et obstructives telles que la BPCO, la sinusite chronique, l'asthme, la maladie de Crohn, la rectocolite hémorragique.

22. Utilisation de composés selon l'une des revendications 1 à 17, pour la production d'un médicament destiné à prévenir et à traiter les maladies du système nerveux périphérique ou central telles que la dépression, la dépression bipolaire ou maniaque, les états d'angoisse aiguë ou chronique, la schizophrénie, la maladie d'Alzheimer, la maladie de Parkinson, la sclérose en plaques aiguë ou chronique ou les douleurs aiguës ou chroniques et les lésions cérébrales dues à un accident vasculaire cérébral, une hypoxie ou un traumatisme crânien.

23. Formulations pharmaceutiques **caractérisées par** la teneur en un ou plusieurs composés de formule 1 selon une ou plusieurs des revendications 1 à 17 en combinaison avec une ou plusieurs substances actives choisies dans le groupe comprenant les bêtamimétiques, les corticoïdes, d'autres inhibiteurs de PDE4, des inhibiteurs de l'EGFR et des antagonistes de LTD4, des inhibiteurs de CCR3, 1 des inhibiteurs de iNOS et des inhibiteurs de SYK.
